(19) Europäisches Patentamt
European Patent Office
Office européen des brevets

(11) Veröffentlichungsnummer: **0 226 837**
**A2**

(12) **EUROPÄISCHE PATENTANMELDUNG**

(21) Anmeldenummer: **86116247.7**

(22) Anmeldetag: **24.11.86**

(51) Int. Cl.4: **C07D 239/34**, C07D 239/38, C07D 413/12, C07D 213/70, C07D 239/56, C07D 239/46, C07D 239/78, C07D 405/12, C07D 417/12, C07D 403/12, C07D 401/12

(30) Priorität: **05.12.85 DE 3543037**
**24.01.86 DE 3602016**

(43) Veröffentlichungstag der Anmeldung:
**01.07.87 Patentblatt 87/27**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Sasse, Klaus, Dr.**
**Pützweg 13**
**D-5060 Bergisch Gladbach 2(DE)**
Erfinder: **Fischer, Reiner, Dr.**
**Rembrandtstrasse 15**
**D-4019 Monheim(DE)**
Erfinder: **Hagemann, Hermann, Dr.**
**Kandinskystrasse 52**
**D-5090 Leverkusen 1(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**
Erfinder: **Lürssen, Klaus, Dr.**
**August-Kierspel-Strasse 151**
**D-5060 Bergisch Gladbach 2(DE)**

(54) **Pyri(mi)dyl-oxy-und -thio-benzoesäure-Derivate.**

(57) Die Erfindung betrifft Pyri(mi)dyl-oxy-bzw. -thio-benzoesäure-Derivate der Formel (I),

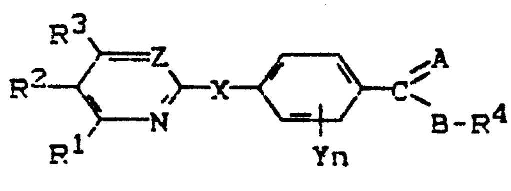

(I)

in welcher
$R^1$, $R^2$, $R^3$, $R^4$, A, B, Z, X, Y und n die in der Beschreibung angegebene Bedeutung haben,

EP 0 226 837 A2

mehrere Verfahren zu ihrer Herstellung und ihre Verwendung als Herbizide und/oder Wachstumsregulatoren.

## Pyri(mi)dyl-oxy-und -thio-benzoesäure-Derivate

Die vorliegende Erfindung betrifft neue Pyri(mi)dyl-oxy-und -thio-benzoesäure-Derivate, mehrere Verfahren zu ihrer Herstellung und ihre Verwendung als Herbizide und Wachstumsregulatoren.

Es ist bereits bekannt, daß bestimmte Benzoesäuren und deren Derivate herbizide Eigenschaften besitzen (vgl. R. Wegler, "Chemie der Pflanzenschutz-und Schädlingsbekämpfungsmittel" Bd. 2, Seiten 289-295; Bd. 5, Seiten 209-217, Springer-Verlag, Berlin 1970/1977).

Ferner ist bekannt, daß zahlreiche Pyridyl-und Pyrimidylether und -thioether herbizide Wirkung aufweisen (vgl. R. Wegler, "Chemie der Pflanzenschutz-und Schädlingsbekämpfungsmittel" Bd. 2, Seiten 353-354 und Bd. 5, Seite 318, Springer-Verlag, Berlin 1970/1977, DDR-Patent Nr. 109 170). Auch sind herbizid wirksame Benzoesäuren und deren Pyri(mi)dyl-oxy-bzw. -thio-substituierten Derivate bekannt (vgl. EP 147 477 und EP 1 187).

Die herbizide Wirksamkeit dieser vorbekannten Verbindungen gegenüber Schadpflanzen ist jedoch ebenso wie ihre Verträglichkeit gegenüber wichtigen Kulturpflanzen nicht immer in allen Anwendungsbereichen zufriedenstellend.

Über eine Wirksamkeit als Wachstumsregulator ist im Zusammenhang mit diesen vorbekannten Pyri(mi)dyl-oxy-bzw. -thio-substituierten Benzoesäure-Derivaten nichts bekannt.

Es wurden nun neue Pyri(mi)dyl-oxy-bzw. -thio-benzoesäure-Derivate der Formel (I),

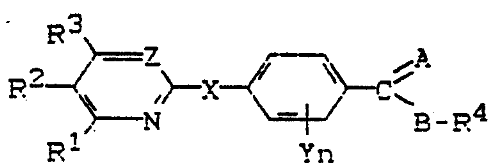

(I)

in welcher

$R^1$, $R^2$ und $R^3$ unabhängig voneinander jeweils für Wasserstoff, Halogen, Alkyl, für gegebenenfalls substituiertes Alkoxy, Halogenalkyl, Alkenyl oder für gegebenenfalls substituiertes Amino steht oder

$R^1$ und $R^2$ oder $R^2$ und $R^3$ gemeinsam für einen ankondensierten 3-bis 6-gliedrigen carbocyclischen Ring stehen,

mit der Maßgabe, daß mindestens einer der Reste $R^1$, $R^2$ oder $R^3$ für Alkyl oder für einen Teil des 3-bis 6-gliedrigen ankondensierten carbocyclischen Ringes steht,

Z für eine (-CH=)-Gruppe oder ein Stickstoffatom steht,

X für Sauerstoff oder Schwefel steht,

Y für Halogen, Nitro, Cyano, Amino, Alkylcarbonylamino, Alkoxycarbonylamino, Phenoxycarbonylalkylamino, Alkyl, Alkoxy oder Halogenalkyl steht, wobei Y gleich oder verschieden sein kann,

n für eine ganze Zahl von 0 bis 4 steht,

A für Sauerstoff, Schwefel, einen Rest $R^5$-N= oder einen Rest $R^6$O-N= steht,

wobei

$R^5$ für Wasserstoff, Alkyl, Hydroxyalkyl, Alkenyl, für jeweils gegebenenfalls substituiertes Cycloalkyl, Aryl oder Aralkyl steht,

$R^6$ für Wasserstoff, jeweils gegebenenfalls substituiertes Alkyl oder einfach oder mehrfach ungesättigtes Alkenyl steht,

B für Sauerstoff, Schwefel, für einen Rest $-\underset{|}{N}-R^7$ oder $-\underset{|}{N}-OR^8$ steht,

wobei

$R^7$ für Wasserstoff, für gegebenenfalls durch Halogen, Cyano oder durch einen Rest -D-$R^9$ substituiertes Alkyl oder für Alkenyl steht, wobei

D für Sauerstoff, Schwefel, Sulfinyl oder Sulfonyl steht und

$R^9$ für Wasserstoff, Alkyl, einfach oder mehrfach ungesättigtes Alkenyl oder für jeweils gegebenenfalls substituiertes Aryl oder Aralkyl steht, oder für -CO-Alkyl steht,

$R^8$ für Wasserstoff, jeweils gegebenenfalls substituiertes Alkyl oder einfach oder mehrfach ungesättigtes Alkenyl steht und

$R^4$ für gegebenenfalls substituiertes gesättigtes oder ungesättigtes Alkyl steht, wobei als Substituenten ausgewählt sind:

Halogen, Nitro; Cyano, ein Rest -D-$R^9$, Alkoximo, die Gruppen

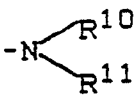 , 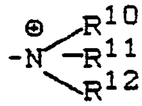 ,

-CO-$OR^{13}$, -CO-$NR^{14}R^{15}$, -CS-$NR^{14}R^{15}$, -$SO_2$-$NR^{14}R^{15}$;
Cycloalkyl, Aryl oder ein 5-bzw. 6-gliedriger Heterocyclus,
$R^4$ weiterhin für jeweils gegebenenfalls substituiertes Cycloalkyl, Aryl oder einen 5-bis 7-gliedrigen Heterocyclus steht, wobei
D und $R^9$ die oben angegebene Bedeutung haben,
$R^{10}$ für Wasserstoff oder Alkyl steht,
$R^{11}$ für Alkyl, Acyl, Alkylsulfonyl, Arylsulfonyl oder jeweils gegebenenfalls substituiertes Aryl oder Aralkyl steht oder
$R^{10}$ und $R^{11}$ gemeinsam mit dem Stickstoff, an den sie gebunden sind, auch für einen 5-bis 7-gliedrigen Heterocyclus stehen,
$R^{12}$ für Alkyl steht,
$R^{13}$ für Alkyl steht,
$R^{14}$ und $R^{15}$ unabhängig voneinander jeweils für Wasserstoff, Alkyl, Cycloalkyl oder für jeweils gegebenenfalls substituiertes Aryl oder Aralkyl stehen oder
$R^{14}$ und $R^{15}$ gemeinsam mit dem Stickstoff, an den sie gebunden sind, für einen 5-bis 7-gliedriegen Heterocyclus stehen oder
$R^4$ gemeinsam mit $R^5$ und B oder mit $R^6$ und B oder mit $R^7$ und B oder mit $R^8$ und B oder mit A und B einen 5-oder 6-gliedrigen Ring bilden kann.

Weiterhin wurde gefunden, daß man die neuen Pyri(mi)dyloxy-und -thio-benzoesäure-Derivate der Formel (I),

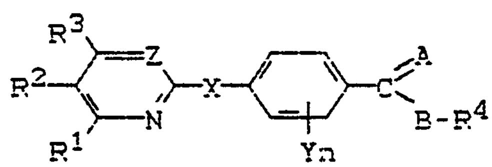 (I)

in welcher $R^1$, $R^2$ und $R^3$ unabhängig voneinander jeweils für Wasserstoff, Halogen, Alkyl, für gegebenenfalls substituiertes Alkoxy, Halogenalkyl, Alkenyl oder für gegebenenfalls substituiertes Amino steht oder
$R^1$ und $R^2$ oder $R^2$ und $R^3$ gemeinsam für einen ankondensierten 3-bis 6-gliedrigen carbocyclischen Ring stehten,
mit der Maßgabe, daß mindestens einer der Reste $R^1$, $R^2$ oder $R^3$ für Alkyl oder für einen Teil des 3-bis 6-gliedrigen ankondensierten carbocyclischen Ringes steht,
Z für eine (-CH=)-Gruppe oder ein Stickstoffatom steht,
X für Sauerstoff oder Schwefel steht,
Y für Halogen, Nitro, Cyano, Amino, Alkylcarbonylamino, Alkoxycarbonylamino, Phenoxycarbonylamino, Alkyl, Alkoxy oder Halogenalkyl steht, wobei Y gleich oder verschieden sein kann,
n für eine ganze Zahl von 0 bis 4 steht,
A für Sauerstoff, Schwefel, einen Rest $R^5$-N= oder einen Rest $R^6$O-N= steht,
wobei
$R^5$ für Wasserstoff, Alkyl, Hydroxyalkyl, Alkenyl, für jeweils gegebenenfalls substituiertes Cycloalkyl, Aryl oder Aralkyl steht,
$R^6$ für Wasserstoff, jeweils gegebenenfalls substituiertes Alkyl oder einfach oder mehrfach ungesättigtes Alkenyl steht,
B für Sauerstoff, Schwefel, für einen Rest -N(-)-$R^7$ oder -N(-)-$OR^8$ steht,

wobei
$R^7$ für Wasserstoff, für gegebenenfalls durch Halogen, Cyano oder durch einen Rest -D-$R^9$ substituiertes Alkyl oder für Alkenyl steht, wobei
D für Sauerstoff, Schwefel, Sulfinyl oder Sulfonyl steht und

$R^9$ für Wasserstoff, Alkyl, einfach oder mehrfach ungesättigtes Alkenyl oder für jeweils gegebenenfalls substituiertes Aryl oder Aralkyl steht, oder für -CO-Alkyl steht,

$R^8$ für Wasserstoff, jeweils gegebenenfalls substituiertes Alkyl oder einfach oder mehrfach ungesättigtes Alkenyl steht und

$R^4$ für gegebenenfalls substituiertes gesättigtes oder ungesättigtes Alkyl steht, wobei als Substituenten ausgewählt sind:

Halogen, Nitro, Cyano, ein Rest -D-$R^9$, Alkoximo, die Gruppen

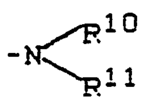
,
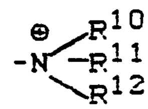
,

-CO-$OR^{13}$, -CO-$NR^{14}R^{15}$, -CS-$NR^{14}R^{15}$, -$SO_2$-$NR^{14}R^{15}$, Cycloalkyl, Aryl oder ein 5-bzw. 6-gliedriger Heterocyclus;

$R^4$ weiterhin für jeweils gegebenenfalls substituiertes Cycloalkyl, Aryl oder einen 5-bis 7-gliedrigen Heterocyclus steht,

wobei

D und $R^9$ die oben angegebene Bedeutung haben,

$R^{10}$ für Wasserstoff oder Alkyl steht,

$R^{11}$ für Alkyl, Acryl, Alkylsulfonyl, Arylsulfonyl oder jeweils gegebenenfalls substituiertes Aryl oder Aralkyl steht oder

$R^{10}$ und $R^{11}$ gemeinsam mit dem Stickstoff, an den sie gebunden sind, auch für einen 5-bis 7-gliedrigen Heterocyclus stehen,

$R^{12}$ für Alkyl steht,

$R^{13}$ für Alkyl steht,

$R^{14}$ und $R^{15}$ unabhängig voneinander jeweils für Wasserstoff, Alkyl, Cycloalkyl oder für jeweils gegebenenfalls substituiertes Aryl oder Aralkyl stehen oder

$R^{14}$ und $R^{15}$ gemeinsam mit dem Stickstoff, an den sie gebunden sind, für einen 5-bis 7-gliedriegen Heterocyclus stehen oder

$R^4$ gemeinsam mit $R^5$ und B oder mit $R^6$ und B oder mit $R^7$ und B oder mit $R^8$ und B oder mit A und B einen 5-oder 6-gliedrigen Ring bilden kann,

nach den im folgenden beschriebenen Verfahren erhält:

Man erhält

(A-$a_1$) Verbindungen der Formel (I), in denen A für Sauerstoff steht, wenn man Benzoesäure-Derivate der Formel (II),

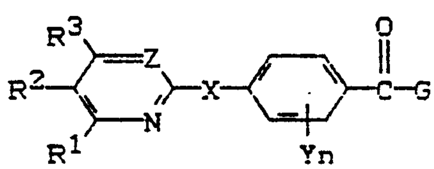
(II)

in welcher

$R^1$, $R^2$, $R^3$, Z, X, Y und n die oben angegebene Bedeutung haben und

G für halogen, für den Rest -O-C(=O)-O-Alkyl oder -O-$SO_2$-Aryl oder für Imidazolyl steht, mit Verbindungen der Formel (III),

H-B-$R^4$ (III)

in welcher

B und $R^4$ die oben angegebene Bedeutung haben, gegebenenfalls in Gegenwart eines verdünnungsmittels und gegebenenfalls in Gegenwart eines Säurebindemittels umsetzt,

oder man erhält

(A-$a_2$) Verbindungen der Formel (I), in denen A für Schwefel steht, wenn man Thiobenzoylchloride der Formel (IV),

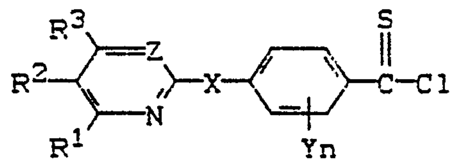

(IV)

in welcher
$R^1$, $R^2$, $R^3$, Z, X, Y und n die oben angegebene Bedeutung haben,
mit Verbindungen der Formel (III),
$H-B-R^4$ (III)
in welcher
B und $R^4$ die oben angegebene Bedeutung haben,
gegebenenfalls in Gegenwart eines Verdünnungsmittels sowie in Gegenwart eines Säurebindemittels umsetzt,
oder man erhält
($A-a_3$) Verbindungen der Formel (I), in denen A und B für Schwefel stehen, wenn man Dithiobenzoesäuren der Formel (V),

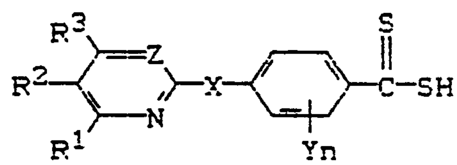

(V)

in welcher
$R^1$, $R^2$, $R^3$, Z, X, Y und n die oben angegebene Bedeutung haben,
($a_3$-$\alpha$) mit Alkylierungsmitteln der Formel (VI),
$L-R^{4-1}$ (VI)
in welcher
$R^{4-1}$ für gegebenenfalls substituiertes Alkyl oder Aralkyl steht und
L für Halogen oder für den Rest $R^{4-1}$ $-O-SO_2-O-$steht,
in Gegenwart eines Verdünnungsmittels und gegebenenfalls in Gegenwart eines Säurebindemittels umsetzt, oder
($a_3$-$\beta$) mit Olefinen der Formel (VII),

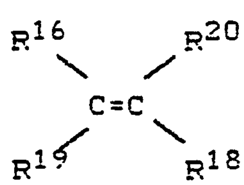

(VII)

in welcher
$R^{16}$, $R^{18}$, $R^{19}$ und $R^{20}$ unabhängig voneinander jeweils für Wasserstoff, Nitro, Cyano, für jeweils gegebenenfalls substituiertes Alkyl, Cycloalkyl oder Aryl stehen oder für einen Rest $-COOR^{13}$, $-CO-NR^{14}R^{15}$, $-CS-NR^{14}R^{15}$, $-SO_2-NR^{14}R^{15}$ oder den Rest $-D-R^9$ stehen oder
$R^{16}$ und $R^{20}$ gemeinsam eine dritte Kohlenstoff-Kohlenstoffbindung bilden,
wobei
D, $R^9$, $R^{13}$, $R^{14}$ ;und $R^{15}$ die oben angegebene Bedeutung haben,
in Gegenwart eines Verdünnungsmittels und gegebenenfalls in Gegenwart eines Katalysators umsetzt,
oder man erhält
($A-a_4$) Verbindungen der Formel (I), in denen A für Schwefel und B für den Rest $-\underset{|}{N}-R^7$ oder $-\underset{|}{N}-OR^8$ stehen, wenn man

($a_4$-$\alpha$) Dithiobenzoesäure-Derivate der Formel (Va),

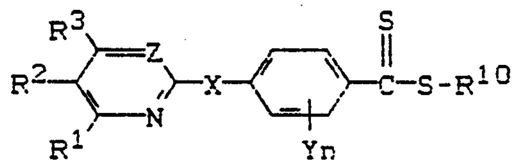

(Va)

in welcher
$R^1$, $R^2$, $R^3$, Z, X, Y und n die oben angegebene Bedeutung haben und
$R^{10}$ für Waserstoff oder Alkyl, insbesondere für Wasserstoff oder $C_1$-$C_4$-Alkyl steht,
mit Aminen der Formel (VIIIa) oder Hydroxylaminen der Formel (VIIIb),

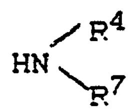

(VIIIa)

oder

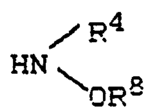

(VIIIb)

in welchen
$R^4$, $R^7$ und $R^8$ die oben angegebene Bedeutung haben,
gegebenenfalls in Gegenwart eines Verdünnungsmittels und gegebenenfalls in Gegenwart eines Katalysators umsetzt, oder wenn man
($a_4$-$\beta$) Benzimidchloride der Formel (IXa),

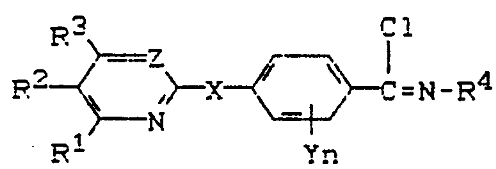

(IXa)

in welcher
$R^1$, $R^2$, $R^3$, $R^4$, Z, X, Y und n die oben angegebene Bedeutung haben,
mit Schwefelwasserstoff in Gegenwart eines Verdünnungsmittels umsetzt, oder wenn man
($a_4$-$\gamma$) Benzoesäureamide der Formel (Ia)

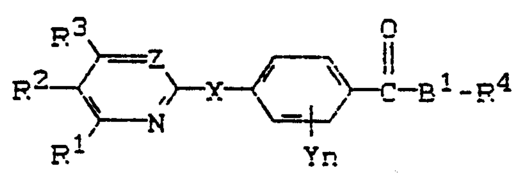

(Ia)

in welcher
$R^1$, $R^2$, $R^3$, $R^4$, Z, X, Y und n die oben angegebene Bedeutung haben und
$B^1$ für den Rest $-\underset{|}{N}-R^7$ oder $-\underset{|}{N}-OR^8$ steht,

mit Schwefelungsagentien gegebenenfalls in Gegenwart eines Verdünnungsmittels umsetzt,
oder man erhält
($A$-$a_5$) Verbindungen der Formel (I), in denen A für einen Rest $R^5$-N= steht, wenn man
($a_5$-$\alpha$) Benzimidchloride der Formel (IXb),

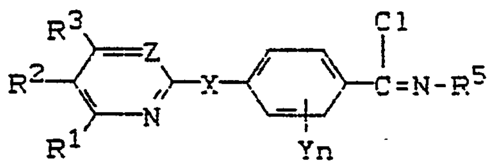

(IXb)

in welcher
$R^1$, $R^2$, $R^3$, $R^5$, Z, X, Y und n die oben angegebene Bedeutung haben,
mit Verbindungen der Formel (III),
H-B-$R^4$ (III)
in welcher
B und $R^4$ die oben angegebene Bedeutung haben,
gegebenenfalls in Gegenwart eines Verdünnungsmittels sowie in Gegenwart eines Säurebindemittels umsetzt,
oder wenn man
($a_5$-$\beta$) Thiobenzamide der Formel (X)

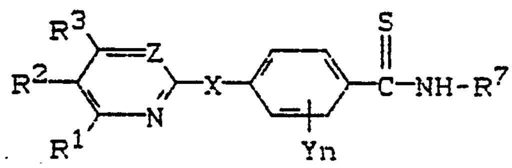

(X)

in welcher
$R^1$, $R^2$, $R^3$, $R^7$, Z, X, Y und n die oben angegebene Bedeutung haben,
mit Alkylierungsmitteln der Formel (VI),
L-$R^{4-1}$ (VI)
in welcher
$R^{4-1}$ und L die oben angegebene Bedeutung haben,
gegebenenfalls in Gegenwart eines Verdünnungsmittels und gegebenenfalls in Gegenwart eines Säurebindemittels umsetzt,
oder man erhält
(A-$a_6$) Verbindungen der Formel (I), in denen A für den Rest $R^6$-O-N= steht, wenn man
($a_6$-$\alpha$) Hydroximsäurehalogenide der Formel (XIa),

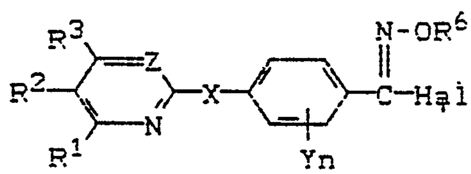

(XIa)

in welcher
$R^1$, $R^2$, $R^3$, $R^6$, Z, X, Y und n die oben angegebene Bedeutung haben und
Hal für Chlor oder Brom steht,
mit Verbindungen der Formel (III),
H-B-$R^4$ (III)
in welcher
B und $R^4$ die oben angegebene Bedeutung haben,
gegebenenfalls in Gegenwart eines Verdünnungsmittels sowie in Gegenwart eines Säurebindemittels umsetzt oder wenn man
($a_6$-$\beta$) Hydroxamsäuren der Formel (XIb),

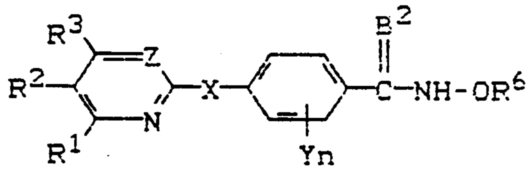

(XIb)

in welcher
$R^1$, $R^2$, $R^3$, $R^6$, Z, X, Y und n die oben angegebene Bedeutung haben und
$B^2$ für Sauerstoff oder Schwefel steht,
mit Alkylierungsmitteln der Formel (VI),
$L-R^{4-1}$ (VI)
in welcher
L und $^{4-1}$ die oben angegebene Bedeutung haben
gegebenenfalls in Gegenwart eines Verdünnungsmittels und gegebenenfalls in Gegenwart eines Säurebindemittels umsetzt oder wenn man
($a_4$-$\gamma$) Thiobenzoesäureester der Formel (Ib),

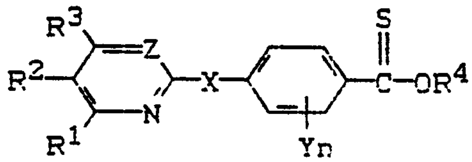

(Ib)

in welcher
$R^1$, $R^2$, $R^3$, $R^4$, Z, X, Y und n die oben angegebene Bedeutung haben,
mit Hydroxylaminen der Formel (XII),
$R^6-O-NH_2$ (XII)
in welcher
$R^6$ die oben angegebene Bedeutung hat,
gegebenenfalls in Gegenwart eines Verdünnungsmittels umsetzt,
oder man erhält
(A-$a_7$) Verbindungen der Formel (I), in denen $R^4$, A und B gemeinsam einen heterocyclischen Ring bilden, wenn man
($a_7$-$\alpha$) Benzonitrile der Formel (XIII),

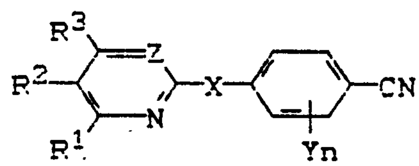

(XIII)

in welcher
$R^1$, $R^2$, $R^3$, Z, X, Y und n die oben angegebene Bedeutung haben,
mit 2-oder 3-Hydroxyalkylaminen der Formel (XIV),

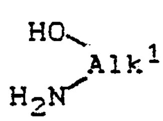

(XIV)

in welcher
$Alk^1$ für eine gegebenenfalls substituierte $C_2$-$C_3$-Alkylenkette steht, wobei als Substituenten ausgewählt sind: Fluor, Chlor, $C_1$-$C_3$-Alkyl, $C_1$-$C_3$-Alkoxy, Hydroxy-$C_1$-$C_3$-alkyl, $C_3$-$C_6$-Cycloalkyl oder gegebenenfalls durch Fluor, Chlor, Methyl, Ethyl, Methoxy oder Trifluormethyl substituiertes Phenyl,
gegebenenfalls in Gegenwart eines Verdünnungsmittels und in Gegenwart eines Katalysators umsetzt zu den Verbindungen der Formel (Ic),

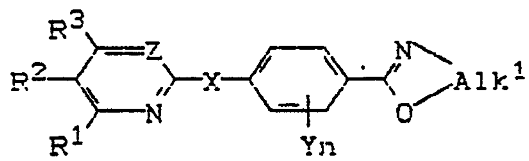 (Ic)

in welcher
$R^1$, $R^2$, $R^3$, $Alk^1$, Z, X, Y und n die oben angegebene Bedeutung haben,
oder wenn man
($a_7$-β) Thiobenzamide der Formel (Xa)

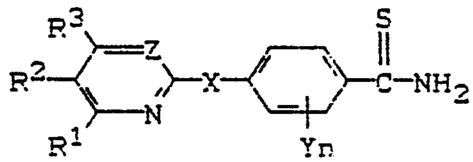 (Xa)

in welcher
$R^1$, $R^2$, $R^3$, Z, X, Y und n die oben angegebene Bedeutung haben,
mit α-Halogencarbonyl-Derivaten der Formel (XV),

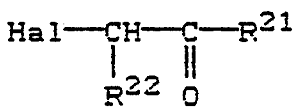 (XV)

in welcher
Hal für Chlor oder Brom steht,
$R^{21}$ und $R^{22}$ unabhängig voneinander für Wasserstoff, Alkyl, Cycloalkyl oder gegebenenfalls substituiertes Aryl steht,
gegebenenfalls in Gegenwart eines Verdünnungsmittels sowie in Gegenwart eines Säurebindemittels umsetzt zu Verbindungen der Formel (Id),

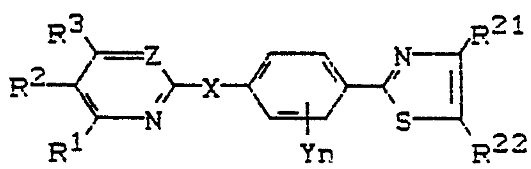 (Id)

in welcher
$R^1$, $R^2$, $R^3$, $R^{21}$, $R^{22}$, Z, X, Y und n die oben angegebene Bedeutung haben,
oder wenn man
($a_7$-γ) Hydroxamsäuren der Formel (Ib-1),

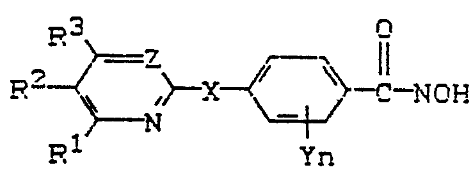 (Ib-1)

in welcher
$R^1$, $R^2$, $R^3$, Z, X, Y und n die oben angegebene Bedeutung haben,
mit bifunktionellen Alkylierungsmitteln der Formel (VIa),
$L^1$-$Alk^2$-$L^2$ (VIa)
in welcher
$L^1$ und $L^2$ für Chlor oder Brom stehen und
$Alk^2$ für eine gegebenenfalls substituierte $C_2$-oder $C_3$-Alkylenkette steht, wobei als Substituenten ausgewählt sind: $C_1$-$C_3$-Alkyl, $C_3$-$C_6$-Cycloalkyl oder gegebenenfalls durch Fluor, Chlor, Methyl, Ethyl, Methoxy oder Trifluormethyl substituiertes Phenyl,
gegebenenfalls in Gegenwart eines Verdünnungsmittels sowie in Gegenwart eines Säurebindemittels umsetzt,
oder wenn man
($a_7$-δ) Benzamidoxime der Formel (XVI),

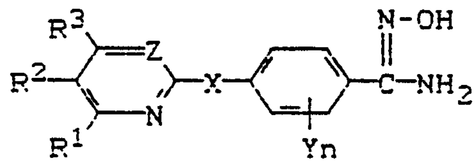
(XVI)

in welcher
$R^1$, $R^2$, $R^3$, Z, X, Y und n die oben angegebene Bedeutung haben,
mit Acrylierungsmitteln der Formel (XVII),

$G-\overset{O}{\overset{||}{C}}-R^{4-2}$ (XVII)

in welcher
G die oben angegebene Bedeutung hat und
$R^{4-2}$ für einen um ein Kohlenstoffatom verkürzten Rest $R^4$ steht, wobei $R^4$ die oben angegebene Bedeutung hat, insbesondere für gegebenenfalls durch Halogen, Aryl oder den Rest -D-$R^9$ substituiertes Alkyl, für Cycloalkyl oder für gegebenenfalls substituiertes Aryl steht,
gegebenenfalls in Gegenwart eines Verdünnungsmittels und gegebenenfalls in Gegenwart eines Säurebindemittels umsetzt und die so erhaltenen Verbindungen der Formel (XVIa),

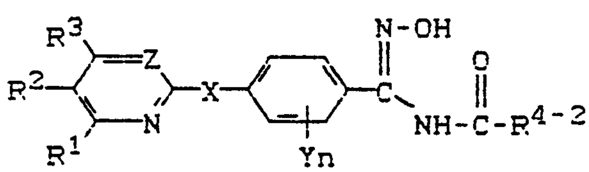
(XIVa)

in welcher
$R^1$, $R^2$, $R^3$, $R^{4-2}$, Z, X, Y und n die oben angegebene Bedeutung haben,
mit wasserentziehenden Mitteln umsetzt zu den Pyri(mi)dyl-oxy-oder -thiobenzoesäure-Derivate der Formel-(Ie),

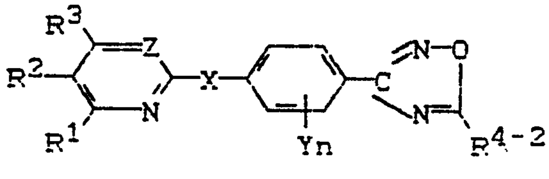
(Ie)

in welcher
$R^1$, $R^2$, $R^3$, $R^{4-2}$, Z, X, Y und n die oben angegebene Bedeutung haben,

oder man erhält

(B) Pyri(mi)dyl-oxy-und -thiobenzoesäure-Derivate der Formel (I), wenn man

(B-$b_1$) Pyri(mi)din-Derivate der Formel (XVIII)

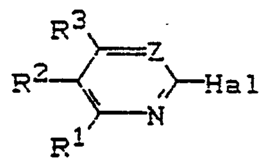

(XVIII)

in welcher

$R^1$, $R^2$, $R^3$ und Z die oben angegebene Bedeutung haben und

Hal für Chlor oder Brom steht,

mit 4-Hydroxy oder 4-Mercapto-benzoesäure-Derivaten der Formel (XIX),

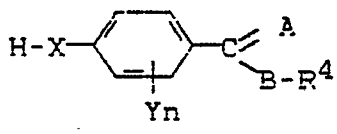

(XIX)

in welcher

X, Y, n, A, B und $R^4$ die oben angegebene Bedeutung haben,

gegebenenfalls in Gegenwart eines Verdünnungsmittels sowie in Gegenwart eines Säurebindemittels umsetzt, oder wenn man

(B-$b_2$) 2-Mercapto-pyri(mi)dine der Formel (XX)

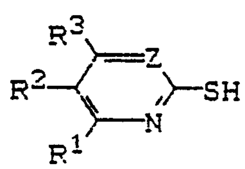

(XX)

in welcher

$R^1$, $R^2$, $R^3$, und Z die oben angegebene Bedeutung haben,

mit 4-Halogenbenzoesäure-Derivaten der Formel (XXI),

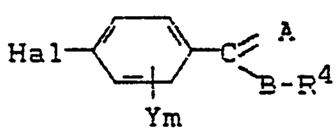

(XXI)

in welcher

$R^4$, A, B und Y die oben angegebene Bedeutung haben,

m für 1, 2 oder 3 steht und

Hal für Chlor oder Brom steht,

gegebenenfalls in Gegenwart eines Verdünnungsmittels und in Gegenwart eines Säurebindemittels umsetzt, oder man erhält

(C) Verbindungen der Formel (I), aus anderen Verbindungen der Formel (I) durch Austausch eines Substituenten Y, wenn man

(C-$c_1$) Verbindungen der Formel (I), in denen Y in 3-und/oder 5-Stellung für Wasserstoff steht, durch

Umsetzung mit einem Halogenierungsmittel in Gegenwart eines Verdünnungsmittels und gegebenenfalls in Gegenwart eines Katalysators umwandelt in solche Verbindungen der Formel (I), in denen Y für Chlor oder Brom steht,

oder wenn man

($C-c_2$) Verbindungen der Formel (I), in denen Y für Nitro steht, durch Umsetzung mit Reduktionsmitteln nach bekannten Verfahren umwandelt in solche Verbindungen der Formel (I), in denen Y für Amino steht,

und

($C-c_3$) gegebenenfalls diese Amino-substituierten Verbindungen der Formel (I) (Y = steht für Amino) mit Säurechloriden den Formel (XXII),

$R^{23}-\overset{O}{\overset{\|}{C}}-Cl$ (XXII)

in welcher

$R^{23}$ für $C_1$-$C_4$ Alkyl, $C_1$-$C_4$-Alkoxy oder Di-$C_1$-$C_4$-alkylamino steht,

oder mit Methyl-oder Ethylisocyanat in allgemein üblicher Art und Weise acyliert,

oder wenn man

($C-c_4$) Verbindungen der Formel (I), in denen Y für Amino steht, durch Umsetzung mit salpetriger Säure oder einem Alkylnitrit, insbesondere Methyl-oder Ethylnitrit, gegebenenfalls in Gegenwart eines Katalysators nach bekannten Verfahren umwandelt in solche Verbindungen der Formel (I), in denen Y für Halogen oder Cyano steht,

oder man erhält

(D) Verbindungen der Formel (I), in denen $R^3$ für den Rest $-O-R^{24}$ oder $-NR^{25}R^{26}$ steht, wobei $R^{24}$, $R^{25}$ und $R^{26}$ die unten angegebene Bedeutung haben,

wenn man Pyri(mi)dyl-oxy-und -thio-benzoesäure-Derivate der Formel (If),

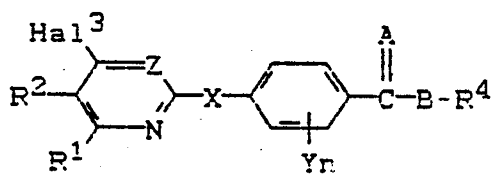

(If)

in welcher

$R^1$, $R^2$, $R^4$, Z, X, Y, n, A und B die oben angegebene Bedeutung haben und

$Hal^3$ für Fluor, Chlor oder Brom steht,

mit Alkoholen der Formel (XXIII),

$H-O-R^{24}$ (XXIII)

in welcher

$R^{24}$ für gegebenenfalls durch Fluor, Chlor oder Methoxy substituiertes $C_1$-$C_4$-Alkyl oder $C_2$-$C_4$-Alkenyl steht,

gegebenenfalls in Gegenwart eines Verdünnungsmittels und in Gegenwart eines Säurebindemittels umsetzt,

oder

mit Ammoniak, primären oder sekundären Aminen der Formel (XXIV),

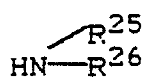

(XXIV)

in welcher

$R^{25}$ und $R^{26}$ unabhängig voneinander jeweils für Wasserstoff oder $C_1$-$C_4$-Alkyl, stehen oder

$R^{25}$ und $R^{26}$ gemeinsam mit dem N-Atom auch einen 5-bis 7-gliedrigen heterocyclischen Ring bilden können,

in Gegenwart eines Verdünnungsmittels und in Gegenwart eines Säurebindemittels umsetzt.

Schließlich wurde gefunden, daß die neuen Pyri(mi)dyloxy-und -thiobenzoesäure-Derivate der Formel - (I) herbizide, insbesondere auch selektiv-herbizide, Eigenschaften und darüberhinaus auch pflanzenwachstumsregulatorische Eigenschaften besitzen.

Überraschenderweise zeigen die erfindungsgemäßen Pyri(mi)dyloxy-und -thiobenzoesäure-Derivate der Formel (I) eine deutlich verbesserte allgemein-herbizide Wirksamkeit gegenüber Schadpflanzen als die aus dem Stand der Technik bekannten Pyridyl-und Pyrimidyl-ether und -thioether, welche chemisch und wirkungsmäßig naheliegende Verbindungen sind. Außerdem zeigen die erfindungsgemäßen Verbindungen der Formel (I) pflanzenwachstumsregulatorische Wirksamkeit.

Die erfindungsgemäßen Pyri(mi)dyloxy-und -thiobenzoesäure-Derivate sind durch die Formel (I) allgemein definiert. Bevorzugt sind Verbindungen der Formel (I), bei welchen

$R^1$, $R^2$ und $R^3$ unabhängig voneinander jeweils für Wasserstoff, Fluor, Chlor, Brom, für Alkyl mit 1 bis 6 Kohlenstoffatomen, für Alkenyl mit 2 bis 6 Kohlenstoffatomen, für gegebenenfalls durch $C_2$-$C_4$-Alkenyl oder $C_1$-$C_2$-Alkoxy substituiertes Alkoxy, Alkylamino, Dialkylamino mit jeweils 1 bis 4 Kohlenstoffatomen in den einzelnen Alkylteilen, wobei im Fall des Dialkylamino die Alkylsubstituenten mit dem Stickstoffatom, an das sie gebunden sind, einen 5-bis 7-gliedrigen heterocyclischen Ring bilden können, für Amino, Alkenylamino oder Dialkenylamino mit jeweils 3 bis 4 Kohlenstoffatomen in den einzelnen Alkenylteilen, oder für Trifluormethyl stehen oder

$R^1$ und $R^2$ oder $R^2$ und $R^3$ gemeinsam für einen 5-oder 6-gliedrigen carbocyclischen Ring stehen, mit der Maßgabe, daß mindestens einer der Reste $R^1$, $R^2$ oder $R^3$ für Alkyl mit 1 bis 6 Kohlenstoffatomen, Trifluormethyl oder für einen Teil des ankondensierten 5-oder 6-gliedrigen carbocyclischen Ring steht,

Z für eine (-CH=)-Gruppe oder ein Stickstoffatom steht,

X für Sauerstoff oder Schwefel steht,

Y für Fluor, Chlor, Brom, Iod, Nitro, Cyano, Amino, für Alkyl, Alkoxy, Halogenalkyl, Alkylcarbonylamino, Alkoxycarbonylamino mit jeweils 1 bis 4 Kohlenstoffatomen im Alkylteil und im Fall des Halogenalkyl mit 1 bis 5 Halogenatomen steht, wobei Halogen für Fluor, Chlor, Brom oder Iod steht, oder für Phenoxycarbonylamino steht, wobei Y gleich oder verschieden sein kann,

n für eine ganze Zahl 0, 1, 2, 3 oder 4 steht,

A für Sauerstoff, Schwefel, einen Rest $R^5$-N= oder einen Rest $R^6$O-N= steht,

wobei

$R^5$ für Wasserstoff, für Alkyl, Hydroxyalkyl oder Alkoxyalkyl mit jeweils 1 bis 4 Kohlenstoffatomen in den einzelnen Alkylteilen, für Alkenyl mit 3 bis 4 Kohlenstoffatomen, für gegebenenfalls durch Fluor, Chlor, Brom, $C_1$-$C_4$-Alkyl oder $C_1$-$C_4$-Alkoxy substituiertes Cycloalkyl mit 3 bis 7 Kohlenstoffatomen, für jeweils gegebenenfalls durch Fluor, Chlor, Brom, Iod, $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl oder Halogen-$C_1$-$C_4$-alkylsulfonyl substituiertes Phenyl, Benzyl oder Phenethyl steht,

$R^6$ für Wasserstoff oder für Alkyl mit 1 bis 4 Kohlenstoffatomen oder Alkenyl mit 3 bis 4 Kohlenstoffatomen steht,

B für Sauerstoff, Schwefel oder für einen Rest $-\underset{|}{N}-R^7$ steht,

wobei

$R^7$ für Wasserstoff, für gegebenenfalls durch Fluor, Chlor, Brom, Iod, Cyano oder durch einen Rest -D-$R^9$ substituiertes Alkyl mit 1 bis 4 Kohlenstoffatomen oder für Alkenyl mit 3 bis 4 Kohlenstoffatomen steht,

wobei

D für Sauerstoff, Schwefel, Sulfinyl oder Sulfonyl steht und

$R^9$ für Wasserstoff, für Alkyl mit 1 bis 4 Kohlenstoffatomen, für Alkenyl mit 3 bis 4 Kohlenstoffatomen, für jeweils gegebenenfalls substituiertes Phenyl, Benzyl oder Phenethyl, wobei als Phenylsubstituenten Fluor, Chlor, Brom, $C_1$-$C_4$-Alkyl oder $C_1$-$C_4$-Alkoxy ausgewählt sind,

$R^4$ für jeweils gegebenenfalls substituiertes Alkyl mit 1 bis 12 Kohlenstoffatomen, jeweils einfach oder mehrfach ungesättigtes Alkenyl oder Alkinyl mit jeweils bis zu 12 Kohlenstoffatomen, wobei als Substituenten jeweils ausgewählt sind:

Fluor, Chlor, Cyano, Alkoximino mit 1 bis 4 Kohlenstoffatomen im Alkylteil, gegebenenfalls durch $C_1$-$C_4$-Alkyl und/oder Fluor und/oder Chlor substituiertes Cycloalkyl mit 3 bis 6 Kohlenstoffatomen, gegebenenfalls durch Fluor, Chlor, Brom, Nitro, Trifluormethyl, $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Phenyl, einen 5-oder 6-gliedrigen Heterocyclus der 1 bis 3 weitere Heteroatome aus der Reihe Sauerstoff und/oder Schwefel und/oder Stickstoff enthalten kann, ein Rest -D-$R^9$, ein Rest

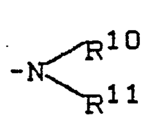 , 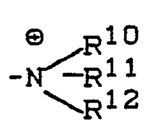 ,

$-CO-OR^{13}$, $-CO-NR^{14}R^{15}$, $-CS-NR^{14}R^{15}$, $-SO_2-NR^{14}R^{15}$;
wobei
D und $R^9$ die oben angegebene Bedeutung haben,
$R^{10}$ für Wasserstoff oder Alkyl mit 1 bis 4 Kohlenstoffatomen steht,
$R^{11}$ für Alkyl mit 1 bis 4 Kohlenstoffatomen, Acyl, Alkylsulfonyl mit 1 bis 4 Kohlenstoffatomen oder Phenylsulfonyl steht oder
$R^{10}$ und $R^{11}$ gemeinsam mit dem Stickstoff, an den sie gebunden sind, für eine 5-bis 7-gliedriegen Heterocyclus stehen,
$R^{12}$ für Alkyl mit 1 bis 4 Kohlenstoffatomen steht,
$R^{13}$ für Alkyl mit 1 bis 4 Kohlenstoffatomen steht,
$R^{14}$ und $R^{15}$ jeweils unabhängig voneinander für Wasserstoff oder Alkyl mit 1 bis 6 Kohlenstoffatomen stehen oder
$R^{14}$ und $R^{15}$ gemeinsam mit dem Stickstoff, an den sie gebunden sind, für einen 5-oder 6-gliedrigen Heterocyclus stehen, oder
$R^4$ weiterhin für gegebenenfalls durch Fluor, Chlor, $C_1$-$C_4$-Alkyl, $C_1$-$C_4$-Alkoxy, $C_2$-$C_4$-Alkenyl und/oder Halogen-$C_1$-$C_4$-alkyl, wobei Halogen für 1 bis 5 Fluor-und/oder Chloratome steht, substituiertes Cycloalkyl mit 3 bis 8 Kohlenstoffatomen steht, für gegebenenfalls substituiertes Phenyl, wobei als Substituenten ausgewählt sind: Fluor, Chlor, Brom, $C_1$-$C_4$-Alkyl, Halogen-$C_1$-$C_4$-alkyl, wobei Halogen für 1 bis 5 Fluor-und/oder Chlor-und/oder Bromatome steht, oder ein Rest $-D^1-R^{17}$ steht, wobei
$D^1$ für Sauerstoff, Schwefel, Sulfinyl oder Sulfonyl steht,
$R^{17}$ für Wasserstoff, $C_1$-$C_4$-Alkyl, Halogen-$C_1$-$C_4$-alkyl, wobei Halogen für 1 bis 5 Fluor-und/oder Chloratome steht, $C_3$-$C_4$-Alkenyl ein Rest

$$-CO-O-C_1-C_4\text{-Alkyl},\quad -CO-N\begin{matrix} C_1-C_4\text{-Alkyl} \\ C_1-C_4\text{-Alkyl} \end{matrix}$$

, gegebenenfalls am Stickstoff einfach oder zweifach durch $C_1$-$C_4$-Alkyl substituiertes Sulfonamid,
$R^4$ weiterhin für einen 5-bis 7-gliedrigen Heterocyclus steht, der ein bis drei gleiche oder verschiedene Heteroatome aus der Reihe Sauerstoff, Stickstoff oder Schwefel enthalten kann und welcher gegebenenfalls substituiert ist durch Fluor, Chlor, Brom, Iod, $C_1$-$C_4$-Alkyl, Halogen-$C_1$-$C_4$-alkyl, wobei Halogen für 1 bis 5 gleiche oder verschiedene Fluor-, Chlor-, Brom-oder Iodatome steht, durch Amino, Alkylamino oder Dialkylamino mit jeweils 1 bis 4 Kohlenstoffatomen in den einzelnen Alkylteilen, durch Nitro, Cyano, den Rest $-D-R^9$ (mit gleicher Bedeutung für D und $R^9$ wie oben), den Rest
$-CO-O-C_1$-$C_4$-Alkyl, $CO-NH-C_1$-$C_4$-Alkyl oder $-CO-N(C_1$-$C_4$-Alkyl$)_2$ steht und der carbocyclische oder weitere heterocyclische Ringe ankondensiert enthalten kann, oder
$R^4$ gemeinsam mit $R^5$, $R^6$, $R^7$ oder $R^8$ und B oder gemeinsam mit A und B einen 5-oder 6-gliedrigen Ring bildet.

Besonders bevorzugt sind Verbindungen der Formel (I), bei welchen
$R^1$, $R^2$ und $R^3$ unabhängig voneinander jeweils für Wasserstoff, Fluor, Chlor, Brom, für Alkyl mit 1 bis 4 Kohlenstoffatomen, fuur gegebenenfalls durch $C_2$-$C_4$-Alkenyl oder $C_1$-$C_2$-Alkoxy substituiertes Alkoxy, Alkylamino, Dialkylamino mit jeweils 1 bis 4 Kohlenstoffatomen in den einzelnen Alkylteilen, wobei im Fall des Dialkylamino die Alkylsubstituenten mit dem Stickstoffatom, an das sie gebunden sind, eine 5-bis 7-gliedrigen heterocyclischen Ring bilden können, für Amino, Alkenylamino oder Dialkenylamino mit jeweils 3 bis 4 Kohlenstoffatomen in den einzelnen Alkenylteilen, für Alkenyl mit 2 bis 6 Kohlenstoffatomen, oder für Trifluormethyl stehen oder
$R^1$ und $R^2$ oder $R^2$ und $R^3$ gemeinsam für einen 5-oder 6-gliedrigen carbocyclischen Ring stehen, mit der Maßgabe, daß mindestens einer der Reste $R^1$, $R^2$ oder $R^3$ für Alkyl mit 1 bis 4 Kohlenstoffatomen, Trifluormethyl oder für einen Teil des ankondensierten 5-oder 6-gliedrigen carbocyclischen Ring steht,
Z für eine (-CH=)-Gruppe oder ein Stickstoffatom steht,
X für Sauerstoff oder Schwefel steht,
Y für Fluor, Chlor, Brom, Iod, Nitro, Amino, für Alkyl, Alkoxy, Halogenalkyl, Alkylcarbonylamino, Alkoxycarbonylamino mit jeweils 1 bis 3 Kohlenstoffatomen im Alkylteil und im Fall des Halogenalkyl mit 1 bis 3 gleichen oder verschiedenen Halogenatomen steht, wobei Halogen für Fluor, Chlor, Brom oder Iod steht, für Phenoxycarbonylamino steht, wobei Y gleich oder verschieden sein kann,
n für eine ganze Zahl 0, 1, 2, 3 oder 4 steht,
A für Sauerstoff, Schwefel, einen Rest $R^5$-N= oder einen Rest $R^6$O-N= steht,

wobei
$R^5$ für Wasserstoff, für Alkyl oder Hydroxyalkyl mit jeweils 1 bis 4 Kohlenstoffatomen, für Alkenyl mit 3 bis 4 Kohlenstoffatomen, für gegebenenfalls einfach bis dreifach, gleich oder verschieden durch Fluor, Chlor, Brom, $C_1$-$C_2$-Alkyl oder $C_1$-$C_2$-Alkoxy substituiertes Cycloalkyl mit 3 bis 6 Kohlenstoffatomen, für jeweils gegebenenfalls einfach bis dreifach, gleich oder verschieden durch Fluor, Chlor, Brom, Iod, $C_1$-$C_2$-Alkyl, $C_1$-$C_2$-Alkoxy, $C_1$-$C_2$-Alkylthio, $C_1$-$C_2$-Alkylsulfinyl, $C_1$-$C_2$-Alkylsulfonyl oder Halogen-$C_1$-$C_2$-alkylsulfonyl substituiertes Phenyl, Benzyl oder Phenethyl steht,
$R^6$ für Wasserstoff oder für Alkyl mit 1 bis 4 Kohlenstoffatomen steht,
B für Sauerstoff, Schwefel oder für einen Rest -N(-)-$R^7$ steht,

wobei
$R^7$ für Wasserstoff, für gegebenenfalls einfach bis dreifach, gleich oder verschieden durch Fluor, Chlor, Brom, Iod, Cyano, Alkenyl mit 3 mit 4 Kohlenstoffatomen oder durch einen Rest -D-$R^9$ substituiertes Alkyl mit 1 mit 4 Kohlenstoffatomen steht,
wobei
D für Sauerstoff, Schwefel oder Sulfonyl steht und
$R^9$ für Wasserstoff, für Alkyl mit 1 bis 4 Kohlenstoffatomen, für Alkenyl mit 3 bis 4 Kohlenstoffatomen, für jeweils gegebenenfalls einfach bis dreifach, gleich oder verschieden substituiertes Phenyl, Benzyl oder Phenethyl, wobei als Phenylsubstituenten Fluor, Chlor, Brom, $C_1$-$C_2$-Alkyl oder $C_1$-$C_2$-Alkoxy ausgewählt sind,
$R^4$ für gegebenenfalls einfach bis fünffach, gleich oder verschieden substituiertes Alkyl mit 1 bis 10 Kohlenstoffatomen, jeweils gegebenenfalls einfach bis fünffach, gleich oder verschieden substituiertes einfach oder mehrfach ungesättigtes Alkenyl oder Alkinyl mit jeweils bis zu 10 Kohlenstoffatomen steht, wobei als Substituenten jeweils ausgewählt sind:
Fluor, Chlor, Cyano, Alkoximino mit 1 bis 4 Kohlenstoffatomen im Alkylteil, gegebenenfalls einfach bis deifach, gleich oder verschieden durch $C_1$-$C_4$-Alkyl substituiertes Cycloalkyl mit 3 bis 6 Kohlenstoffatomen, gegebenenfalls einfach bis dreifach, gleich oder verschieden durch Fluor, Chlor, Brom, Iod, Nitro, Trifluormethyl, $C_1$-$C_2$-Alkyl, $C_1$-$C_2$-Alkoxy, $C_1$-$C_2$-Alkylthio, $C_1$-$C_2$-Alkylsulfinyl oder durch $C_1$-$C_4$-Alkylsulfonyl substituiertes Phenyl, einen 5-oder 6-gliedrigen Heterocyclus der 1 bis 3 Heteroatome aus der Reihe Sauerstoff und/oder Schwefel und/oder Stickstoff enthalten kann, ein Rest -D-$R^9$, ein Rest

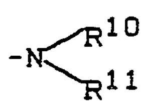 , 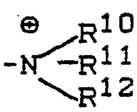 ,

-CO-$OR^{13}$, -CO-$NR^{14}R^{15}$, -CS-$NR^{14}R^{15}$,
wobei
D und $R^9$ die oben angegebene Bedeutung haben,
$R^{10}$ für Wasserstoff oder Alkyl mit 1 bis 4 Kohlenstoffatomen steht,
$R^{11}$ für Alkyl mit 1 bis 4 Kohlenstoffatomen oder Acyl steht,
$R^{10}$ und $R^{11}$ gemeinsam mit dem Stickstoff, an den sie gebunden sind, für einen 5-bis 6-gleidriegen Heterocyclus stehen,
$R^{12}$ für Alkyl mit 1 bis 4 Kohlenstoffatomen steht,
$R^{13}$ für Alkyl mit 1 bis 4 Kohlenstoffatomen steht,
$R^{14}$ und $R^{15}$ jeweils unabhängig voneinander für Wasserstoff oder Alkyl mit 1 bis 6 Kohlenstoffatomen stehen oder
$R^{14}$ und $R^{15}$ gemeinsam mit dem Stickstoff, an den sie gebunden sind, für einen 5-oder 6-gliedrigen Heterocyclus stehen, oder
$R^4$ weiterhin für gegebenenfalls einfach bis dreifach, gleich oder verschieden durch Fluor, Chlor oder $C_1$-$C_4$-Alkyl, $C_1$-$C_4$-Alkoxy, $C_2$-$C_4$-Alkenyl oder Halogen-$C_1$-$C_2$-alkyl, wobei Halogen für 1 bis 3 Fluor-und/oder Chloratome steht, substituiertes Cycloalkyl mit 3 bis 7 Ring-Kohlenstoffatomen steht, für gegebenenfalls einfach bis dreifach, gleich oder verschieden substitiertes Phenyl, wobei als Substituenten ausgewählt sind: Fluor, Chlor, Brom, $C_1$-$C_4$-Alkyl, Trifluormethyl, oder ein Rest -$D^1$-$R^{17}$ steht, wobei
$D^1$ für Sauerstoff, Schwefel, Sulfinyl oder Sulfonyl steht,
$R^{17}$ für Wasserstoff oder $C_1$-$C_4$-Alkyl steht,
$R^4$ weiterhin für einen 5-bis 7-gliedrigen Heteocyclus steht, der ein bis drei gleiche oder verschiedene Heteroatome aus der Reihe Sauerstoff, Stickstoff oder Schwefel enthalten kann und welcher gegebenenfalls

einfach bis dreifach, gleich oder verschieden substituiert ist durch Fluor, Chlor, Brom, Iod, $C_1$-$C_2$-Alkyl, Halogen-$C_1$-$C_2$-alkyl, wobei Halogen für 1 bis 3 gleiche oder verschiedene Fluor-oder Chloratome steht, durch Amino, Alkylamino oder Dialkylamino mit jeweils 1 bis 2 Kohlenstoffatomen in den einzelnen Alkylteilen, durch Nitro, Cyano, oder durch einen $C_1$-$C_4$-Alkoxy-, $C_1$-$C_4$-Alkylthio-oder $C_1$-$C_4$-Alkylsulfonylrest, oder

$R^4$ gemeinsam mit $R^5$, $R^6$, $R^7$ oder $R^8$ und B oder gemeinsam mit A und B einen 5-oder 6-gliedrigen Ring bildet.

Ganz besonders bevorzugt ist die Gruppe von Verbindungen der Formel (I), bei welchen

$R^1$, $R^2$ und $R^3$ unabhängig voneinander jeweils für Wasserstoff, Fluor, Chlor, Brom, für Alkyl mit 1 bis 4 Kohlenstoffatomen, für gegebenenfalls durch $C_2$-$C_4$-Alkenyl oder $C_1$-$C_2$-Alkoxy substituiertes Alkoxy, Alkylamino, Dialkylamino mit jeweils 1 bis 4 Kohlenstoffatomen in den einzelnen Alkylteilen, wobei im Fall des Dialkylamino die Alkylsubstituenten mit dem Stickstoffatom, an das sie gebunden sind, einen 5-bis 7-gliedrigen heterocyclischen Ring bilden können, für Amino, Alkenylamino oder Dialkenylamino mit jeweils 3 bis 4 Kohlenstoffatomen in den einzelnen Alkenylteilen, für Alkenyl mit 2 bis 6 Kohlenstoffatomen, für Trifluormethyl oder

$R^1$ und $R^2$ oder $R^2$ und $R^3$ gemeinsam für einen 5-oder 6-gliedrigen carbocyclischen Ring stehen, mit der Maßgabe, daß mindestens einer der Reste $R^1$, $R^2$ oder $R^3$ für Alkyl mit 1 bis 4 Kohlenstoffatomen, Trifluormethyl oder für einen Teil des ankondensierten 5-oder 6-gliedrigen carbocyclischen Ring steht,

Z für ein Stickstoffatom steht,

X für Sauerstoff oder Schwefel steht,

Y für Fluor, Chlor, Brom, Iod, Nitro, Amino, für Alkyl, Alkoxy, Halogenalkyl, Alkylcarbonylamino, Alkoxycarbonylamino mit jeweils 1 bis 3 Kohlenstoffatomen im Alkylteil und im Fall des Halogenalkyl mit 1 bis 3 Halogenatomen steht, wobei Halogen für Fluor, Chlor, Brom und/oder Iod steht, oder für Phenoxycarbonylamino steht, wobei Y gleich oder verschieden sein kann,

n für eine ganze Zahl 0, 1, 2 oder 3 steht,

A für Sauerstoff oder Schwefel steht,

B für Sauerstoff, Schwefel oder für einen Rest $-\underset{|}{N}-R^7$ steht,

wobei

$R^7$ für Wasserstoff, für gegebenenfalls einfach bis dreifach, gleich oder verschieden durch Fluor, Chlor, Brom, Iod, Cyano oder Alkenyl mit 3 bis 4 Kohlenstoffatomen substituiertes Alkyl mit 1 bis 4 Kohlenstoffatomen steht,

$R^4$ für jeweils gegebenenfalls einfach bis fünffach, gleich oder verschieden substituiertes Alkyl mit 1 bis 10 Kohlenstoffatomen, jeweils einfach oder mehrfach ungesättigtes Alkenyl oder Alkinyl mit jeweils bis zu 10 Kohlenstoffatomen steht, wobei als Substituenten jeweils ausgewählt sind:

Fluor, Chlor, Cyano, Alkoximino mit 1 bis 4 Kohlenstoffatomen im Alkylteil, gegebenenfalls durch $C_1$-$C_4$-Alkyl substituiertes Cycloalkyl mit 3 bis 6 Kohlenstoffatomen, gegebenenfalls durch Fluor, Chlor, Brom, Iod, Nitro, Trifluormethyl, $C_1$-$C_2$-Alkyl, $C_1$-$C_2$-Alkoxy, $C_1$-$C_2$-Alkylthio, $C_1$-$C_2$-Alkylsulfinyl oder durch $C_1$-$C_4$-Alkylsulfonyl substituiertes Phenyl, einen 5-oder 6-gliedrigen Heterocyclus der 1 bis 3 Heteroatome aus der Reihe Sauerstoff und/oder Schwefel und/oder Stickstoff enthalten kann, ein Rest -D-$R^9$, ein Rest

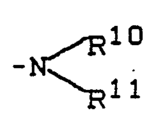 , 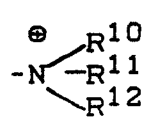 ,

-CO-$OR^{13}$, -CO-$NR^{14}R^{15}$, -CS-$NR^{14}R^{15}$,

wobei

D für Sauerstoff, Schwefel oder Sulfonyl steht und

$R^9$ für Wasserstoff, für Alkyl mit 1 bis 4 Kohlenstoffatomen, für Alkenyl mit 3 bis 4 Kohlenstoffatomen, für jeweils gegebenenfalls einfach bis dreifach, gleich oder verschieden substituiertes Phenyl, Benzyl oder Phenethyl, wobei als Phenylsubstituenten Fluor, Chlor, Brom, $C_1$-$C_2$-Alkyl oder $C_1$-$C_2$-Alkoxy ausgewählt sind,

$R^{10}$ für Wasserstoff oder Alkyl mit 1 bis 4 Kohlenstoffatomen steht,

$R^{11}$ für Alkyl mit 1 bis 4 Kohlenstoffatomen oder Acyl steht,

$R^{10}$ und $R^{11}$ gemeinsam mit dem Stickstoff, an den sie gebunden sind, für einen 5-bis 6-gleidriegen Heterocyclus stehen,

$R^{12}$ für Alkyl mit 1 bis 4 Kohlenstoffatomen steht,

$R^{13}$ für Alkyl mit 1 bis 4 Kohlenstoffatomen steht,

$R^{14}$ und $R^{15}$ jeweils unabhängig voneinander für Wasserstoff oder Alkyl mit 1 bis 6 Kohlenstoffatomen stehen oder

$R^{14}$ und $R^{15}$ gemeinsam mit dem Stickstoff, an den sie gebunden sind, für einen 5-oder 6-gliedrigen Heterocyclus stehen, oder

$R^4$ weiterhin für gegebenenfalls einfach bis dreifach, gleich oder verschieden durch Fluor, Chlor oder $C_1$-$C_4$-Alkyl, $C_1$-$C_4$-Alkoxy, $C_2$-$C_4$-Alkenyl oder Halogen-$C_1$-$C_2$-alkyl, wobei Halogen für 1 bis 3 Fluor-und/oder Chloratome steht, substituiertes Cycloalkyl mit 3 bis 6 Kohlenstoffatomen steht, für Benzyl, Phenethyl, für gegebenenfalls einfach bis dreifach, gleich oder verschieden substituiertes Phenyl, wobei als Substituenten ausgewählt sind: Fluor, Chlor, Brom, $C_1$-$C_4$-Alkyl oder Trifluormethyl, oder ein Rest -$D^1$-$R^{17}$ steht, wobei

$D^1$ für Sauerstoff steht,

$R^{17}$ für Wasserstoff oder $C_1$-$C_4$-Alkyl steht,

$R^4$ weiterhin für einen 5-bis 7-gliedrigen Heteocyclus steht, der ein bis drei gleiche oder verschiedene Heteroatome aus der Reihe Sauerstoff, Stickstoff oder Schwefel enthalten kann und welcher gegebenenfalls einfach bis dreifach, gleich oder verschieden substituiert ist durch Fluor, Chlor, Brom, Iod, $C_1$-$C_2$-Alkyl, Halogen-$C_1$-$C_2$-alkyl, wobei Halogen für 1 bis 3 gleiche oder verschiedene Fluor-oder Chloratome steht, durch Amino, Alkylamino oder Dialkylamino mit jeweils 1 bis 2 Kohlenstoffatomen in den einzelnen Alkylteilen, durch Nitro, Cyano, $C_1$-$C_4$-Alkoxy-, $C_1$-$C_4$-Alkylthio oder $C_1$-$C_4$-Alkylsulfonyl, oder

$R^4$ gemeinsam mit $R^5$, $R^6$, $R^7$ oder $R^8$ und B oder gemeinsam mit A und B einen 5-oder 6-gliedrigen Ring bildet.

Als insbesondere bevorzugte Gruppe von Verbindungen der Formel (I) sind die Verbindungen, in denen $R^1$, $R^2$, $R^3$, Z, X, Y, n, B, $R^7$, $R^4$, D, $R^9$, $R^{10}$, $R^{11}$, $R^{12}$, $R^{13}$, $R^{14}$, $R^{15}$, $D^1$ und $R^{17}$ die oben angegebene ganz besonders bevorzugte Definition haben und A für Sauerstoff steht.

Eine weitere ganz besonders bevorzugte Gruppe von Verbindungen der Formel (I) sind diejenigen, bei welchen

$R^1$, $R^2$ und $R^3$ unabhängig voneinander jeweils für Wasserstoff, Fluor, Chlor, Brom, für Alkyl mit 1 bis 4 Kohlenstoffatomen, für gegebenenfalls durch $C_2$-$C_4$-Alkenyl oder $C_1$-$C_2$-Alkoxy substituiertes Alkoxy, Alkylamino, Dialkylamino mit jeweils 1 bis 4 Kohlenstoffatomen in den einzelnen Alkylteilen, wobei im Fall des Dialkylamino die Alkylsubstituenten mit dem Stickstoffatom, an das sie gebunden sind, einen 5-bis 7-gliedrigen heterocyclischen Ring bilden können, für Amino, Alkenylamino oder Dialkenylamino mit jeweils 3 bis 4 Kohlenstoffatomen in den einzelnen Alkenylteilen, für Alkenyl mit 2 bis 6 Kohlenstoffatomen oder für Trifluormethyl stehen oder

$R^1$ und $R^2$ oder $R^2$ und $R^3$ gemeinsam für einen 5-oder 6-gliedrigen carbocyclischen Ring stehen, mit der Maßgabe, daß mindestens einer der Reste $R^1$,

$R^2$ oder $R^3$ für Alkyl mit 1 bis 4 Kohlenstoffatomen, Trifluormethyl oder für einen Teil des ankondensierten 5-oder 6-gliedrigen carbocyclischen Ring steht,

Z für ein Stickstoffatom steht,

X für Sauerstoff oder Schwefel steht,

Y für Fluor, Chlor, Brom, Iod, Nitro, Amino, für Alkyl, Alkoxy, Halogenalkyl, Alkylcarbonylamino, Alkoxycarbonylamino mit jeweils 1 bis 3 Kohlenstoffatomen in den Alkylteilen und im Fall des Halogenalkyl mit 1 bis 3 Halogenatomen steht, wobei Halogen für Fluor, Chlor, Brom und/oder Iod steht, für Phenoxycarbonylamino steht, wobei Y gleich oder verschieden sein kann,

n für eine ganze Zahl 0, 1, 2 oder 3 steht,

A für einen Rest $R^5$-N= oder einen Rest $R^6$O-N= steht,

wobei

$R^5$ für Wasserstoff, für Alkyl oder Hydroxyalkyl mit jeweils 1 bis 4 Kohlenstoffatomen oder für Alkenyl mit 3 bis 4 Kohlenstoffatomen steht,

$R^6$ für Wasserstoff oder für Alkyl mit 1 bis 4 Kohlenstoffatomen steht,

B für Sauerstoff, Schwefel oder für einen Rest -N(-)-$R^7$ steht,

wobei

$R^7$ für Wasserstoff, für gegebenenfalls einfach bis dreifach, gleich oder verschieden durch Fluor; Chlor, Brom, Iod, Cyano oder Alkenyl mit 3 mit 4 Kohlenstoffatomen substituiertes Alkyl mit 1 bis 4 Kohlenstoffatomen steht,

$R^4$ für jeweils gegebenenfalls einfach bis fünffach, gleich oder verschieden substituiertes Alkyl mit 1 bis 10 Kohlenstoffatomen, jeweils einfach oder mehrfach ungesättigtes Alkenyl oder Alkinyl mit jeweils bis zu 10 Kohlenstoffatomen steht, wobei als Substituenten jeweils ausgewählt sind:

Fluor, Chlor, Cyano, Alkoximino mit 1 bis 4 Kohlenstoffatomen im Alkylteil, gegebenenfalls einfach bis

dreifach gleich oder verschieden durch $C_1$-$C_4$-Alkyl substituiertes Cycloalkyl mit 3 bis 6 Kohlenstoffatomen, gegebenenfalls einfach bis dreifach, gleich oder verschieden durch Fluor, Chlor, Brom, Iod, Nitro, Trifluormethyl, $C_1$-$C_2$-Alkyl, $C_1$-$C_2$-Alkoxy, $C_1$-$C_2$-Alkylthio, $C_1$-$C_2$-Alkylsulfinyl oder durch $C_1$-$C_4$-Alkylsulfonyl substituiertes Phenyl, einen 5-oder 6-gliedrigen Heterocyclus der 1 bis 3 Heteroatome aus der Reihe Sauerstoff und/oder Schwefel und/oder Stickstoff enthalten kann, ein Rest -D-$R^9$, wobei

D für Sauerstoff, Schwefel oder Sulfonyl steht und

$R^9$ für Wasserstoff, für Alkyl mit 1 bis 4 Kohlenstoffatomen, für Alkenyl mit 3 bis 4 Kohlenstoffatomen, für jeweils gegebenenfalls einfach bis dreifach, gleich oder verschieden substituiertes Phenyl, Benzyl oder Phenethyl steht, wobei als Phenylsubstituenten Fluor, Chlor, Brom, $C_1$-$C_2$-Alkyl oder $C_1$-$C_2$-Alkoxy ausgewählt sind,

$R^4$ weiterhin für gegebenenfalls einfach bis dreifach, gleich oder verschieden durch Fluor, Chlor oder $C_1$-$C_4$-Alkyl substituiertes Cycloalkyl mit 3 bis 6 Kohlenstoffatomen steht, für gegebenenfalls einfach bis dreifach, gleich oder verschieden substituiertes Phenyl steht, wobei als Substituenten ausgewählt sind: Fluor, Chlor, Brom, $C_1$-$C_4$-Alkyl, Trifluormethyl oder ein Rest -$D^1$-$R^{17}$ steht, wobei

$D^1$ für Sauerstoff steht,

$R^{17}$ für Wasserstoff oder $C_1$-$C_4$-Alkyl steht,

$R^4$ weiterhin für einen 5-bis 7-gliedrigen Heterocyclus steht, der ein bis drei gleiche oder verschiedene Heteroatome aus der Reihe Sauerstoff, Stickstoff oder Schwefel enthalten kann und welcher gegebenenfalls einfach bis dreifach, gleich oder verschieden substituiert ist durch Fluor, Chlor, Brom, Iod, $C_1$-$C_2$-Alkyl, Halogen-$C_1$-$C_2$-alkyl, wobei Halogen für 1 bis 3 gleiche oder verschiedene Fluor-oder Chloratome steht, durch Amino, Alkylamino oder Dialkylamino mit jeweils 1 bis 2 Kohlenstoffatomen in den einzelnen Alkylteilen, durch Nitro, Cyano, $C_1$-$C_4$-Alkoxy, $C_1$-$C_4$-Alkylthio oder $C_1$-$C_4$-Alkylsulfonyl, oder

$R^4$ gemeinsam mit $R^5$, $R^6$, $R^7$ oder $R^8$ und B oder gemeinsam mit A und B einen 5-oder 6-gliedrigen Ring bildet.

Eine andere ganz besonders bevorzugte Gruppe von Verbindungen der Formel (I) sind diejenigen, bei welchen

Z für eine (-CH=)-Gruppe steht,

$R^1$, $R^2$, und $R^3$, unabhängig voneinander jeweils für Wasserstoff, Methyl, Ethyl, Fluor, Chlor, Brom, Amino oder Methylamino stehen,

X für Schwefel steht,

Y für Fluor, Chlor, Brom, Methyl oder Methoxy steht,

n für 0, 1 oder 2 steht,

A für Sauerstoff oder Schwefel steht,

B für Sauerstoff oder einen Rest $-\underset{|}{N}-R^7$ steht, wobei

$R^7$ für Wasserstoff, Methyl oder Ethyl steht und

$R^4$ für $C_1$-bis $C_6$-Alkyl steht.

Besonders hervorzuheben sind die Verbindungen der Formel (I), bei welchen

Z für Stickstoff steht,

$R^1$, $R^2$ und $R^3$ unabhängig voneinander jeweils für Wasserstoff, Methyl, Ethyl, Chlor, Amino oder Methylamino stehen,

X für Schwefel steht,

Y für Fluor, Chlor, Amino, Nitro, Methyl oder Methoxy steht,

n für 0, 1 oder 2 steht,

A für Sauerstoff oder Schwefel steht,

B für Sauerstoff oder einen Rest $-\underset{|}{N}-R^7$ steht, wobei

wobei $R^7$ für Wasserstoff oder Methyl steht und

$R^4$ für gegebenenfalls einfach bis fünffach, gleich oder verschieden substituiertes Alkyl mit 1 bis 8 Kohlenstoffatomen steht, wobei als Substituenten ausgewählt sind: Fluor, Chlor, Trifluormethyl, Methoxy und Cyano; $R^4$ weiterhin für jeweils gegebenenfalls einfach bis dreifach, gleich oder verschieden durch Fluor und Chlor substituiertes Alkenyl oder Alkinyl mit jeweils 2 bis 6 Kohlenstoffatomen steht, jeweils gegebenenfalls durch Methyl oder Ethyl substituiertes Cyclopropyl, Cyclopentyl oder Cyclohexyl steht.

Verwendet man beispielsweise 4-(4,6-Dimethylpyrimidyl-2-oxy)-benzoylchlorid und tert. Butylamin als Ausgangsstoffe, so läßt sich der Reaktionsverlauf des erfindungsgemäßen Verfahrens (A-$a_1$) durch das folgende Formelschema darstellen:

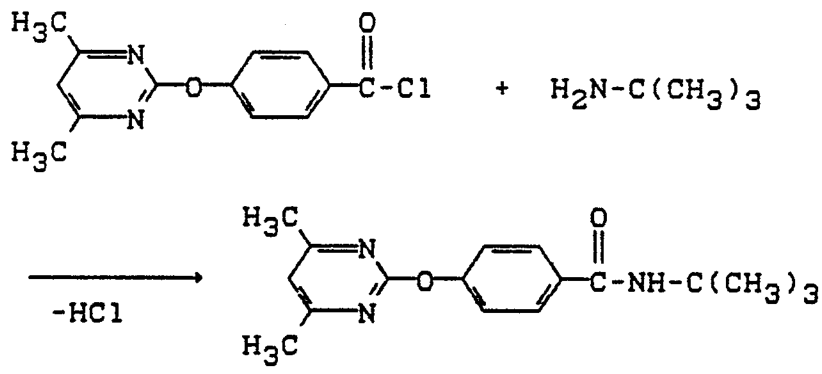


Verwendet man beispielsweise das gemischte Anhydrid aus 4-(4-Methyl-pyrimidyl-2-mercapto)-benzoesäure und Kohlensäureethylester sowie 1,1-Dimethyl-2-hydroxyethylamin oder O-Methyl-N-isopropylhydroxylamin als Ausgangsstoffe, so läßt sich der Reaktionsverlauf des erfindungsgemäßen Verfahrens (A-$a_1$) durch das folgende Formelschema darstellen:

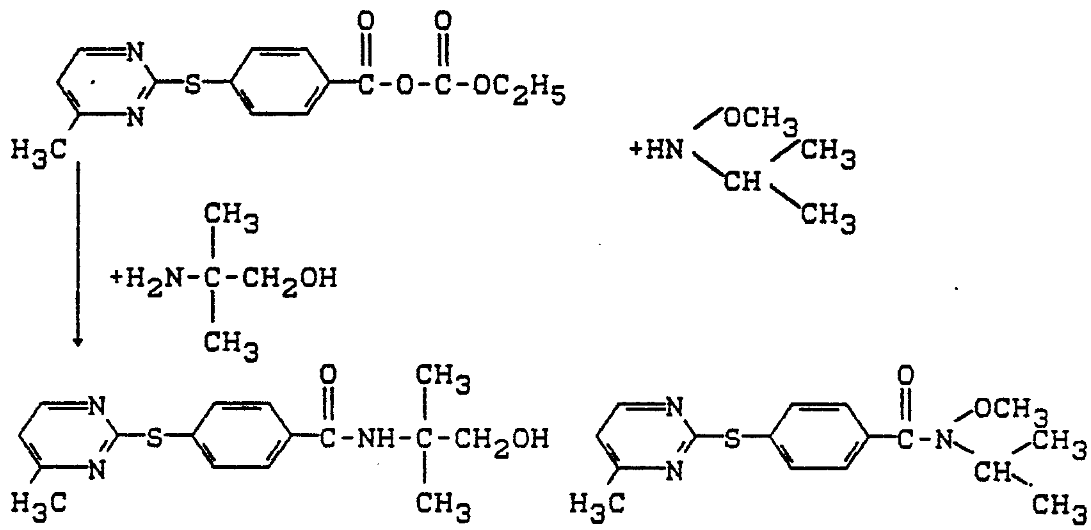

Verwendet man beispielsweise das gemischte Anhydrid aus 4-(6-Methyl-pyridyl-2-mercapto)-benzoesäure und p-Toluolsulfonsäure und tert. Butanol als Ausgangsstoffe, so läßt sich der Verlauf des erfindungsgemäßen Verfahrens (A-$a_1$) durch das folgende Formelschema darstellen:

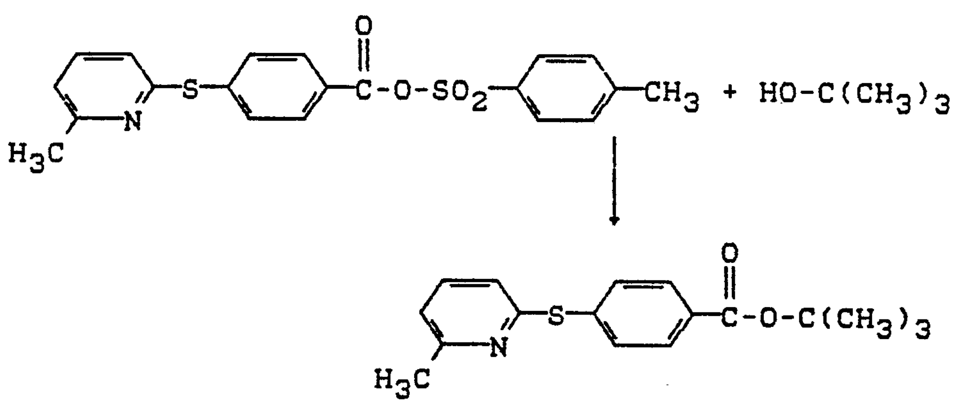

Verwendet man beispielsweise 4-(4,5-Dimethyl-pyrimidyl-2-mercapto)-benzoyl-imidazol und tert. Butanthiol als Ausgangsstoffe, so läßt sich der Reaktionsverlauf des erfindungsgemäßen Verfahrens (A-$a_1$) durch das folgende Formelschema darstellen:

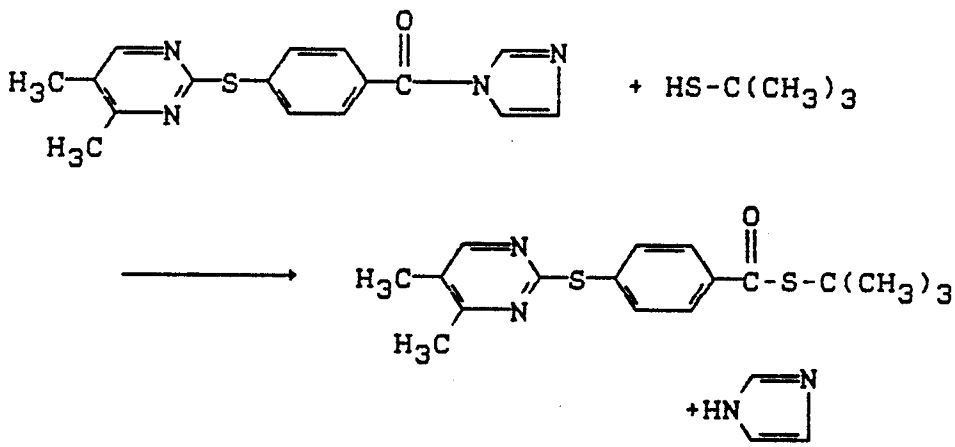

Verwendet man beispielsweise 4-(4,6-Dimethyl-pyrimidyl-2-mercapto)-thiobenzoylchlorid und Acetoncyanhydrin als Ausgangsstoffe, so läßt sich der Reaktionsverlauf des erfindungsgemäßen Verfahrens (A-$a_2$) durch das folgende Formelschema darstellen:

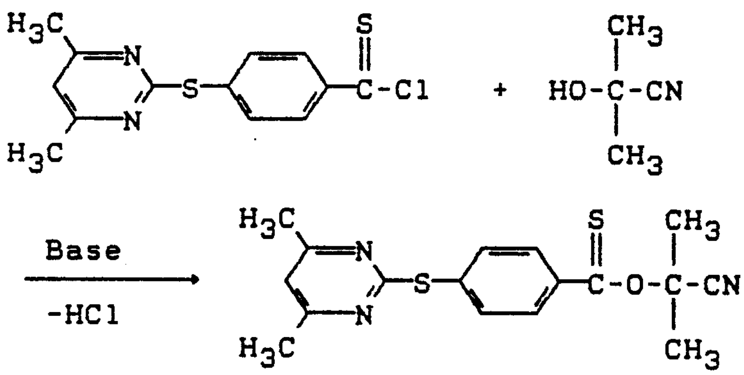


Verwendet man beispielsweise 4-(4,6-Dimethyl-pyrimidyl-2-oxy)-dithiobenzoesäure und Isopropylbromid als Ausgangsstoffe, so läßt sich der Reaktionsverlauf des erfindungsgemäßen Verfahrens ($a_3$-$\alpha$) durch das folgende Formelschema darstellen:

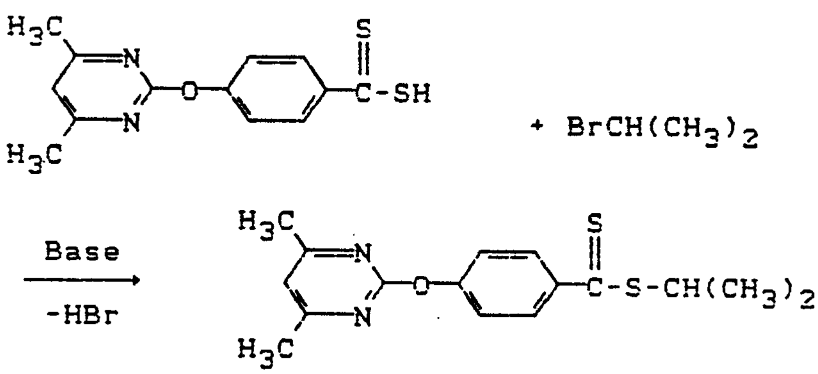


Verwendet man beispielsweise 4-(4,6-Dimethyl-pyrimidyl-2-oxy)-dithiobenzoesäure und Acrylnitril als Ausgangsstoffe, so läßt sich der Reaktionsverlauf des erfindungsgemäßen Verfahrens ($a_3$-$\beta$) durch das folgende Formelschema darstellen:

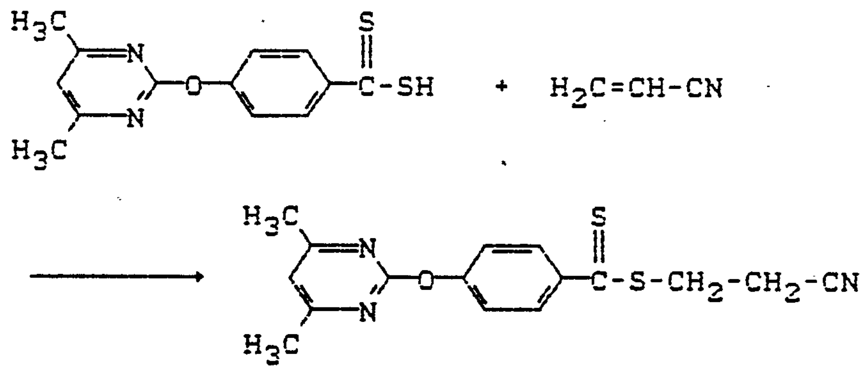

Verwendet man beispielsweise 4-(4,6-Dimethyl-pyrimidyl-2-oxy)-dithiobenzoesäure und 3-Chloranilin als Ausgangsstoffe, so läßt sich der Reaktionsverlauf des erfindungsgemäßen Verfahrens ($a_4$-α) durch das folgende Formelschema darstellen:

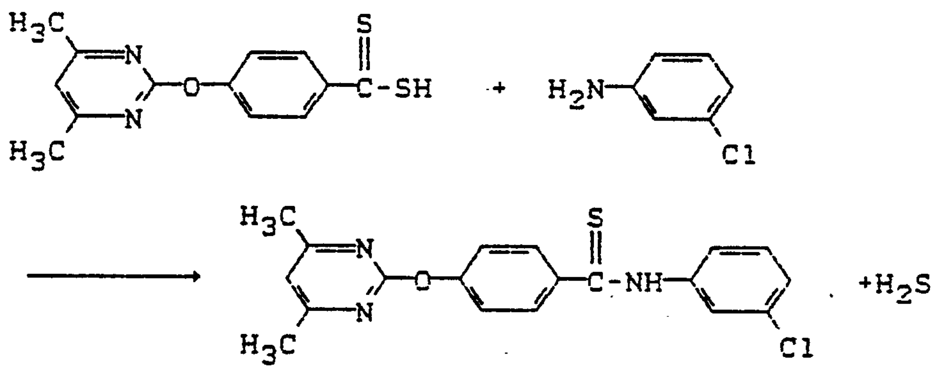

Verwendet man beispielsweise 4-(4,6-Dimethyl-pyrimidyl-2-mercapto)-N-methyl-benzimidchlorid und Schwefelwasserstoff als Ausgangsstoffe, so läßt sich der Reaktionsverlauf des erfindungsgemäßen Verfahrens ($a_4$-β) durch das folgende Formelschema darstellen:

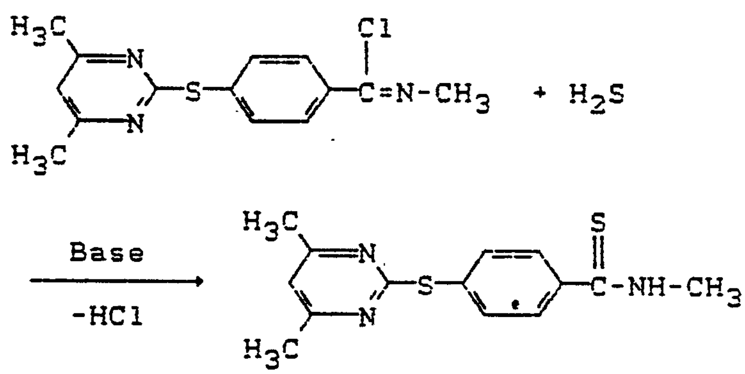


Verwendet man beispielsweise 4-(4,6-Dimethyl-pyrimidyl-2-mercapto)-benzoesäure-tert.-butyl-amid und das Lawesson-Reagenz 2,4-Bis-(4-methoxy-phenyl)-2,4-dithiono-1,2,3,4-dithiadiphosphetan als Ausgangsstoffe, so läßt sich der Reaktionsverlauf des erfindungsgemäßen Verfahrens ($a_4$-γ) durch das folgende Formelschema darstellen:

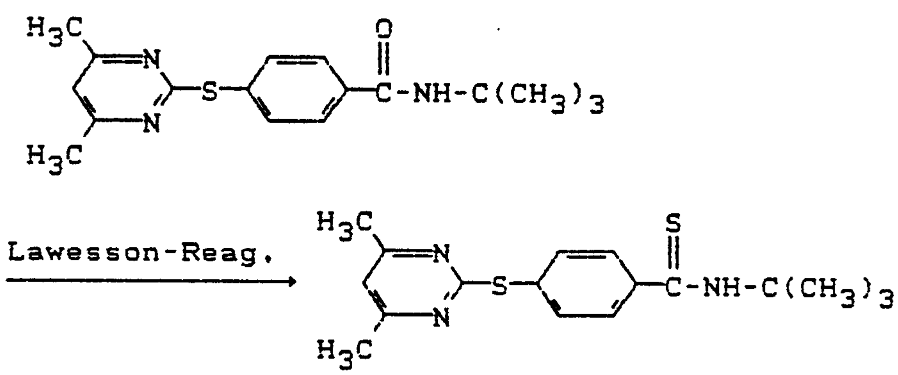
Lawesson-Reag.

Verwendet man beispielsweise 4-(4,6-Dimethyl-pyrimidyl-2-mercapto)-benzoesäure-N-tert.-butyl-imidchlorid und Ethanol bzw. Ethanthiol bzw. Dimethylamin bzw. N,O-Dimethylhydroxylamin als Ausgangsstoffe, so lassen sich die Reaktionsverläufe des erfindungsgemäßen Verfahrens ($a_5$-α) durch das folgende Formelschema darstellen:

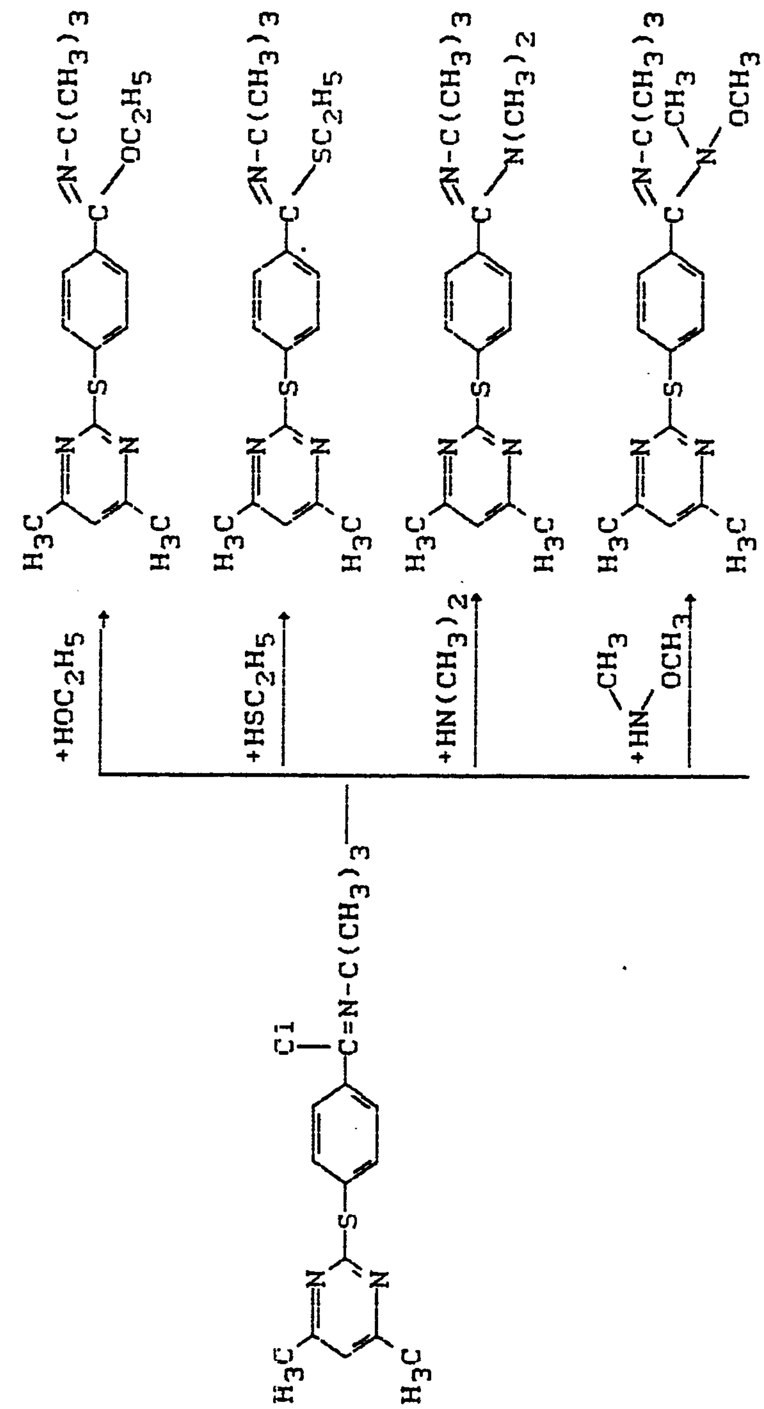


Verwendet man beispielsweise 4-(4,6-Dimethyl-pyrimidyl-2-mercapto)-thiobenzoesäure-tert.-butylamid und Dimethylsulfat als Ausgangsstoffe, so läßt sich der Reaktionsverlauf des erfindungsgemäßen Verfahrens ($a_5$-β) durch das folgende Formelschema darstellen:

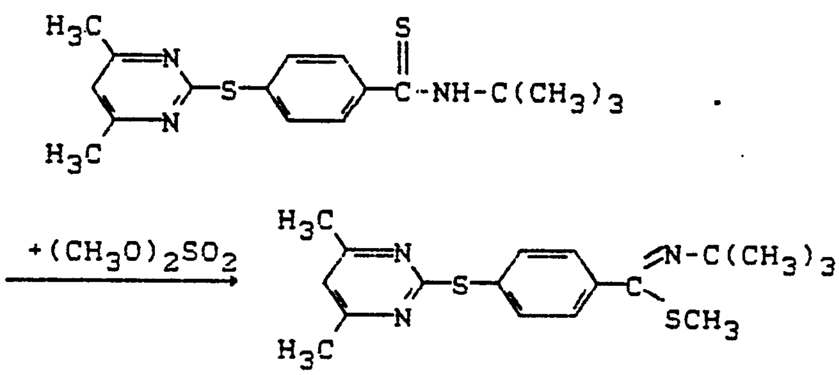
+(CH3O)2SO2

Verwendet man beispielsweise 4-(4,6-Dimethyl-pyrimidyl-2-mercapto)-benzoesäure-methoximid-chlorid und tert.-Butanol bzw. tert.-Butanthiol bzw. tert.-Butylamin als Ausgangsstoffe, so lassen sich die Reaktionsverläufe des erfindungsgemäßen Verfahrens ($a_6$-α) durch das folgende Formelschema darstellen:

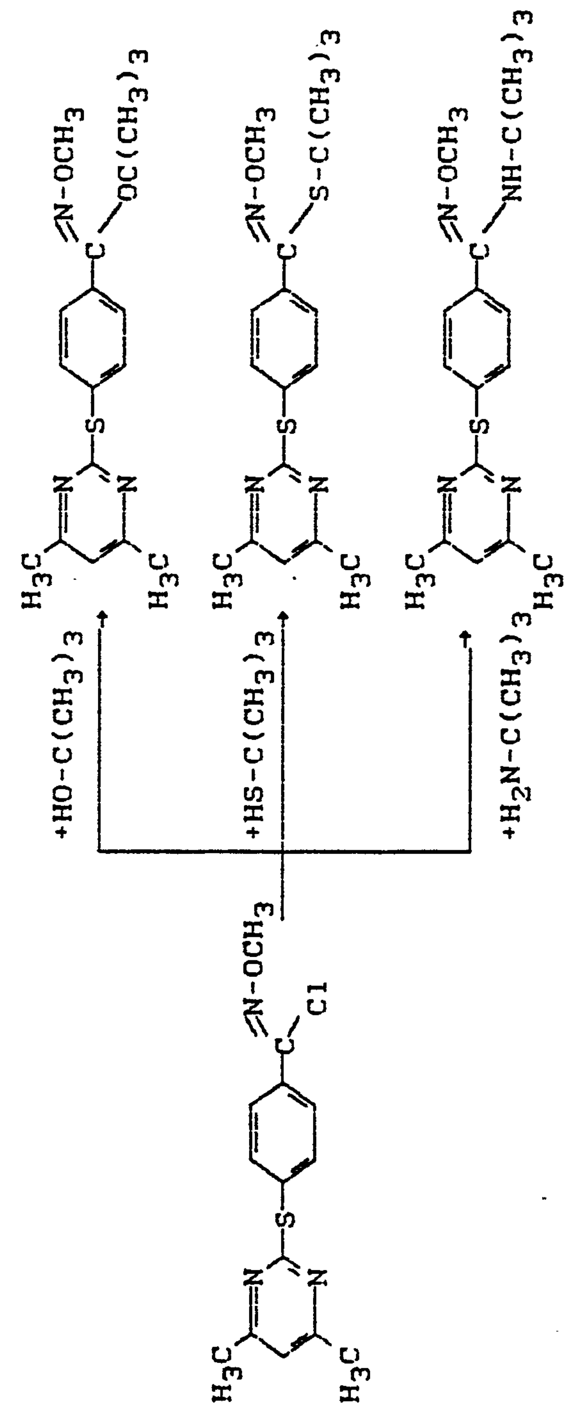


Verwendet man beispielsweise 4-(4,6-Dimethyl-pyrimidyl-2-mercapto)-benzhydroxamsäure-O-methylester und Allylbromid als Ausgangsstoffe, so läßt sich der Reaktionsverlauf des erfindungsgemäßen Verfahrens ($a_6$-β) durch das folgende Formelschema darstellen:

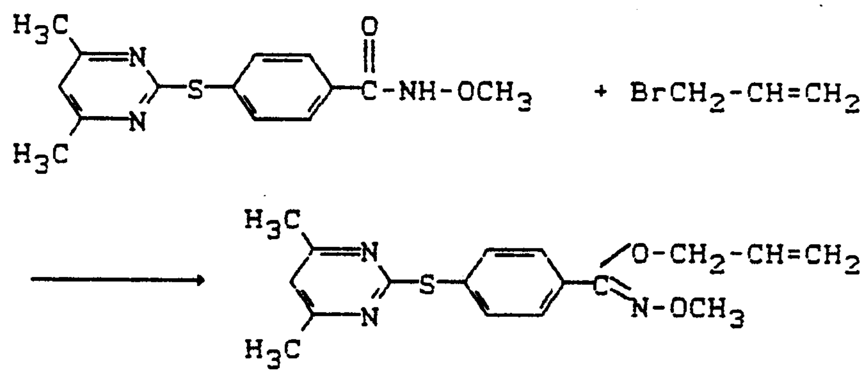

Verwendet man beispielsweise 4-(4,6-Dimethyl-pyrimidyl-2-mercapto)-thiobenzoesäure-O-methylester und O-methylhydroxylamin als Ausgangsstoffe, so läßt sich der Reaktionsverlauf des erfindungsgemäßen Verfahrens $(a_6-\gamma)$ durch das folgende Formelschema darstellen:

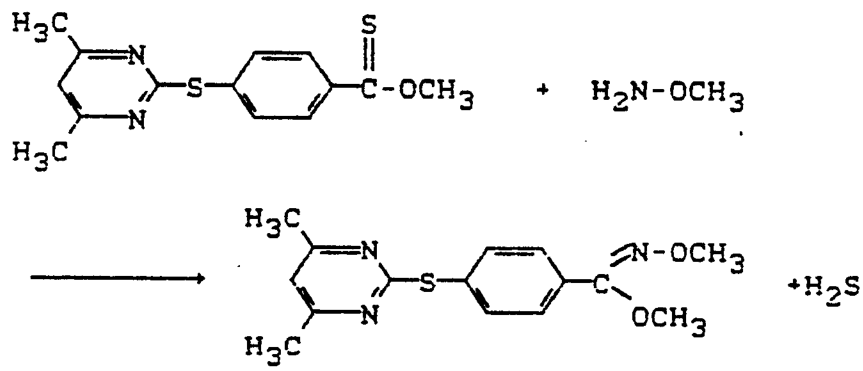

Verwendet man beispielsweise 4-(4,6-Dimethyl-pyrimidyl-2-mercapto)-benzonitril und 2-Methyl-2-amino-propanol als Ausgangsstoffe, so läßt sich der Reaktionsverlauf des erfindungsgemäßen Verfahrens $(a_7-\alpha)$ durch das folgende Formelschema darstellen:

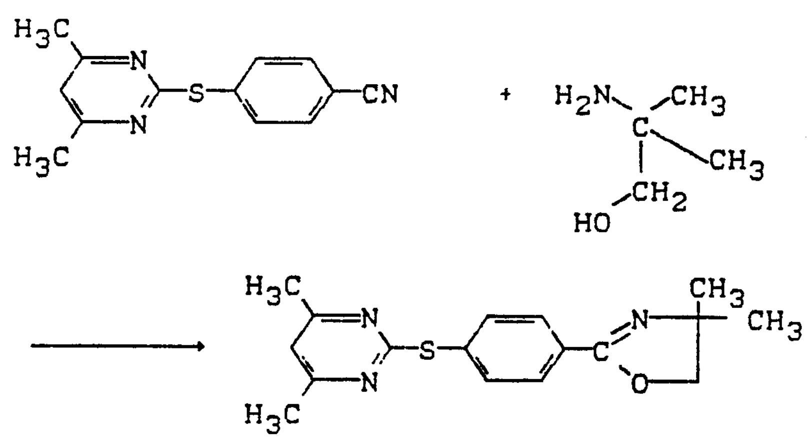

Verwendet man beispielsweise 4-(4,6-Dimethyl-pyrimidyl-2-mercapto)-benzoesäurethioamid und Brompinakolin als Ausgangsstoffe, so läßt sich der Reaktionsverlauf des erfindungsgemäßen Verfahrens $(a_7-\beta)$ durch das folgende Formelschema darstellen:

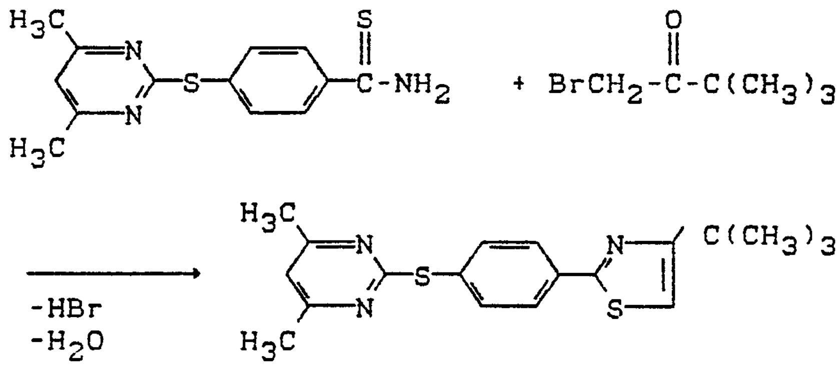


Verwendet man beispielsweise 4-(4,6-Dimethyl-pyrimidyl-2-mercapto)-benzhydroxamsäure und 1,2-Dibromethan als Ausgangsstoffe, so läßt sich der Reaktionsverlauf des erfindungsgemäßen Verfahrens ($a_1$-γ) durch das folgende Formelschema darstellen:

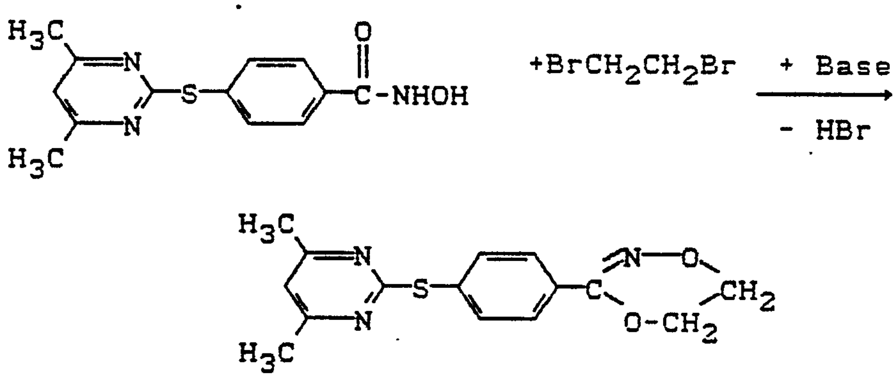


Verwendet man beispielsweise 4-(4,6-Dimethyl-pyrimidyl-2-mercapto)-benzamidoxim und Pivalsäurechlorid als Ausgangsstoffe, so läßt sich der Reaktionsverlauf des erfindungsgemäßen Verfahrens ($a_1$-δ) durch das folgende Formelschema darstellen:

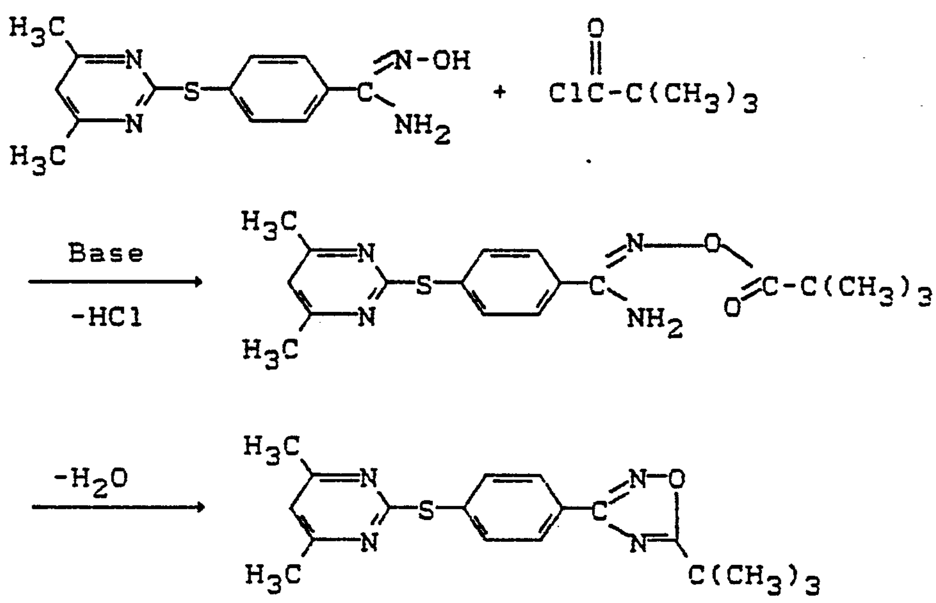


Verwendet man beispielsweise 2-Chlor-4,6-dimethyl-pyrimidin und 4-Mercapto-benzoesäure-tert.-butylamid als Ausgangsstoffe, so läßt sich der Reaktionsverlauf des erfindungsgemäßen Verfahrens (B-$b_1$) durch das folgende Formelschema darstellen:

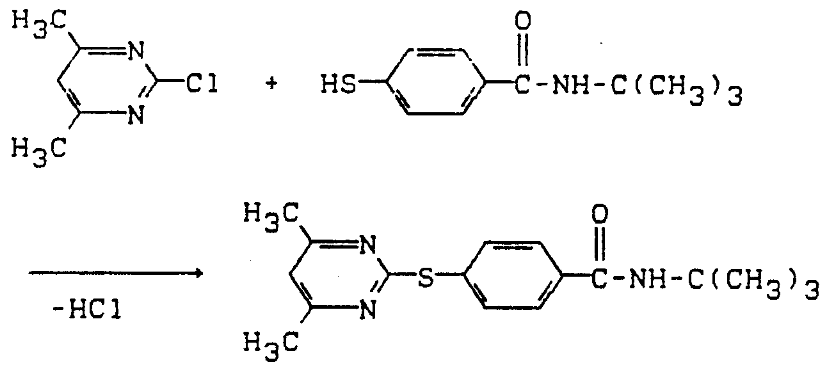


Verwendet man beispielsweise 2-Mercapto-4,6-dimethyl-pyrimidin und 4-Chlor-3-nitro-benzoesäure-tert.-butylamid als Ausgangsstoffe, so läßt sich der Reaktionsverlauf des erfindungsgemäßen Verfahrens (B-$b_2$) durch das folgende Formelschema darstellen:

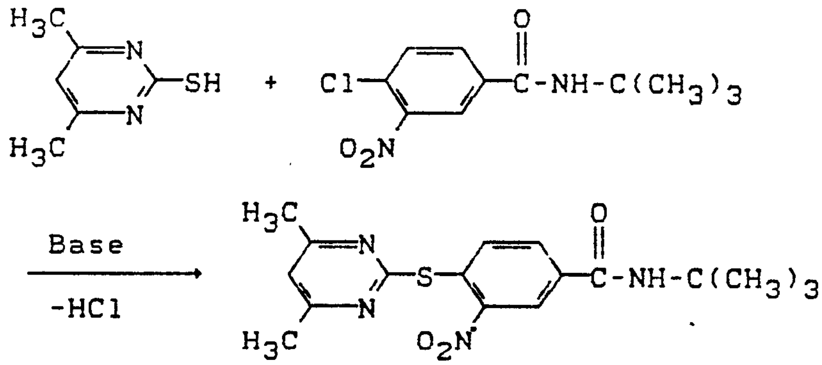


Verwendet man beispielsweise 4-(4,6-Dimethyl-pyrimidyl-2-mercapto)-3-methyl-benzoesäure-tert.-butylamid und Chlor als Ausgangsstoffe, so läßt sich der Reaktionsverlauf des erfindungsgemäßen Verfahrens -(C-$c_1$) durch das folgende Formelschema darstellen:

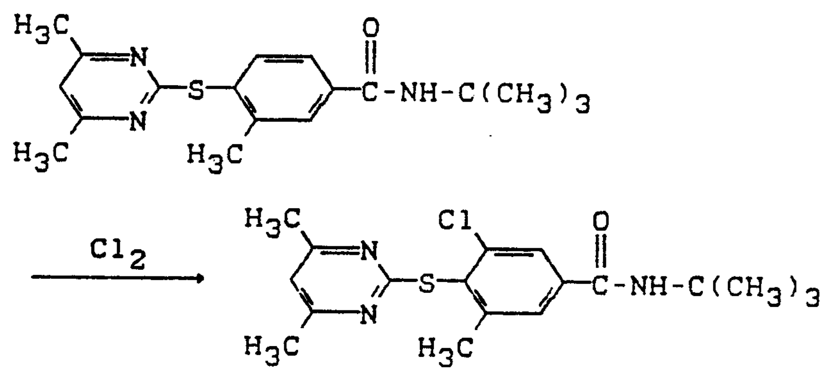


Reduziert man beispielsweise 4-(4,6-Dimethyl-pyrimidyl-2-mercapto)-3-nitro-benzoesäure-tert.-butylamid mit Wasserstoff zu der entsprechenden Amino-Verbindung und setzt diese mit Acetylchlorid bzw. salpetriger Säure und Kupferchlorid um, so lassen sich die Reaktionsverläufe der erfindungsgemäßen Verfahren -(C-$c_2$), (C-$c_3$) und (C-$c_4$)) durch das folgende Formelschema darstellen:

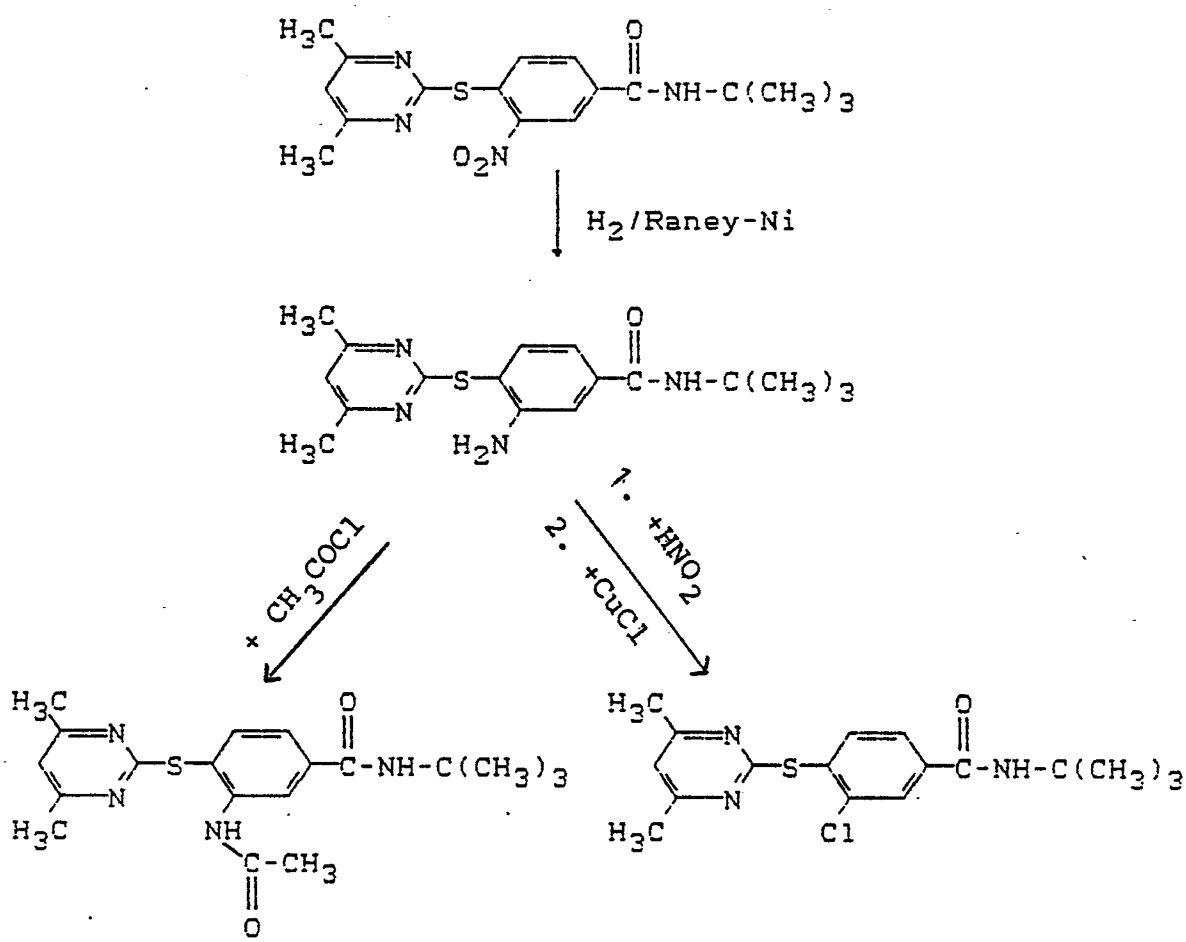


Verwendet man beispielsweise 4-(4-Chlor-6-methyl-pyrimidyl-2-mercapto)-benzoesäure-tert.-butylamid und Ammoniak als Ausgangsstoffe, so läßt sich der Reaktionsverlauf des erfindungsgemäßen Verfahrens -(D) durch das folgende Formelschema darstellen:

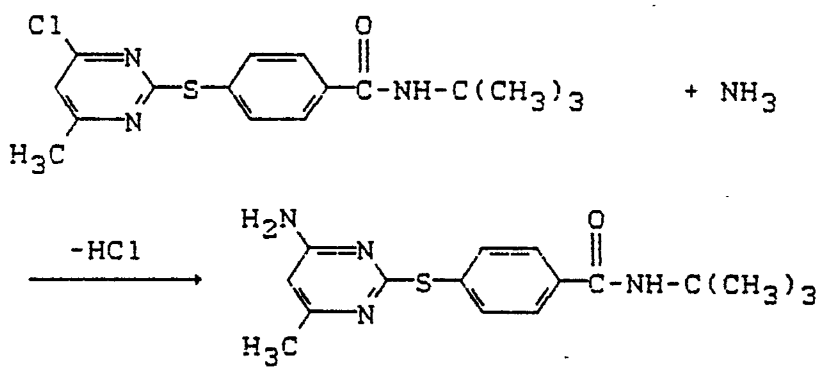


Die zur Durchführung des erfindungsgemäßen Verfahrens (A-$a_1$) benötigten Benzoesäure-Derivate sind durch die Formel (II) allgemein definiert.

In dieser Formel (II) stehen $R^1$, $R^2$, $R^3$, Z, X, Y und n vorzugsweise bzw. besonders bevorzugt für diejenigen Reste, die bereits im Zusammenhang mit der Beschreibung der erfindungsgemäßen Stoffe der Formel (I) als bevorugt bzw. besonders bevorzugt für diese Substituenten genannt wurden. Ganz besonders bevorzugt stehen die obengenannten Substituenten der Formel (II) für die als ganz besonders bevorzugt aufgezählten entsprechenden Reste der Formel (I). G steht vorzugsweise für Chlor, $C_1$-$C_2$-Alkoxycarbonyloxy, für Toluolsulfonyloxy oder Benzolsulfonyloxy.

Die Ausgangsverbindungen der Formel (II) sind noch nicht bekannt. Man erhält sie, wenn man Carbonsäure-Derivate der Formel (XXV)

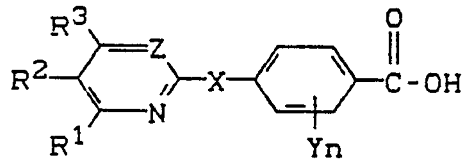

(XXV)

in welcher
$R^1$, $R^2$, $R^3$, Z, X, Y und n die oben angegebene Bedeutung haben,
oder deren Metallsalze, insbesondere deren Alkali-oder Erdalkalisalze oder deren tert.-Ammoniumsalze mit anorganischen Säurehalogeniden, insbesondere Phosgen, Thionylchlorid, Phosphoroxychlorid oder Phosphortrichlorid oder mit Chlorkohlensäurealkylestern, insbesondere Chlorkohlensäuremethyl-oder -ethylester oder mit Arylsulfonsäurechloriden, insbesondere Benzol-oder Toluolsulfonsäurechlorid,
oder mit Imidazol oder einem reaktiven Derivat davon,
in üblicher Art und Weise gegebenenfalls in Gegenwart eines Verdünnungsmittels wie beispielsweise Tetrahydrofuran und gegebenenfalls in Gegenwart eines Säurebindemittels wie beispielsweise Triethylamin bei Temperaturen zwischen 0°C bis 100°C umsetzt.

Die gemischten Anhydride der Formel (II), (d.h. G steht für O-CO-O Alk oder -O-$SO_2$-Aryl) brauchen nicht in reiner Form isoliert zu werden, sondern können in situ dem Verfahren (A-$a_1$) unterworfen werden.

Die Carbonsäure-Derivate der Formel (XXV) sind ebenfalls noch nicht bekannt.

Man erhält sie, wenn man Pyr(mi)din-Derivate der Formel (XVIII),

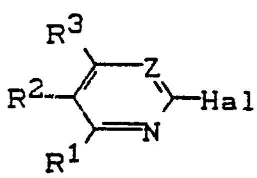

(XVIII)

in welcher
$R^1$, $R^2$, $R^3$, und Z die oben angegebene Bedeutung haben, und
Hal für Chlor oder Brom steht,
E) mit 4-Hydroxy-oder 4-Mercapto-benzonitrilen der Formel (XXVI),

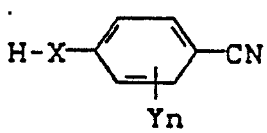

(XXVI)

in welcher
X, Y und n die angegebene Bedeutung haben,
gegebenenfalls in Gegenwart eines Verdünnungsmittels wie beispielsweise Sulfolan sowie in Gegenwart eines Säurebindemittels wie beispielsweise Kaliumhydroxid bei Temperaturen zwischen 30 und 150°C umsetzt zu den Pyri(mi)dyl-oxy-bzw. thio-benzonitrilen der Formel (XIII),

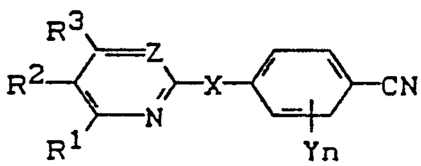

(XIII)

in welcher
$R^1$, $R^2$, $R^3$, Z, X, Y und n die oben angegebene Bedeutung haben,
und diese nach üblichen Methoden unter sauren oder alkalischen Bedingungen verseift,
oder

F) mit 4-Hydroxy-oder 4-Mercaptobenzoesäureestern der Formel (XIXa),

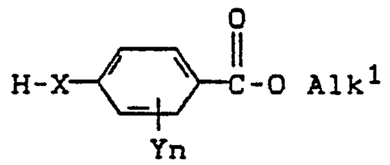

(XIXa)

in welcher X, Y und n die oben angegebene Bedeutung haben und Alk' für Alkyl, insbesondere für Methyl oder Ethyl steht, gegebenenfalls in Gegenwart eines Verdünnungsmittels wie beispielsweise Sulfolan sowie in Gegenwart eines Säurebindemittels wie beispielsweise Kaliumhydroxid bei Temperaturen zwischen 30 und 150°C umsetzt zu den Pyri(mi)dyl-oxy-bzw. thiobenzoesäureestern der Formel (Ig)

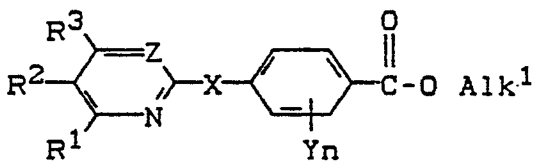

(Ig)

in welcher $R^1$, $R^2$, $R^3$, Z, X, Y, n und Alk' die oben angegebene Bedeutung haben, und diese nach üblichen Methoden unter alkalischen Bedingungen verseift.

Die Pyri(mi)din-Derivate der Formel (XVIII) sind bekannt oder lassen sich nach bekannten Methoden in einfacher Weise herstellen, indem man beispielsweise 2-Hydroxypyridine bzw. 2-Hydroxypyrimidine der Formel (XXVIIa),

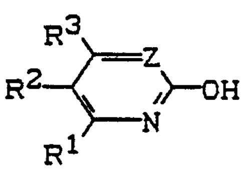

(XXVIIa)

in welcher $R^1$, $R^2$, $R^3$, und Z die oben angegebene Bedeutung haben, mit anorganischen Säurehalogeniden wie beispielsweise Phosphoroxychlorid oder Phosphorpentachlorid, gegebenenfalls in Gegenwart eines Verdünnungsmittels wie beispielsweise Toluol oder Tetrachlormethan umsetzt, oder indem man 2-Aminopyridine bzw. 2-Aminopyrimidine der Formel (XXVIIb),

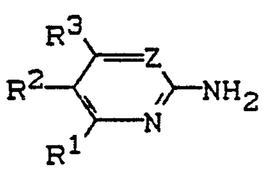

(XXVIIb)

in welcher $R^1$, $R^2$, $R^3$, und Z die oben angegebene Bedeutung haben, in üblicher Art und Weise mit salpetriger Säure in Gegenwart von Halogenwasserstoffsäuren umsetzt.

Die 2-Hydroxy-pyridine und -pyrimidine der Formel (XXVIIa) und die 2-Amino-pyridine und -pyrimidine der Formel (XXVIIb) sind allgemein bekannte Verbindungen der organischen Chemie.

Die 4-Hydroxy-bzw. 4-Mercapto-benzonitrile der Formel (XXVI) sind teilweise bekannt.

Man erhält sowohl die bekannten als auch die nicht bekannten 4-Hydroxy-bzw. 4-Mercapto-benzonitrile der Formel (XXVI) , wenn man beispielsweise 4-Hydroxy-bzw. 4-Mercapto-benzamide der Formel

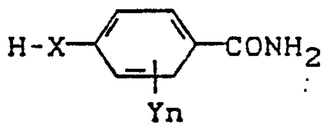

(XXVIa)

in üblicher Art und Weise dehydratisiert.

Die 4-Hydroxy-bzw. 4-Mercapto-benzamide der Formel (XXVIa) sind bekannt oder lassen sich nach bekannten Verfahren herstellen.

Die 4-Hydroxy-bzw. 4-Mercaptobenzoesäureester der Formel (XIXa) werden weiter unten bei der Beschreibung der Ausgangsprodukte für Verfahren ($B-b_1$) beschrieben.

Die zur Durchführung des erfindungsgemäßen Verfahrens ($A-a_1$) weiterhin benötigten Verbindungen sind durch die Formel (III) allgemein definiert.

In der Formel (III) stehen B und $R^4$ vorzugsweise bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden.

Die Ausgangsverbindungen der Formel (III) sind bekannte Verbindungen, wie beispielsweise Alkohole, Phenole, Mercaptane, Thiophenole, Amine oder Hydroxylamine, oder lassen sich nach bekannten Methoden herstellen.

Die zur Durchführung des erfindungsgemäßen Verfahrens ($A-a_2$) benötigten Thiobenzoylchloride sind durch die Formel (IV) allgemein definiert.

In der Formel (IV) stehen $R^1$, $R^2$, $R^3$, Z, X, Y und n bevorzugt bzw. besonders bevorzugt für diejenigen Reste, die bereits im Zusammenhang der Beschreibung der erfindungsgemäßen Stoffe der Formel (I) als bevorzugt bzw. besonders bevorzugt genannt wurden.

Die Thiobenzoylchloride der Formel (IV) sind bisher nicht bekannt. Sie lassen sich nach bekannten Methoden herstellen, indem man Dithiobenzoesäuren der Formel (V),

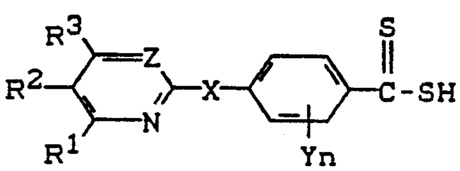

(V)

in welcher

$R^1$, $R^2$, $R^3$, Z, X, Y und n die oben angegebene Bedeutung haben,

mit Thionylchlorid oder Phosgen in üblicher Art und Weise umsetzt (vgl. Houben-Weyl, Methoden der organischen Chemie, Bd. E 5, Seite 620, Georg Thieme Verlag Stuttgart 1985).

Die zur Durchführung des erfindungsgemäßen Verfahrens ($A-a_2$) weiterhin benötigten Verbindungen der Formel (III) wurden bereits bei Verfahren ($A-a_1$) beschrieben.

Die zur Durchführung des erfindungsgemäßen Verfahrens ($A-a_3$) benötigten Dithiobenzoesäuren sind durch die Formel (V) allgemein definiert.

In der Formel (V) stehen $R^1$, $R^2$, $R^3$, Z, X, Y und n bevorzugt bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden.

Die Dithiobenzoesäuren der Formel (V) lassen sich nach bekannten Verfahren (vgl. Houben-Weyl, Methoden der organischen Chemie, Bd. IX, Seite 747, Georg Thieme Verlag Stuttgart 1955) aus Grignard-Verbindungen der Formel (XXVIII),

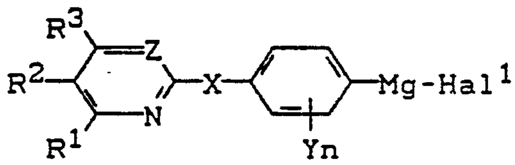

(XXVIII)

in welcher
R¹, R², R³, Z, X, Y und n die oben angegebene Bedeutung haben und
Hal¹ für Halogen, insbesondere für Chlor oder Brom steht,
mit Schwefelkohlenstoff in Gegenwart eines Verdünnungsmittels wie beispielsweise Diethylether oder Tetrahydrofuran bei Temperaturen von -30°C bis +50°C umsetzt,
oder man erhält Dithiobenzoesäuren der Formel (V), wenn man Bis-thiobenzoyl-disulfide der Formel (XXIX),

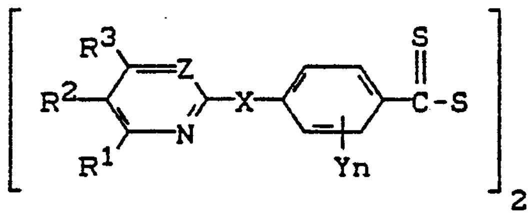

(XXIX)

in welcher
R¹, R², R³, Z, X, Y und n die oben angegebene Bedeutung haben,
mit unedlen Metallen, wie z.B. Zink oder Eisen in Gegenwart von Mineralsäuren oder Alkalien in Wasser oder wäßrigen Alkoholen bei Temperaturen von 0 bis 100°C reduziert.

Grignard-Verbindungen der Formel (XXVIII) lassen sich nach bekannten Methoden herstellen.

Bis-thiobenzoyl-disulfide der Formel (XXIX) erhält man, wenn man Pyri(mi)din-Derivate der Formel - (XVIII),

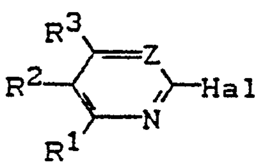

(XVIII)

in welcher
R¹, R², R³, Z und Hal die oben angegebene Bedeutung haben,
mit Bis-[4-hydroxy-(4-mercapto)-thiobenzoyl]-disulfiden der Formel (XXX),

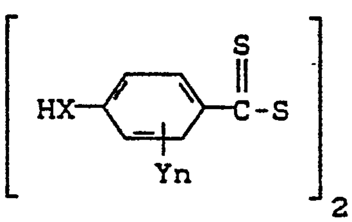

(XXX)

in welcher
X, Y und n die oben angegebene Bedeutung haben,
gegebenenfalls in Gegenwart eines Verdünnungsmittels wie beispielsweise Sulfolan sowie in Gegenwart eines Säurebindemittels wie beispielsweise Kaliumhydroxid bei Temperaturen zwischen +30°C und +130°C umsetzt.

Die Verbindungen der Formel (XXX) sind bekannte Stoffe und lassen sich nach bekannten Methoden herstellen (vgl. Houben-Weyl, Methoden der organischen Chemie, Bd. IX, Seite 748 oder Bd. E5, Seiten 899 ff, Seite 915, Georg Thieme Verlag Stuttgart 1955/1985).

Die zur Durchführung des erfindungsgemäßen Verfahrens (A-$a_3$)/Variante (α) weiterhin benötigten Alkylierungsmittel sind durch die Formel (VI) allgemein definiert.

In der Formel (VI) steht $R^{4-1}$ vorzugsweise für $C_1$-$C_{12}$-Alkyl oder Phenyl-$C_1$-$C_6$-alkyl, L steht vorzugsweise für Chlor, Brom oder Iod, p-Toluolsulfonyloxy, Methoxysulfonyloxy oder Ethoxysulfonyloxy. Die Alkylierungsmittel der Formel (VI) sind allgemein bekannte Verbindungen der organischen Chemie.

Die zur Durchführung des erfindungsgemäßen Verfahrens (A-$a_3$)/Variante (β) benötigten Olefine sind durch die Formel (VII) allgemein definiert.

In der Formel (VI) stehen $R^{16}$, $R^{18}$, $R^{19}$ und $R^{20}$ jeweils unabhängig voneinander vorzugsweise für Wasserstoff, gegebenenfalls einfach oder mehrfach, gleich oder verschieden durch Fluor, Chlor, Brom, $C_1$-$C_4$-Alkoxy, Halogen-$C_1$-$C_4$-alkyl, substituiertes Alkyl mit 1 bis 10 Kohlenstoffatomen, gegebenenfalls einfach bis driefach, gleich oder verschieden durch Methyl, Ethyl, Fluor und Chlor substituiertes Cycloalkyl mit 3 bis 6 Kohlenstoffatomen, gegebenenfalls einfach bis fünffach, gleich oder verschieden durch Fluor, Chlor, Brom, Nitro, Trifluormethyl, Methyl, Ethyl, Methoxy oder Methylthio, substituiertes Phenyl, für Nitro, Cyano, -$COOR^{13}$, -$CONR^{14}R^{15}$, -$CSR^{14}R^{15}$, -$SO_2$-$NR^{14}R^{15}$ oder den Rest D-$R^9$ wobei $R^{13}$, $R^{14}$, $R^{15}$, D und $R^9$ vorzugsweise diejenige Bedeutung haben, die bereits bei der Beschreibung der erfindungsgemäßen Stoffe der Formel (I) vorzugsweise für diejenigen Substituenten genannt wurden, oder $R^{16}$ und $R^{20}$ bedeuten gemeinsam eine weitere C-C-Bindung. Die Olefine der Formel (VII) sind allgemein bekannte Verbindungen der organischen Chemie.

Die zur Durchführung des erfindungsgemäßen Verfahrens (A-$a_4$)/Variante (α) benötigten Dithiobenzoesäure-Derivate sind durch die Formel (Va) allgemein definiert. In der Formel (Va) stehen $R^1$, $R^2$, $R^3$, Z, X, Y und n vorzugsweise für diejenigen Reste, die bereits im Zusammenhang mit der Beschreibung der erfindungsgemäßen Stoffe der Formel (I) als bevorzugt für diese Substituenten genannt wurden, $R^{10}$ steht vorzugsweise für Wasserstoff oder $C_1$-$C_4$-Alkyl.

Die Dithiobenzoesäure-Derivate der Formel (Va) in welcher $R^{10}$ für Alkyl steht, sind erfindungsgemäße Verbindungen und erhältlich nach Verfahren (A-$a_3$)/Variante (α). Verbindungen der Formel (Va), in denen $R^{10}$ für Wasserstoff steht, entsprechen den Verbindungen der Formel (V) und sind erhältlich nach den dort beschriebenen Verfahren.

Die zur Durchführung des erfindungsgemäßen Verfahrens (A-$a_4$)/Variante (α) weiterhin benötigten Amine bzw. Hydroxylamine sind durch die Formeln (VIIIa) bzw. (VIIIb) allgemein definiert. In den Formeln (VIIIa) bzw. (VIIIb) stehen $R^4$, $R^7$ und $R^8$ bevorzugt bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden.

Die Amine bzw. Hydroxylamine der Formeln (VIIIa) bzw. (VIIIb) sind allgemein bekannte Verbindungen der organischen Chemie.

Die zur Durchführung des erfindungsgemäßen Verfahrens (A-$a_4$)/Variante (β) benötigten Benzimidchloride sind durch die Formel (IXa) allgemein definiert. In der Formel (IXa) stehen $R^1$, $R^2$, $R^3$, $R^4$,Z, X, Y und n bevorzugt bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden.

Die Benzimidchloride der Formel (IXa) sind noch nicht bekannt.

Man erhält sie, wenn man Benzamide der Formel (Ih),

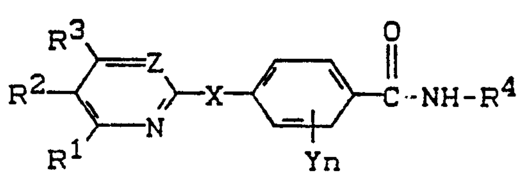

(Ih)

in welcher

$R^1$, $R^2$, $R^3$, $R^4$, Z, X, Y und n die oben angegebene Bedeutung haben,

mit anorganischen Säurechloriden wie beispielsweise Phosphoroxychlorid, Phosphorpentachlorid, Thionylchlorid oder Phosgen in Gegenwart eines Verdünnungsmittels wie beispielsweise Toluol oder Dichlormethan bei Temperaturen zwischen O und +100°C umsetzt.

Die Benzamide der Formel (Ih) sind erfindungsgemäße Verbindungen und erhältlich nach Verfahren (A-$a_1$).

Die zur Durchführung des erfindungsgemäßen Verfahrens (A-$a_4$)/Variante ($\gamma$) als Ausgangsstoffe benötigten Benzoesäureamide sind durch die Formel (Ia) allgemein definiert. In der Formel (Ia) stehen $R^1$, $R^2$, $R^3$, $R^4$, Z, X, Y, n und $B^1$ bevorzugt bzw. besonders bevorzugt für diejenigen Reste, die bereits bei der Beschreibung der erfindungsgemäßen Stoffe der Formel (I) als bevorzugt bzw. besonders bevorzugt für diese Substituenten genannt wurden.

Die Benzoesäureamide der Formel (Ia) sind erfindungsgemäße Verbindungen und erhältlich nach Verfahren (A-$a_1$).

Zur Durchführung des erfindungsgemäßen Verfahrens (A-$a_4$)/Variante ($\gamma$) werden als Ausgangsstoffe Schwefelagentien benötigt. Infrage kommen alle üblicherweise für solche Reaktionen verwendbaren SChwefelungsagentien, vorzugsweise verwendet man das Dekasulfid des Phosphors ($P_4S_{10}$) oder 2,4-Bis-(4-methoxy-phenyl)-2,4-dithiono-1,3,2,4-dithiadiphosphetan (Lawesson-Reagenz). Die Schwefelungsagentien sind allgemein bekannte Verbindungen der anorganischen bzw. organischen Chemie.

Die zur Durchführung des erfindungsgemäßen Verfahrens (A-$a_5$)/Variante ($\alpha$) als Ausgangsstoffe benötigten Benzimidchloride sind durch die Formel (IXb) allgemein definiert. In der Formel (IXb) stehen $R^1$, $R^2$, $R^3$, $R^5$, Z, X, Y und n bevorzugt bzw. besonders bevorzugt für diejenigen Reste, die bereits im Zusammenhang mit der Beschreibung der erfindungsgemäßen Stoffe der Formel (I) als bevorzugt für diese Substituenten genannt wurden.

Die Benzimidchloride der Formel (IXb) sind noch nicht bekannt. Man erhält sie in Analogie zu dem Verfahren, welches bereits für die Herstellung der Verbindungen Formel (IXa) beschrieben wurde.

Die zur Durchführung des erfindungsgemäßen Verfahrens (A-$a_5$)/Variante ($\alpha$) weiterhin als Ausgangsstoffe benötigten Verbindungen der Formel (III) wurden bereits bei der Beschreibung des Herstellungsverfahrens (A-$a_1$) beschrieben.

Die zur Durchführung des erfindungsgemäßen Verfahrens (A-$a_5$)/Variante ($\beta$) als Ausgangsstoffe benötigten Thiobenzamide sind durch die Formel (X) allgemein definiert. In der Formel (X) stehen $R^1$, $R^2$, $R^3$, $R^7$, Z, X, Y und n bevorzugt bzw. besonders bevorzugt für diejenigen Reste, die bereits im Zusammenhang mit der Beschreibung der erfindungsgemäßen Stoffe der Formel (I) als bevorzugt für diese Substituenten genannt wurden.

Man erhält Thiobenzamide der Formel (X), wenn man an Pyri(mi)dyl-oxy-bzw. -thiobenzonitrile der Formel (XIII) ,

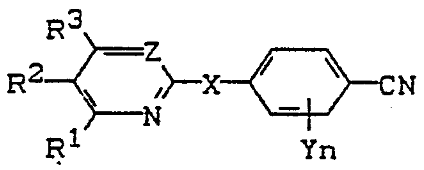

(XIII)

in welcher

$R^1$, $R^2$, $R^3$, Z, X, Y und n die oben angegebene Bedeutung haben und

Schwefelwasserstoff in üblicher Art und Weise gegebenenfalls in Gegenwart eines Verdünnungsmittels wie beispiels weise Pyridin und einer Base wie beispielsweise Natriummethylat oder -ethylat, Pyridin oder Triethylamin addiert.

Die Herstellung der Pyri(mi)dyl-oxy-bzw. -thiobenzonitrile der Formel (XIII) wurde bereits weiter oben bei der Beschreibung der Carbonsäure-Derivate der Formel (XXV) beschrieben.

Die zur Durchführung des erfindungsgemäßen Verfahrens (A-$a_5$)/Variante $\gamma$ weiterhin als Ausgangsstoffe benötigten Alkylierungsmittel sind durch die Formel (VI) allgemein definiert und wurden bereits bei der Beschreibung des Verfahrens (A-$a_3$)/Variante $\alpha$ beschrieben.

Die zur Durchführung des erfindungsgemäßen Verfahrens (A-$a_6$)/Variante $\alpha$ als Ausgangsstoffe benötigten Hydroximsäurehalogenide sind durch die Formel (XIa) allgemein definiert. In der Formel (XIa) stehen $R^1$, $R^2$, $R^3$, $R^6$, Z, X, Y und n bevorzugt bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden, Hal steht vorzugsweise für Chlor oder Brom.

Man erhält die Hydroximsäurehalogenide der Formel (XI) nach bekannten Methoden z.B. durch Halogenierung der entsprechenden Aldoxime (vgl. Houben-Weyl, Methoden der organischen Chemie, Bd. 5/3, Seite 638 f., Georg Thieme Verlag Stuttgart 1962).

Die zur Durchführung des erfindungsgemäßen Verfahrens (A-$a_6$)/Variante ($\alpha$) weiterhin als Ausgangsstoffe benötigten Verbindungen der Formel (III) wurden bereits weiter oben beschrieben.

Die zur Durchführung des erfindungsgemäßen Verfahrens (A-$a_6$)/Variante $\beta$ weiterhin als Ausgangsstoffe benötigten Hydroxamsäuren sind durch die Formel (XIb) allgemein definiert. In der Formel (XIb) stehen $R^1$, $R^2$, $R^3$, $R^6$, Z, X, Y und n bevorzugt bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden, $B^2$ steht für Sauerstoff oder Schwefel.

Man erhält die Hydroxamsäuren der Formel (XIb), wenn man entweder Benzoesäure-Derivate der Formel (II) ,

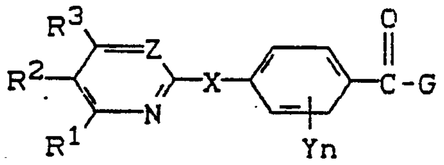

(II)

in welcher
$R^1$, $R^2$, $R^3$, Z, X, Y, n und G die oben angegebene Bedeutung haben,
oder Thiobenzoylchloride der Formel (IV),

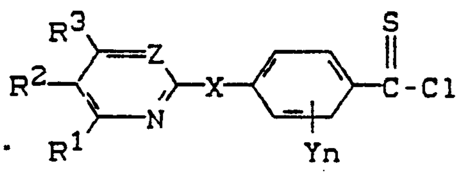

(IV)

in welcher
$R^1$, $R^2$, $R^3$, Z, X, Y und n die oben angegebene Bedeutung haben,
oder Dithiobenzoesäuren der Formel (V),

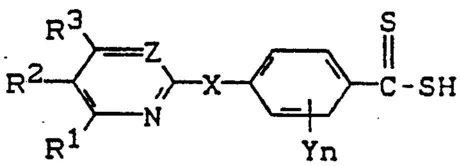

(V)

in welcher
$R^1$, $R^2$, $R^3$, Z, X, Y und n die oben angegebene Bedeutung haben,
mit Hydroxylaminen der Formel (XII).

$R^6O\text{-}NH_2$ (XII)

in welcher
$R^6$ die oben angegebene Bedeutung hat,
gegebenenfalls in Gegenwart eines Verdünnungsmittels wie beispielsweise Toluol, Tetrahydrofuran oder Dioxan und gegebenenfalls in Gegenwart eines Säurebindemittels wie beispielsweise Triethylamin bei Temperaturen zwischen 0 und +100°C umsetzt.

Die zur Durchführung des erfindungsgemäßen Verfahrens (A-$a_6$)/Variante $\beta$ weiterhin als Ausgangsstoffe benötigten Alkylierungsmittel der Formel (VI) sind bereits weiter oben beschrieben worden.

Die zur Durchführung des erfindungsgemäßen Verfahrens (A-$a_6$)/Variante $\gamma$ als Ausgangsstoffe benötigten Thiobenzoesäureester sind durch die Formel (Ib) allgemein definiert. In der Formel (Ib) stehen $R^1$, $R^2$, $R^3$, $R^4$, Z, X, Y und n bevorzugt bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden.

Die Thiobenzoesäureester der Formel (Ib) sind erfindungsgemäße Verbindungen und erhältlich nach Verfahren (A-$a_2$).

Die zur Durchführung des erfindungsgemäßen Verfahrens (A-$a_6$)/Variante $\gamma$ weiterhin als Ausgangsstoffe benötigten Hydroxylamine sind durch die Formel (XII) allgemein definiert. In der Formel (XII) steht $R^6$ bevorzugt für Wasserstoff oder Alkyl mit 1 bis 4 Kohlenstoffatomen.

Die Hydroxylamine der Formel (XII) sind allgemein bekannte Verbindungen der organischen Chemie.

Die zur Durchführung des erfindungsgemäßen Verfahrens (A-$a_7$)/Variante $\alpha$ als Ausgangsstoffe benötigten Benzonitrile sind durch die Formel (XIII) allgemein definiert und wurden bereits bei der Bechreibung des Verfahrens (A-$a_1$) beschrieben.

Die zur Durchführung des erfindungsgemäßen Verfahrens (A-$a_7$)/Variante $\alpha$ weiterhin als Ausgangsstoffe benötigten 2-oder 3-Hydroxyalkylamine sind durch die Formel (XIV) allgemein definiert. In der Formel (XIV) steht $Alk^2$ vorzugsweise für gegebenenfalls einfach bis dreifach, gleich oder verschieden durch Methyl, Ethyl, Methoxy, Ethoxy, Fluor oder Chlor substituiertes Ethan-oder Propandiyl.

Die 2-bzw. 3-Hydroxyalkylamine sind allgemein bekannte Verbindungen der organischen Chemie.

Die zur Durchführung des erfindungsgemäßen Verfahrens (A-$a_7$)/Variante $\beta$ als Ausgangsstoffe benötigten Thiobenzamide sind durch die Formel (Xa) allgemein definiert. In der Formel (Xa) stehen $R^1$, $R^2$, $R^3$, Z, X, Y und n bevorzugt bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden.

Die Thiobenzamide der Formel (Xa) wurden bereits bei der Beschreibung der Thiobenzamide der Formel (X) bei Verfahren (A-$a_5$)/Variante ($\beta$) beschrieben.

Die zur Durchführung des erfindungsgemäßen Verfahrens (A-$a_7$)/Variante $\beta$ weiterhin als Ausgangsstoffe benötigten $\alpha$-Halogencarbonyl-Derivate sind durch die Formel (XV) allgemein definiert. In der Formel (XV) stehen $R^{21}$ und $R^{22}$, unabhängig voneinander bevorzugt für Wasserstoff, $C_1$-$C_6$-Alkyl oder für gegebenenfalls ein-bis dreifach, gleich oder verschieden durch Fluor, Chlor, Brom, Methyl, Ethyl, Methoxy, Ethoxy oder Trifluormethyl substituiertes Phenyl, Hal steht für Chlor oder Brom.

Die zur Durchführung des erfindungsgemäßen Verfahrens (A-$a_7$)/Variante $\gamma$ als Ausgangsstoffe benötigten Hydroxamsäuren sind durch die Formel (XIb-1) allgemein definiert. In der Formel (XIb-1) stehen $R^1$, $R^2$, $R^3$, Z, X, Y und n bevorzugt bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden.

Die Hydroxamsäuren der Formel (XIb-1) wurden bereits weiter oben bei der Beschreibung der Verbindungen der Formel (XIb) (Verfahren A-$a_6$)/Variante $\gamma$) beschrieben.

Die zur Durchführung des erfindungsgemäßen Verfahrens (A-$a_7$)/Variante $\gamma$ weiterhin als Ausgangsstoffe benötigten bifunktionellen Alkylierungsmittel sind durch die Formel (VIa) allgemein definiert. In der Formel - (VIa) steht $Alk^2$ vorzugsweise für jeweils gegebenenfalls durch Methyl, Ethyl, Cyclopentyl, Cyclohexyl oder gegebenenfalls durch Fluor, Chlor, Methyl, Methoxy oder Trifluormethyl substituiertes Phenyl, substituiertes Ethandiyl oder Propandiyl, $L^1$ und $L^2$ stehen für Chlor oder Brom.

Die bifunktionellen Alkylierungsmittel der Formel (VIa) sind allgemein bekannte Verbindungen der organischen Chemie.

Die zur Durchführung des erfindungsgemäßen Verfahrens (A-$a_7$)/Variante $\delta$ als Ausgangsstoffe benötigten Benzamidoxime sind durch die Formel (XVI) allgemein definiert. In der Formel (XVI) stehen $R^1$, $R^2$, $R^3$, Z, X, Y und n bevorzugt bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden.

Man erhält die Benzamidoxime der Formel (XVI) , wenn man beispielsweise Benzonitrile der Formel - (XIII) ,

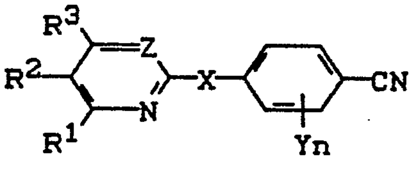

(XIII)

in welcher

$R^1$, $R^2$, $R^3$, Z, X, Y und n die oben angegebene Bedeutung haben,

gegebenenfalls in Gegenwart von Verdünnungsmitteln wie beispielsweise Ethanol bei Temperaturen zwischen O und +100°C in üblicher Art und Weise mit Hydroxylamin umsetzt oder Hydroximsäurechloride der Formle (XIa-1),

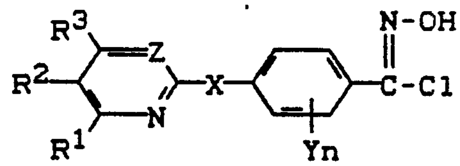

(XIa-1)

in welcher

$R^1$, $R^2$, $R^3$, Z, X, Y und n die oben angegebene Bedeutung haben,

mit Ammoniak gegebenenfalls in Gegenwart eines inerten Verdünnungsmittels wie beispielsweise Tetrahydrofuran bei Temperaturen zwischen O und +100°C in üblicher Art und Weise umsetzt (vgl. Houben-Weyl, Methoden der organischen Chemie, Bd. X/4, Seite 211, Georg Thieme Verlag Stuttgart 1968).

Die Benzonitrile der Formel (XIII) und Hydroximsäurechloride der Formel (XIa-1) wurden bereits weiter oben beschrieben.

Die zur Durchführung des erfindungsgemäßen Verfahrens ($A-a_7$)/Variante $\delta$ weiterhin benötigten Acylierungsmittel sind durch die Formel (XVII) allgemein definiert. In der Formel (XVII) steht $R^{4-2}$ vorzugsweise für gegebenenfalls durch Halogen oder den Rest -D-$R^9$ (Bedeutung für D und $R^9$ wie oben) substituiertes $C_1$-$C_{10}$-Alkyl, $C_3$-$C_6$-Cycloalkyl oder gegebenenfalls durch Fluor, Chlor, Nitro, Trifluormethyl, Methyl, Ethyl oder Methoxy substituiertes Phenyl.

Die zur Durchführung des erfindungsgemäßen Verfahrens ($B-b_1$) als Ausgangsstoffe benötigten Pyri(mi)din-Derivate sind durch die Formel (XVIII) allgemein definiert. In der Formel (XVIII) stehen $R^1$, $R^2$, $R^3$ und z bevorzugt bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden.

Als Beispiele für diese Stoffe seien besonders die folgenden Verbindungen genannt:

2-Chlor-4-methyl-pyridin
2-Chlor-6-methyl-pyridin
2-Chlor-4,6-dimethyl-pyridin
2-Chlor-4-methyl-pyrimidin
2-Chlor-4-ethyl-pyrimidin
2-Chlor-5-methyl-pyrimidin
2-Chlor-5-ethyl-pyrimidin
2-Chlor-5-propyl-pyrimidin
2-Chlor-5-isopropyl-pyrimidin
2,4-Dichlor-6-methyl-pyrimidin
2-Chlor-4-methoxy-6-methyl-pyrimidin
2-Chlor-4-amino-6-methyl-pyrimidin
2-Chlor-4-methylamino-6-methyl-pyrimidin
2-Chlor-4-dimethylamino-6-methyl-pyrimidin
2-Chlor-4,5-dimethyl-pyrimidin
2-Chlor-4-methyl-5-ethyl-pyrimidin
2-Chlor-4-ethyl-5-methyl-pyrimidin
2-Chlor-4,6-dimethyl-pyrimidin
2-Chlor-4-methyl-6-trifluormethyl-pyrimidin
2-Chlor-4-methyl-6-ethyl-pyrimidin
2,5-Dichlor-4,6-dimethyl-pyrimidin
2-Chlor-5-brom-4,6-dimethyl-pyrimidin
2,5-Dichlor-4-methoxy-6-methyl-pyrimidin
2,5-Dichlor-4-methylmercapto-6-methyl-pyrimidin
2-Chlor-4,5,6-trimethyl-pyrimidin
2-Chlor-5-ethyl-4,6-dimethyl-pyrimidin
2-Chlor-4,5-cyclopenteno-pyrimidin
2-Chlor-4,5-cyclopenteno-6-methyl-pyrimidin

2-Chlor-4,5-cyclohexeno-pyrimidin
2-Chlor-4,5-cyclohexeno-6-methyl-pyrimidin
2-Chlor-chinazolin
2,6-Dichlor-chinazolin
2-Chlor-4-methyl-chinazolin

Die Pyri(mi)din-Derivate der Formel (XVIII) wurden bereits weiter oben bei der Beschreibung des Verfahrens (A-$a_1$) beschrieben.

Die zur Durchführung des erfindungsgemäßen Verfahrens (B-$b_1$) weiterhin als Ausgangsstoffe benötigten 4-Hydroxy-bzw. 4-Mercapto-benzoesäure-Derivate sind durch die Formel (XIX) allgemein definiert. In der Formel (XIX) stehen X, Y, n, A, B und $R^4$ vorzugsweise bzw. besonders bevorzugt für diejenigen Reste, die bereits im Zusammenhang mit der Beschreibung der erfindungsgemäßen Stoffe der Formel (I) als bevorzugt bzw. besonders bevorzugt genannt wurden.

Die 4-Hydroxy-bzw. 4-Mercapto-benzoesäure-Derivate der Formel (XIX) sind teilweise bekannt.

Man erhält sowohl die bekannten als auch die nicht bekannten Verbindungen der Formel (XIX) nach bekannten Methoden. So gewinnt man 4-Hydroxy-und 4-Mercaptobenzoesäureester durch Veresterung der entsprechenden freien Säuren mit Alkoholen in Gegenwart starker Säuren nach üblichen Methoden, vgl. beispielsweise Verfahren (A-$a_3$)/Variante ($\alpha$); entsprechende Amide erhält man aus den Säuren durch Umsetzungen mit Aminen, wie beispielsweise Aminen der Formel (VIIIa) oder Hydroxylaminen der Formel (VIIIb), in Gegenwart von wasserentziehenden Mitteln wie beispiels weise Phosphor-(III)-chlorid und gegebenenfalls in Gegenwart von Verdünnungsmitteln bei Temperaturen zwischen 0 und +100°C; 4-Hydroxydithiobenzoesäureester erhält man durch Umsetzung von Phenolen mit Schwefelkohlenstoff in Gegenwart starker Alkalien wie beispielsweise Natriumhydroxid oder Kaliumhydroxid und nachfolgender S-Alkylierung mit den obengenannten Alkylierungsmitteln nach den bei Verfahren (A-$a_3$)/Variante ($\alpha$) genannten Reaktionsbedingungen. 4-Mercaptobenzoesäure-Derivate lassen sich aus entsprechenden 4-Amino-benzoesäure-Derivaten durch Diazotierung mit salpetriger Säure, Umsetzung mit Natriumdisulfid und nachfolgender Reduktion der dabei entstehenden Disulfide beispielsweise mit Zink in saurem Medium in üblicher Art und Weise bei Temperaturen zwischen O und +100°C herstellen.

Die zur Durchführung des erfindungsgemäßen Verfahrens (B-$b_2$) als Ausgangsstoffe benötigten 2-Mercapto-pyri(mi)dine sind durch die Formel (XX) allgemein definiert. In der Formel (XX) stehen $R^1$, $R^2$, $R^3$ und Z bevorzugt bzw. besonders bevorzugt für diejenigen Reste, die bereits im Zusammenhang mit der Beschreibung der erfindungsgemäßen Stoffe der Formel (I) als bevorzugt bzw. besonders bevorzugt genannt wurden.

Die 2-Mercapto-pyri(mi)dine der Formel (XX) sind bekannt oder lassen sich nach bekannten Methoden herstellen, indem man beispielsweise $\beta$-Dicarbonylverbindungen mit Thioharn stoffen, 2-Hydroxy-pyri(mi)dine mit Phosphor-V-sulfid oder dem Lawesson-Reagenz oder 2-Halogen-pyri(mi)dine mit Metallsalzen des Schwefelwasserstoffs wie beispielsweise Natriumsulfid in üblicher Art und Weise umsetzt.

Die zur Durchführung des erfindungsgemäßen Verfahrens (B-$b_2$) weiterhin als Ausgangsstoffe benötigten Verbindungen sind durch die Formel (XXI) allgemein definiert. In der Formel (XXI) stehen A, B, $R^4$, Y und n bevorzugt bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden, Hal steht für Chlor oder Brom und m bevorzugt für 1, 2 oder 3.

Die 4-Halogen-benzoesäure-Derivate der Formel (XXI) sind bekannt oder lassen sich nach bekannten Methoden beispielsweise aus den freien Säuren oder den entsprechenden Säurehalogeniden durch Umsetzung mit den gewünschten Nukleophilen herstellen, vgl. die bei der Beschreibung der Verbindungen der Formel (XIX) aufgeführten Methoden.

Dabei laufen die Reaktionen nach dem Verfahren (B-$b_2$) nur dann in gewünschter Weise ab, wenn das Halogen in den Verbindungen der Formel (XXI) durch zusätzliche elektronenziehende Substituenten im aromatischen Rest aktiviert ist. Für eine solche Aktivierung kommen als Substituenten besonders Fluor, Chlor, Brom, Nitro, Cyano oder Trifluormethyl infrage.

Für das Verfahren (B-$b_2$) sind als besonders geeignete Reaktionskomponenten der Formel (XXI) die folgenden Verbindungen genannt:

3,4,5-Trichlor-benzoesärue
3,5-Dichlor-4-fluor-benzoesäure
Pentachlorbenzoesäure
3-Nitro-4-chlor-benzoesäure
3,5-Dinitro-4-chlor-benzoesäure
3-Cyan-4-chlor-benzoesäure
4-Chlor-3-trifluormethyl-benzoesäure

Die zur Durchführung des erfindungsgemäßen Verfahrens (C-$c_1$) bis (C-$c_4$) als Ausgangsstoffe benötigten Verbindungen sind durch die Formel (I) allgemein definiert, sind erfindungsgemäße Verbindungen und erhältlich nach den oben beschriebenen Herstellungsverfahren.

Die zur Durchführung des erfindungsgemäßen Verfahrens (C-$c_1$) weiterhin als Ausgangsstoffe benötigten Halogenierungsmittel sind allgemein bekannte Verbindungen der organischen Chemie. Vorzugsweise verwendet man Chlor oder Brom in elementarer Form. Gegebenenfalls führt man das erfindungsgemäße Verfahren in Gegenwart eines Katalysators durch. Als Katalysatoren können alle üblicherweise für solche aromatischen elektrophilen Substituenten verwendbaren Katalysatoren wie Lewis-Säuren (Aluminium-III-chlorid) eingesetzt werden.

Die zur Durchführung des erfindungsgemäßen Verfahrens (C-$c_2$) weiterhin als Ausgangsstoffe benötigten Reduktionsmittel sind allgemein bekannte Verbindungen. Vorzugsweise verwendet man Metalle wie Eisen, Zink oder Zinn oder Metallsalze, wie Eisen-II-oder Zinn-II-Salze, insbesondere die Chloride oder Verbindungen des Schwefels wie Sulfide, Sulfite oder Dithionite.

Die zur Durchführung des erfindungsgemäßen Verfahrens (C-$c_3$) weiterhin als Ausgangsstoffe benötigten Säurechloride sind durch die Formel (XXII) allgemein definiert. In der Formel (XXII) steht $R^{23}$ vorzugsweise für Methyl, Ethyl, Methoxy, Ethoxy, Dimethylamino, Diethylamino oder Methylethylamino. Die Verbindungen der Formel (XXII) sind allgemein bekannte Verbindungen der organischen Chemie.

Die zur Durchführung des erfindungsgemäßen Verfahrens (C-$c_4$) weiterhin als Ausgangsstoffe benötigte salpetrige Säure oder Alkylnitrite, insbesondere Methyl-oder Ethylnitrit, sind allgemein bekannte Verbindungen.

Besonders bevorzugt werden durch Verfahren (C) die folgenden Substituenten ineinander überführt: Wasserstoff in Chlor oder Brom; Nitro in Amino; Amino in Chlor, Brom oder Cyano und Amino in Methyl- oder Ethylcarbonylamino.

Die zur Durchführung des erfindungsgemäßen Verfahrens (D) als Ausgangsstoffe benötigten Verbindungen der Formel (I), in denen $R^3$ die Bedeutung Fluor, Chlor oder Brom hat, sind durch die Formel (If) allgemein definiert. Die Verbindungen der Formel (If) sind erfindungsgemäße Verbindungen und erhältlich nach den Verfahren A bis C. In der Formel (If) stehen $R^1$, $R^2$, $R^4$, Z, X, A, B, Y und n bevorzugt bzw. besonders bevorzugt für diejenigen Reste, die bereits im Zusammenhang mit der Beschreibung der erfindungsgemäßen Stoffe als bevorzugt bzw. besonders bevorzugt genannt wurden.

$R^{3-1}$ steht vorzugsweise für Methoxy, Ethoxy, Amino, Methylamino, Ethylamino, Dimethylamino oder Ethylamino.

Die zur Durchführung des erfindungsgemäßen Verfahrens (D) weiterhin als Ausgangsstoffe benötigten Alkohole bzw. primären und sekundären Amine sind durch die Formel (XXIII) bzw. (XXIV) allgemein definiert. In der Formel (XXIII) steht $R^{24}$ vorzugsweise für Methyl oder Ethyl, in Formel (XXIV) stehen $R^{25}$ und $R^{26}$ unabhängig voneinander vorzugsweise für Wasserstoff, Methyl oder Ethyl.

Die Alkohole der Formel (XXIII), die primären und sekundären Amine der Formel (XXIV) sowie Ammoniak sind allgemein bekannte Verbindungen der organischen Chemie.

Als Verdünnungsmittel zur Durchführung des erfindungsgemäßen Verfahrens (A-$a_1$) kommen inerte organische Lösungsmittel infrage.

Vorzugsweise verwendet man Kohlenwasserstoffe, wie Benzin Benzol, Toluol, Xylol und Tetralin, ferner Halogenkohlenwasserstoffe, wie Methylenchlorid, Chloroform, Tetrachlor kohlenstoff, Chlorbenzol und o-Dichlorbenzol, außerdem Ketone, wie Aceton und Methyl-isopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan.

Für den Fall, daß G in der Formel (II) für Halogen steht und die Hydrolysestabilität des Säurehalogenids es zuläßt kann die Umsetzung auch in Gegenwart von Wasser durchgeführt werden.

Verwendet man als Reaktionskomponente der Formel (III) Alkohole oder Amine in flüssiger Form, so können diese mit besonderem Vorzug in entsprechendem Überschuß gleichzeitig als Verdünnungsmittel eingesetzt werden.

Das erfindungsgemäße Verfahren (A-$a_1$) wird gegebenenfalls in Gegenwart eines Säurebindemittels durchgeführt. Als solche kommen alle üblichen anorganischen oder organischen Basen infrage. Vorzugsweise verwendet man tertiäre Amine wie Triethylamin, Pyridin und N,N-Dimethyl-anilin, ferne Erdalkalimetalloxide, wie Magnesium-und Calciumoxid, außerdem Alkali-und Erdalkalimetall-carbonate, wie Natriumcarbonat, Kaliumcarbonat und Calciumcarbonat. Alkohole, Phenole, Mercaptane und Thiophenole können aber auch in Form ihrer vorher bereiteten Metallverbindungen eingesetzt werden. Besitzt die Reaktionskomponente der Formel (III) basische Eigenschaften (B steht für -N-$R^7$ oder -N-$OR^8$) so kann auch diese im entsprechenden Überschuß als Säurebindemittel eingesetzt werden.

Die Reaktionstemperaturen können bei der Durchführung des erfindungsgemäßen Verfahrens (A-$a_1$) innerhalb eines größeren Bereiches variiert werden. Arbeitet man ohne Lösungsmittel und Säurebindemittel, so geht man im allgemeinen so vor, daß man die Komponenten zunächst bei Temperaturen zwischen -20°C und +20°C reagieren läßt und danach auf Temperaturen zwischen 70 und 200°C erhitzt. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren wird im allgemeinen unter Normaldruck durchgeführt.

Zur Durchführung des erfindungsgemäßen Verfahrens (A-$a_1$) setzt man pro Mol Benzoesäure-Derivat der Formel (II) im allgemeinen 1,0 bis 5,0 Mol, vorzugsweise 1,0 bis 2,0 Mol, an Verbindung der Formel (III) und gegebenenfalls 1,0 bis 3,0, vorzugsweise 1,0 bis 2,0, Mol an Säurebindemittel ein.

Bei der Durchführung des erfindungsgemäßen Verfahrens (A-$a_1$) ist es möglich, auf eine vorherige Herstellung der Benzoylhalogenide (in der Formel (II) steht G für Halogen) zu verzichten und die freien Säuren mit den Verbindungen der Formel (III) in Gegenwart eines wasserbindenden Mittels, wie beispielsweise die zur Herstellung der Benzoylhalogenide verwendbaren anorganischen Säurehalogenide, wie Phosphortrichlorid oder Phosphoroxychlorid, umzusetzen.

Die Aufarbeitung erfolgt nach üblichen Methoden. Im allgemeinen geht man bei den erfindungsgemäßen Herstellungsverfahren so vor, daß man ausgefallene Salze entfernt und das verbleibende Reaktionsgemisch durch Abziehen des Verdünnungsmittels einengt. Arbeitet man in Gegenwart von Wasser oder von mit Wasser mischbaren Solventien, so kann man auch so verfahren, daß man das Reaktionsgemisch mit Wasser verdünnt, das entstehende Gemisch absaugt oder mit einem mit Wasser wenig mischbaren organischen Lösungsmittel extrahiert, die organische Phase wäscht, einengt und den verbleibenden Rückstand gegebenenfalls üblichen Reinigungsverfahren unterwirft.

Als Verdünnunsmittel zur Durchführung des Herstellungsverfahrens (A-$a_2$) kommen ebenfalls inerte organische Lösungsmittel oder polare Solventien infrage. Vorzugsweise verwendet man die bei dem Herstellungsverfahren (A-$a_1$) genannten Lösungsmittel.

Als Säurebindemittel zur Durchführung des Herstellungsverfahrens (A-$a_2$) kommen alle üblicherweise verwendbaren organischen oder anorgansichen Basen infrage. Vorzugsweise verwendet man die bei Verfahren (A-$a_1$) genannten Basen.

Die Reaktionstemperaturen können bei dem Herstellungsverfahren (A-$a_2$) in einem größeren Bereich variiert werden. Im allgemeinen arbeitet man bei Temperaturen zwischen -20°C und +100°C, vorzugsweise zwischen 0°C und 50°C.

Das erfindungsgemäße Verfahren (A-$a_2$) wird im allgemeinen bei Normaldruck durchgeführt.

Zur Durchführung des erfindungsgemäßen Verfahrens (A-$a_2$) setzt man pro Mol Thiobenzoylchlorid der Formel (IV) im allgemeinen 1,0 bis 5,0 Mol, vorzugsweise 1,0 bis 2,0 Mol, an Verbindung der Formel (III) und 1,0 bis 5,0 Mol, vorzugsweise 1,0 bis 2,0 Mol, an Säurebindemittel ein. Die Reaktionsdurchführung, Aufarbeitung und Isolierung der Reaktionsprodukte der Formel (I) erfolgt in allgemein üblicher Art und Weise (vgl. Houben-Weyl, Methoden der organischen Chemie, Bd. E5, Seiten 792, 907, 1246, Georg Thieme Verlag Stuttgart 1985).

Als Verdünnungsmittel zur Durchführung der erfindungsgemäßen Verfahren (A-$a_3$)/Variante ($\alpha$) und ($\beta$) kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester Sulfoxide oder Sulfone, wie Dimethylsulfoxid oder Sulfolan, oder Alkohole, wie Methanol, Ethanol oder Propanol.

Als Säurebindemittel zur Durchführung des erfindungsgemäßen Verfahrens (A-$a_3$)/Variante ($\alpha$) kommen alle üblichen anorganischen oer organischen Basen infrage. Vorzugsweise verwendet man tertiäre Amine, wie Triethylamin, Pyridin oder N,N-Dimethylanilin, Erdalkalimetalloxide, wie Magnesium-und Calciumoxid, Alkali-und Erdalkalimetallcarbonate, wie Natriumcarbonat, Kalium-oder Calciumcarbonate, wie Natriumcarbonat, Kalium-oder Calciumcarbonat, Alkalimetallhydroxide, -hydride oder -alkoholate, wie Natriumhydroxid oder Kaliumhydroxid, Natriumhydrid, Natriummethylat oder Kalium-t-butylat.

Das erfindungsgemäße Verfahren (A-$a_3$)/Variante ($\beta$) wird gegebenenfalls in Gegenwart eines Katalysators durchgeführt. Vorzugsweise verwendet man Basen wie Natriumamid, Alkalialkoholat wie Natrium-oder Kaliummethylat oder ein Amin wie Pyridin, Piperidin oder Triethylamin.

Die Reaktionstemperaturen können bei der Durchführung der erfindungsgemäßen Verfahren ($A-a_3$)-/Variante ($\alpha$) und ($\beta$) innerhalb eines größeren Bereichs variiert werden. Im allgemeinen arbeitet man bei Temperaturen zwischen 0°C und 100°C, vorzugsweise zwischen 20°C und 80°C.

Die erfindungsgemäßen Verfahren ($A-a_3$)/Variante ($\alpha$) und ($\beta$) werden im allgemeinen bei Normaldruck durchgeführt. Mit gasförmigen Reaktionskomponenten arbeitet man vorzugsweise im geschlossenen Gefäß.

Zur Durchführung des erfindungsgemäßen Verfahrens ($A-a_3$)/Variante ($\alpha$) setzt man pro Mol Dithiobenzoesäure der Formel (V) 1,0 bis 5,0 Mol, vorzugsweise 1,0 bis 3,0 Mol, an Alkylierungsmittel der Formel -(VI) und gegebenenfalls 1,0 bis 5,0 Mol, vorzugsweise 1,0 bis 3,0 Mol, an Säurebindemittel ein.

Zur Durchführung des erfindungsgemäßen Verfahrens ($A-a_3$)/Variante ($\beta$) setzt man pro Mol Dithiobenzoesäure der Formel (V) 1,0 bis 5,0 Mol, vorzugsweise 1,0 bis 3,0 Mol, an Olefin der Formel (VII) und gegebenenfalls bis zu einem Mol, vorzugsweise 0,01 bis 0,2 Mol, an Katalysator ein.

Reaktionsdurchführung und Aufarbeitung erfolgen bei dem erfindungsgemäßen Verfahren ($A-a_3$)/Variante ($\alpha$) und ($\beta$) nach bekannten Methoden (vgl. Houben-Weyl, Methoden der organischen Chemie, Bd. IX, Seite 760 und Bd. E/5, Seiten 907 und 908, Georg Thieme Verlag Stuttgart 1955/1985).

Als Verdünnungsmittel zur Durchführung der erfindungsgemäßen Verfahren ($A-a_4$)/Variante ($\alpha$) und ($\beta$) kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester Sulfoxide oder Sulfone, wie Dimethylsulfoxid oder Sulfolan, oder Alkohole, wie Methanol, Ethanol oder Propanol.

Die Reaktionstemperaturen können bei der Durchführung des erfindungsgemäßen Verfahrens ($A-a_4$)-/Variante ($\alpha$) innerhalb eines größeren Bereiches variiert werden. Im allgemeinen arbeitet man bei Temperaturen zwischen 0°C und 100°C, vorzugsweise zwischen 20 und 75°C.

Das erfindungsgemäße Verfahren ($A-a_4$)/Variante ($\alpha$) wird im allgemeinen unter Normaldruck durchgeführt.

Zur Durchführung des erfindungsgemäßen Verfahrens ($A-a_4$)/Variante ($\alpha$) setzt man pro Mol Dithiobenzoesäure-Derivat der Formel (Va) 1,0 bis 5,0 Mol, vorzugsweise 1,0 bis 2,0 Mol, an Amin der Formel (VIIIa) bzw. Hydroxylamin der Formel (VIIIb) ein.

Die Reaktionstemperaturen können bei der Durchführung des erfindungsgemäßen Verfahrens ($A-a_4$)-/Variante ($\beta$) in einem größeren Bereich variiert werden. Im allgemeinen arbeitet man bei Temperaturen zwischen 0°C und 100°C, vorzugsweise zwischen +10°C und +50°C. Das erfindungsgemäße Verfahren ($A-a_4$)/Variante ($\beta$) wird im allgemeinen unter Normaldruck durchgeführt.

Zur Durchführung des erfindungsgemäßen Verfahrens ($A-a_4$)/Variante ($\beta$) setzt man pro Mol Benzimidchlorid der Formel (IXa) 1,0 bis 5,0 Mol, vorzugsweise 1,0 bis 2,0 Mol, an Schwefelwasserstoff bzw. seiner Salze ein.

Zur Durchführung des erfindungsgemäßen Verfahrens ($A-a_4$)/Variante $\gamma$ kommen inerte organische Verdünnungsmittel in Frage. Vorzugsweise verwendet man Toluol, Benzol, Xylol.

Die Reaktionstemperaturen können bei der Durchführung des erfindungsgemäßen Verfahrens ($A-a_4$)-/Variante ($\gamma$) in einem größeren Bereich variiert werden. Im allgemeinen arbeitet man bei Temperaturen zwischen +20°C und +150°C, vorzugsweise zwischen +50°C und +120°C.

Das erfindungsgemäße Verfahren ($A-a_4$)/Variante ($\gamma$) wird im allgemeinen unter Normaldruck durchgeführt.

Zur Durchführung des erfindungsgemäßen Verfahrens ($A-a_4$)/Variante ($\gamma$) setzt man pro Mol Benzoesäureamid der Formel (Ia) 1,0 bis 5,0 Mol, vorzugsweise 1,0 bis 2,0 Mol, an Schwefelungsagentien ein.

Reaktionsdurchführungen und Aufarbeitung erfolgen bei den erfindungsgemäßen Verfahren ($A-a_4$)-/Variante ($\alpha$), ($\beta$) und ($\gamma$) nach bekannten Methoden (vgl. Houben-Weyl, Methoden der organischen Chemie, Bd. E/5, Seiten 1249/1250, Seite 1251, Seiten 1243-1245, Georg Thieme Verlag Stuttgart 1985).

Als Verdünnungsmittel zur Durchführung des erfindungsgemäßen Verfahrens ($A-a_5$)/Variante ($\alpha$) kommen inerte organische Lösungsmittel infrage. Vorzugsweise verwendet man die bei Verfahren ($A-a_1$) genannten Lösungsmittel.

Als Säurebindemittel zur Durchführung des erfindungsgemäßen Verfahrens ($A-a_5$)/Variante ($\alpha$) kommen alle üblichen anorganischen oder organischen Basen infrage, Vorzugsweise verwendet man die bei Verfahren ($A-a_1$) genannten Säurebindemittel.

Die Reaktionstemperaturen können bei der Durchführung des erfindungsgemäßen Verfahrens (A-$a_5$)-/Variante (α) innerhalb eines größeren Bereiches variiert werden. Im allgemeinen arbeitet man bei Temperaturen zwischen -20°C und +100°C, vorzugsweise zwischen 0°C und +50°C.

Das erfindungsgemäße Verfahren (A-$a_5$)/Variante (α) wird im allgemeinen unter Normaldruck durchgeführt.

Zur Durchführung des erfindungsgemäßen Verfahrens (A-$a_5$)/Variante (α) setzt man pro Mol Benzimidchlorid der Formel (IXb) 1,0 bis 5,0 Mol, vorzugsweise 1,0 bis 3,0 Mol an Verbindung der Formel (III) und 1,0 bis 5,0 Mol, vorzugsweise 1,0 bis 3,0 Mol an Säurebindemittel ein.

Als Verdünnungsmittel zur Durchführung des erfindungsgemäßen Verfahrens (A-$a_5$)/Variante (β) kommen inerte organische Lösungsmittel infrage. Vorzugsweise verwendet man die bei Verfahren (A-$a_3$)-/Variante (α) genannten Lösungsmittel.

Als Säurebindemittel zur Durchführung des erfindungsgemäßen Verfahrens (A-$a_5$)/Variante (β) kommen alle üblichen anorganischen oder organischen Basen infrage. Vorzugsweise verwendet man die bei Verfahren (A-$a_3$)/Variante (α) genannten Säurebindemittel.

Führt man das erfindungsgemäße Verfahren (A-$a_5$)/Variante (β) in Abwesenheit eines Säurebindemittels durch, erhält man zunächst die Salze der Amino-thioester mit dem Anion $L^{\ominus}$, aus denen man die freien Verbindungen durch Basenzusatz gewinnt.

Die Reaktionstemperaturen können bei der Durchführung des erfindungsgemäßen Verfahrens (A-$a_5$)-/Variante (β) in einem größeren Bereich variiert werden. Im allgemeinen arbeitet man bei Temperaturen zwischen 0°C und 200°C, vorzugsweise zwischen 20°C und +180°C.

Das erfindungsgemäße Verfahren (A-$a_5$)/Variante (β) wird im allgemeinen unter Normaldruck durchgeführt.

Zur Durchführung des erfindungsgemäßen Verfahrens (A-$a_5$)/Variante (β) setzt man pro Mol Thiobenzamid der Formel (X) 1,0 bis 5,0 Mol, vorzugsweise 1,0 bis 3,0 Mol, an Alkylierungsmittel der Formel (VI) und gegebenenfalls 1,0 bis 5,0 Mol, vorzugsweise 1,0 bis 3,0 Mol an Säurebindemittel ein.

Als Verdünnungsmittel zur Durchführung des erfindungsgemäßen Verfahrens (A-$a_6$)/Variante (α) kommen inerte organische Lösungsmittel infrage. Vorzugsweise verwendet man die bei Verfahren (A-$a_1$) genannten Lösungsmittel.

Als Säurebindemittel zur Durchführung des erfindungsgemäßen Verfahrens (A-$a_6$)/Variante (α) kommen alle üblichen anorganischen oder organischen Basen infrage. Vorzugsweise verwendet man die bei Verfahren (A-$a_1$) genannten Säurebindemittel.

Die Reaktionstemperaturen können bei der Durchführung des erfindungsgemäßen Verfahrens (A-$a_6$)-/Variante (α) innerhalb eines größeren Bereiches variiert werden. Im allgemeinen arbeitet man bei Temperaturen zwischen -20°C und +100°C, vorzugsweise zwischen 0°C und +75°C.

Das erfindungsgemäße Verfahren (A-$a_6$)/Variante (α) wird im allgemeinen unter Normaldruck durchgeführt.

Zur Durchführung des erfindungsgemäßen Verfahrens (A-$a_6$)/Variante (α) setzt man pro Mol Hydroximsäurehalogenid der Formel (XIa) 1,0 bis 5,0 Mol, vorzugsweise 1,0 bis 3,0 Mol, an Verbindung der Formel (III) und 1,0 bis 5,0 Mol, vorzugsweise 1,0 bis 3,0 Mol an Säurebindemittel ein.

Reaktionsdurchführung und Aufarbeitung des erfindungsgemäßen Verfahrens (A-$a_6$)/Variante α erfolgen nach bekannten Methoden (vgl. Houben-Weyl, Methoden der organischen Chemie, Bd. X/3, Seite 868, Bd. X/4, Seiten 209 ff und Bd. E/5, Seiten 828 und 1280, Georg Thieme Verlag Stuttgart 1965/1968/1985).

Als Verdünnungsmittel zur Durchführung des erfindungsgemäßen Verfahrens (A-$a_6$)/Variante (β) kommen inerte organische Lösungsmittel infrage. Vorzugsweise verwendet man die bei Verfahren (A-$a_3$)-/Variante (β) genannten Lösungsmittel.

Als Säurebindemittel zur Durchführung des erfindungsgemäßen Verfahrens (A-$a_6$)/Variante (β) kommen alle üblichen anorganischen oder organischen Basen infrage. Vorzugsweise verwendet man die bei Verfahren (A-$a_3$)/Variante (α) genannten Säurebindemittel.

Die Reaktionstemperaturen können bei der Durchführung des erfindungsgemäßen Verfahrens (A-$a_6$)-/Variante (β) innerhalb eines größeren Bereiches variiert werden. Im allgemeinen arbeitet man bei Temperaturen zwischen +20°C und +200°C, vorzugsweise zwischen +50°C und +150°C.

Das erfindungsgemäße Verfahren (A-$a_6$)/Variante (β) wird im allgemeinen unter Normaldruck durchgeführt.

Zur Durchführung des erfindungsgemäßen Verfahrens (A-$a_6$)/Variante (β) setzt man pro Mol Hydroxamsäure der Formel (XIb) 1,0 bis 5,0 Mol, vorzugsweise 1,0 bis 3,0 Mol, an Alkylierungsmittel der Formel -(VI) und gegebenenfalls 1,0 bis 5,0 Mol, vorzugsweise 1,0 bis 3,0 Mol an Säurebindemittel ein.

Reaktionsdurchführung und Aufarbeitung erfolgen bei den erfindungsgemäßen Verfahren (A-$a_6$)/Variante ($\beta$) nach bekannten Methoden (vgl. Houben-Weyl, Methoden der organischen Chemie, Bd. E/5, Seiten 827 und 1287 Georg Thieme Verlag Stuttgart 1985).

Als Verdünnungsmittel zur Durchführung des erfindungsgemäßen Verfahrens (A-$a_6$)/Variante ($\gamma$) kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester Sulfoxide oder Sulfone, wie Dimethylsulfoxid oder Sulfolan, oder Alkohole, wie Methanol, Ethanol oder Propanol.

Die Reaktionstemperaturen können bei dem erfindungsgemäßen Verfahren (A-$a_6$)/Variante ($\gamma$) innerhalb eines größeren Bereiches variiert werden. Im allgemeinen arbeitet man bei Temperaturen zwischen +30°C und +200°C, vorzugsweise zwischen +50°C und +120°C.

Das erfindungsgemäße Verfahren (A-$a_6$)/Variante ($\gamma$) wird im allgemeinen unter Normaldruck durchgeführt.

Zur Durchführung des erfindungsgemäßen Verfahrens setzt man pro Mol Thiobenzoesäureester der Formel (Ib) 1,0 bis 5,0 Mol, vorzugsweise 1,0 bis 3,0 Mol Hydroxylamin der Formel (XII) ein.

Reaktionsdurchführung und Aufarbeitung erfolgen bei dem erfindungsgemäßen Verfahren (A-$a_6$)/Variante ($\gamma$) nach bekannten Methoden (vgl. Houben-Weyl, Methoden der organischen Chemie, Bd. E/5, Seiten 826 und 829 Georg Thieme Verlag Stuttgart 1985).

Als Verdünnungsmittel zur Durchführung des erfindungsgemäßen Verfahrens (A-$a_7$)/Variante ($\alpha$) kommen inerte organische Lösungsmittel infrage. Vorzugsweise verwendet man die bei Verfahren (A-$a_7$)-/Variante ($\beta$) genannten Lösungsmittel.

Das erfindungsgemäße Verfahren (A-$a_7$)/Variante ($\alpha$) wird in Gegenwart eines Katalysators durchgeführt. Vorzugsweise verwendet man Metallsalze wie Cadmiumacetat, Zinkchlorid oder Zinkacetat.

Die Reaktionstemperaturen können bei der Durchführung des erfindungsgemäßen Verfahrens (A-$a_7$)-/Variante ($\alpha$) innerhalb eines größeren Bereiches variiert werden. Im allgemeinen arbeitet man bei Temperaturen zwischen 25°C und 200°C, vorzugsweise zwischen 50°C und 150°C.

Das erfindungsgemäße Verfahren (A-$a_7$)/Variante ($\alpha$) wird im allgemeinen unter Normaldruck durchgeführt.

Zur Durchführung des erfindungsgemäßen Verfahrens (A-$a_7$)/Variante ($\alpha$) setzt man pro Mol Benzonitril der Formel (XVI) 1,0 bis 5,0 Mol, vorzugsweise 1,0 bis 3,0 Mol an 2-bzw. 3-Hydroxyalkylamin der Formel -(XVII) und 0,01 bis 0,5 Mol, vorzugsweise 0,05 bis 0,3 Mol an Katalysator ein.

Die Reaktionsdurchführung des erfindungsgemäßen Verfahrens (A-$a_7$)/Variante ($\alpha$) erfolgt nach bekannten Methoden (vgl. Angewandte Chemie 84, 343 (1972)).

Als Verdünnungsmittel zur Durchführung des erfindungsgemäßen Verfahrens (A-$a_7$)/Variante ($\beta$) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester Sulfoxide oder Sulfone, wie Dimethylsulfoxid oder Sulfolan, oder Alkohole, wie Methanol, Ethanol oder Propanol.

Als Säurebindemittel zur Durchführung des erfindungsgemäßen Verfahrens (A-$a_7$)/Variante ($\beta$) kommen alle üblichen anorganischen oder organischen Basen infrage. Vorzugsweise verwendet man tertiäre Amine, wie Triethylamin, Pyridin oder N,N-Dimethylanilin, Erdalkalimetalloxide, wie Magnesium-und Calciumoxid, Alkali-und Erdalkalimetallcarbonate, wie Natriumcarbonat, Kalium-oder Calciumcarbonat, Alkalimetallhydroxide, -hydride oder -alkoholate, wie Natriumhydroxid oder Kaliumhydroxid, Natriumhydrid, Natriummethylat oder Kalium-t-butylat.

Die Reaktionstemperaturen können bei der Durchführung des erfindungsgemäßen Verfahrens (A-$a_7$)-/Variante ($\beta$) innerhalb eines größeren Bereiches variiert werden. Im allgemeinen arbeitet man bei Temperaturen zwischen 0°C und 150°C, vorzugsweise zwischen 20°C und 120°C.

Das erfindungsgemäße Verfahren (A-$a_7$)/Variante ($\beta$) wird im allgemeinen unter Normaldruck durchgeführt.

Zur Durchführung des erfindungsgemäßen Verfahrens (A-$a_7$)/Variante ($\beta$) setzt man pro Mol Thiobenzamid der Formel (Xa) 1,0 bis 5,0 Mol, vorzugsweise 1,0 bis 3,0 Mol an $\alpha$-Halogencaronyl-Derivat der Formel (XV) und 1,0 bis 5,0 Mol, vorzugsweise 1,0 bis 3,0 Mol an Säurebindemittel ein.

Als Verdünnungsmittel zur Durchführung des erfindungsgemäßen Verfahrens (A-$a_7$)/Variante ($\gamma$) kommen inerte organische Lösungsmittel infrage. Vorzugsweise verwendet man die bei Verfahren (A-$a_6$)-/Variante ($\beta$) genannten Verdünnungsmittel.

Als Säurebindemittel zur Durchführung des erfindungsgemäßen Verfahrens (A-$a_7$)/Variante ($\gamma$) kommen alle üblichen anorganischen oder organischen Basen infrage. Vorzugsweise verwendet man die bei Verfahren (A-$a_6$)/Variante ($\beta$) genannten Säurebindemittel.

Die Reaktionstemperaturen können bei der Durchführung des erfindungsgemäßen Verfahrens (A-$a_7$)-/Variante ($\gamma$) innerhalb eines größeren Bereiches variiert werden. Im allgemeinen arbeitet man bei Temperaturen zwischen 30°C und 200°C, vorzugsweise zwischen 50°C und 150°C.

Das erfindungsgemäße Verfahren (A-$a_7$)/Variante ($\gamma$) wird im allgemeinen unter Normaldruck durchgeführt.

Zur Durchführung des erfindungsgemäßen Verfahrens (A-$a_7$)/Variante ($\gamma$) setzt man pro Mol Hydroxamsäure der Formel (XIb-1) 1,0 bis 5,0 Mol, vorzugsweise 1,0 bis 3,0 Mol an bifunktionellem Alkylierungsmittel der Formel (VIa) und 1,0 bis 10,0 Mol, vorzugsweise 1,0 bis 6,0 Mol an Säurebindemittel ein.

Als Verdünnungsmittel zur Durchführung des erfindungsgemäßen Verfahrens (A-$a_7$)/Variante ($\delta$) kommen inerte organische Lösungsmittel infrage. Vorzugsweise verwendet man die bei Verfahren (A-$a_7$)/Variante ($\beta$) genannten.

Als Säurebindemittel zur Durchführung des erfindungsgemäßen Verfahrens (A-$a_7$)/Variante ($\delta$) kommen alle üblichen anorganischen oder organischen Basen infrage. Vorzugsweise verwendet man tertiäre Amine, wie Triethylamin, Pyridin oder N,N-Dimethylanilin, Erdalkalimetalloxide, wie Magne sium-und Calciumoxid, Alkali-und Erdalkalimetallcarbonate, wie Natriumcarbonat, Kalium-oder Calciumcarbonat, Alkalimetallhydroxide, -hydride oder -alkoholate, wie Natriumhydroxid oder Kaliumhydroxid, Natriumhydrid, Natriummethylat oder Kalium-t-butylat.

Die Reaktionstemperaturen können bei der Durchführung des erfindungsgemäßen Verfahrens (A-$a_7$)-/Variante ($\delta$) innerhalb eines größeren Bereiches variiert werden. Im allgemeinen arbeitet man bei Temperaturen zwischen 0°C und +200°C, vorzugsweise zwischen +20°C und +150°C.

Das erfindungsgemäße Verfahren (A-a )/Variante ($\delta$) wird im allgemeinen unter Normaldruck durchgeführt.

Zur Durchführung des erfindungsgemäßen Verfahrens (A-$a_7$)/Variante ($\delta$) setzt man pro Mol Benzamidoxim der Formel (XIII) 1,0 bis 5,0 Mol, vorzugsweise 1,0 bis 3,0 Mol an Acylierungsmittel der Formel (XIV) und gegebenenfalls 1,0 bis 5,0 Mol, vorzugsweise 1,0 bis 3,0 Mol an wasserentziehendem Mittel ein.

Eine umfassende Darstellung der Reaktionsbedingungen für Verfahren (A-$a_7$)/Variante ($\delta$) findet sich in A. Weissberger, The Chemistry of Heterocyclic Compounds Vol. 17, Seiten 245 ff, Interscience Publisher - (1962).

Als Verdünnungsmittel zur Durchführung des erfindungsgemäßen Verfahrens (B-$b_1$) kommen alle üblichen inerten organischen Lösungsmittel infrage. Vorzugsweise verwendet man Kohlenwasserstoffe, wie beispielsweise Benzin, Benzol, Toluol und Xylol, ferner Ether, wie Dioxan, Glykoldimethylether und Diglykoldimethylether, außerdem Nitrile, wie Acetonitril und auch stark polare Solventien, wie Dimethylsulfoxid, Sulfolan, Dimethylformamid und N-Methylpyrrolidon oder Amine, wie Pyridin oder Chinolin.

Als Säurebindemittel zur Durchführung des erfindungsgemäßen Verfahrens (B-$b_1$) kommen alle für derartige Umsetzungen üblicherweise verwendbaren Säureakzeptoren infrage, Vorzugsweise verwendet man Alkali-und Erdalkalioxide, -hydroxide und -carbonate, wie Natriumhydroxid, Kaliumhydroxid, Calciumoxid, Natriumcarbonat und Kaliumcarbonat, ferner Alkali-alkoholate, -amide und -hydride, wie zum Beispiel Natrium-methylat, Natriumethylat, Kalium-tert.-butylat, Natrium-amid und Natrium-hydrid, sowie tertiäre Amine Triethylamin, N,N-Dimethylanilin, Pyridin oder N,N-Dimethylaminopyridin.

Verwendet man Basen wie beispielsweise Pyridin oder Chinolin als Lösungsmittel, so können diese mit besonderem Vorzug in entsprechendem Überschuß gleichzeitig als Säurebindemittel eingesetzt werden.

Die Reaktionstemperaturen können bei der Durchführung des Verfahrens (B-$b_1$) innerhalb eines größeren Bereiches variiert werden. Im allgemeinen arbeitet man bei Temperaturen zwischen 0°C und 200°C, vorzugsweise zwischen 50°C und 150°C.

Das erfindungsgemäße Verfahren (B-$b_1$) wird im allgemeinen unter Normaldruck durchgeführt.

Zur Durchführung des erfindungsgemäßen Verfahrens (B-$b_1$) setzt man pro Mol Pyri(mi)din-Derivat der Formel (XVIII) im allgemeinen 0,5 bis 10,0 Mol, vorzugsweise 0,5 bis 2,0 Mol an 4-Hydroxy-oder 4-Mercaptobenzoesäure-Derivat der Formel (XIX) und 1,0 bis 10,0 Mol, vorzugsweise 1,0 bis 3,0 Mol an Säurebindemittel ein. Die Reaktionsdurchführung, Aufarbeitung und Isolierung der Reaktionsprodukte der Formel (I) erfolgt in üblicher Art und Weise.

Als Verdünnungsmittel zur Durchführung des erfindungsgemäßen Verfahrens (B-$b_2$) kommen alle üblichen inerten organischen Lösungsmittel infrage. Vorzugsweise verwendet man die bei Verfahren (B-$b_1$) genannten Lösungsmittel.

Als Säurebindemittel zur Durchführung des erfindungsgemäßen Verfahrens (B-$b_2$) kommen alle für derartige Umsetzungen üblicherweise verwendbaren Säureakzeptoren infrage. Vorzugsweise verwendet man die bei Verfahren (B-$b_1$) genannten Säurebindemittel.

Die Reaktionstemperaturen können bei der Durchführung des erfindungsgemäßen Verfahrens (B-$b_2$) in einem größeren Bereich variiert werden. Im allgemeinen arbeitet man bei Temperaturen zwischen 0°C und 200°C, vorzugsweise zwischen 50°C und 150°C.

Im allgemeinen arbeitet man bei der Durchführung des Verfahrens (B-$b_2$) unter Normaldruck.

Zur Durchführung des erfindungsgemäßen Verfahrens (B-$b_2$) setzt man pro Mol 2-Mercapto-pyri(mi)din-Derivat der Formel (XX) 0,5 bis 10,0 Mol, vorzugsweise 1,0 bis 2,0 Mol an 4-Halogenbenzoesäure-Derivat der Formel (XXI) und 1,0 bis 10,0 Mol, vorzugsweise 1,0 bis 3,0 Mol an Säurebindemittel ein. Die Reaktionsdurchführung, Aufarbeitung und Isolierung der Reaktionsprodukte der Formel (I) erfolgt nach allgemein üblichen Methoden.

Als Verdünnungsmitel zur Durchführung des Herstellungsverfahrens (C) werden die für den jeweiligen Reaktionstyp üblichen Lösungsmittel eingesetzt.

So werden Halogenierungsreaktionen (Verfahren (C-$c_1$)) vorzugsweise in Halogenkohlenwasserstoffen wie Dichlormethan, Chloroform, Tetrachlormethan oder Chlorbenzol durchgeführt.

Die Reaktionstemperaturen können in einem größeren Bereich variiert werden. Im allgemeinen arbeitet man bei Temperaturen zwischen -20°C und +150°C, vorzugsweise zwischen 0°C und +100°C.

Das Halogenierungsmittel wird vorzugsweise in stöchiometrischer Menge oder mit einem Überschuß bis zu 0,3 Mol eingesetzt, gegebenenfalls setzt man 0,01 bis 0,1 Mol, vorzugsweise 0,01 bis 0,05 Mol Katalysator zu.

Die Reduktion von Nitroverbindungen zu Aminoverbindungen (Verfahren (C-$c_2$)) erfolgt mit katalytisch erregtem Wasserstoff, vorzugsweise in Alkoholen oder cyclischen Ethern wie Tetrahydrofuran oder Dioxan als Lösungsmittel.

Die Reaktionstemperaturen können in einem breiteren Bereich variiert werden. Diese betragen 0°C bis 150°C, vorzugsweise 10°C bis 100°C.

Als Katalysator wird Raney Nickel bevorzugt, das mit 0,01 bis 0,1 Mol eingesetzt wird. Vorzugsweise arbeitet man unter Druck zwischen 1 und 50 bar. Die Reduktion mit Metallen wie Eisen, Zink oder Zinn, Metallsalzen in niederen Wertigkeitsstufen wie Eisen-II-salze, Zinn-II-salze oder mit Verbindungen des Schwefels in niederen Wertigkeitsstufen wie Sulfide, Sulfite oder Dithionite wird vorzugsweise in Wasser, gegebenenfalls im Gemisch mit Alkoholen durchgeführt und (im Falle metallischer Reduktionsmittel) in gleichzeitiger Gegenwart von Alkalien oder Mineralsäuren. Im Falle metallischer Reduktionsmittel kann auch in Essigsäure gearbeitet werden.

Die Reaktionstemperaturen betragen 0 bis 120°C, vorzugsweise 20 bis 100°C. Das Reduktionsmittel wird mit mindestens 1 Mol, vorzugsweise in einem geeigneten Überschuß von 1,0 bis 3,0 Mol, eingesetzt.

Die Acylierung von Verbindungen mit einer Aminogruppe im Phenylteil (Verfahren (C-$c_3$)) erfolgt unter den hierfür grundsätzlich bekannten Bedingungen. Als Verdünnungsmittel sid alle gegenüber Säurehalogeniden bzw. -anhydriden inerte organische Lösungsmittel geeignet, die z.B. auch für das Herstellungsverfahren (A-$a_1$) aufgeführt sind.

Als säurebindende Mittel werden Alkali-und Erdalkalicarbonate oder tertiäre Amine wie Triethylamin, N,N-Dimethylanilin oder Pyridin verwendet.

Die Reaktionstemperaturen können in einem größeren Bereich variiert werden. In allgemeinen arbeitet man zwischen -20°C und +150°C, vorzugsweise bei 0°C bis +100°C.

Austauschreaktionen von Aminogruppen gegen Halogen oder Cyan am Phenylteil (Verfahren (C-$c_4$) werden unter den für solche Reaktionen grundsätzlich bekannten Bedingungen durchgeführt. Eine ausführliche Beschriebung dieser als Sandmeyer-Reaktion bekannten Methode findet sich in Houben-Weyl, Methoden der organischen Chemie, Bd. 5/3, Seiten 846 ff, Georg Thieme Verlag Stuttgart 1985. Hierzu läßt man eine aus der Aminoverbindung in verdünnter Mineralsäure mit Nitrit erzeugte Diazoniumsalzlösung in eine Kupfer-I-salzlösung laufen. Die Umsetzung erfolgt unter Stickstoffabspaltung. Die Anfangstemperatur kann zwischen 0°C und +75°C schwanken. Im allgemeinen wird die Reaktion durch Erwärmen auf 70°C

bis 100°C zu Ende geführt. Falls die verwendete Mineralsäure das gleiche Anion wie das Kupfersalz besitzt (z.B. $Cl^{\ominus}$) genügen 0,1 bis 0,2 Mol Kupfersalz als Katalysator, in anderen Fällen verwendet man z.B. Schwefelsäure zur Bereitung des Diazoniumsalzes und benötigt dann mindestens die stöchiometrische Menge des Kupfer-I-salzes.

Als Verdünnungsmittel zur Durchführung des erfindungsgemäßen Verfahrens (D) kommen alle üblichen inerten organischen Lösungmittel infrage. Vorzugsweise verwendet man die bei Verfahren ($B-b_1$) genannten Lösungsmittel.

Verwendet man als Reaktionskomponente Alkohole der Formel (XXII) in flüsiger Form, so können diese mit besonderem Vorzug in entsprechendem Überschuß gleichzeitig als Verdünnungsmittel eingesetzt werden.

Als Säurebindemittel zur Durchführung des erfindungsgemäßen Verfahrens (D) kommen alle für derartige Umsetzungen üblicherweise verwendbaren Säureakzeptoren infrage. Vorzugsweise verwendet man die bei Verfahren ($B-b_1$) genannten Basen. Es ist auch möglich, das Amin der Formel (XXIII) in entsprechendem Überschuß gleichzeitig als Säurebindemittel einzusetzen.

Die Reaktionstemperaturen können bei der Durchführung des erfindungsgemäßen Verfahrens (D) in einem größeren Bereiches variiert werden. Im allgemeinen arbeitet man bei Temperaturen zwischen 0°C und 150°C, vorzugsweise zwischen 20°C und 100°C.

Die Umsetzungen mit Ammoniak verlaufen langsamer, so daß man mit gasförmigem Ammoniak in höher siedenden Lösungsmitteln bei 80°C bis 150°C arbeiten muß oder aber die Reaktionen in geschlossenen Gefäßen unter Druck durchführt.

Zur Durchführung des erfindungsgemäßen Verfahrens (D) setzt man pro Mol Pyri(mi)dyl-oxy-bzw. -thiobenzoesäure-Derivat der Formel (If) 1,0 bis 5,0 Mol, vorzugsweise 1,0 bis 3,0 Mol an Alkohol der Formel - (XXII) oder 1,0 bis 5,0 Mol, vorzugsweise 1,0 bis 3,0 Mol an Ammoniak oder Amin der Formel (XXIII) und 1,0 bis 5,0 Mol, vorzugsweise 1,0 bis 3,0 Mol an Säurebindemittel ein. Die Reaktionsdurchführung, Aufarbeitung und Isolierung der Reaktionsprodukte erfolgt nach grundsätzlich bekannten Methoden.

Die erfindungsgemäßen Wirkstoffe können als Defoliants, Desiccants, Krautabtötungsmittel und insbesondere als Unkrautvernichtungsmittel verwendet werden: Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrieund Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämfpung mono-und dikotyler Unkräuter, vorzugsweise in monokotylen Kulturen wie beispielsweise Weizen einsetzen.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe, Feinstverkapselungen in polymeren Stoffen und in Hüllmassen für Saatgut, sowie ULV-Formulierungen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Auch Mischungen mit N-Benzthiazolyl-N-methyl-N'-methyl-harnstoff, N,N-Dimethyl-N'-(4-isopropylphenyl)-harnstoff, N,N-Dimethyl-N'-(3-chlor-4-methylphenyl)-harnstoff, 2-Chlor-N-{[(4-methoxy-6-methyl-1,3,5-triazin-2-yl)-amino]-carbonyl}-benzolsulfonamid, 2-Chlor-4-ethylamino-6-isopropylamino-1,3,5-triazin, 2-Chlor-4-ethylamino-6-cyanopropylamino)-1,3,5-triazin, 4-Ethylamino-2-tert.-butylamino-6-methylthio-5-triazin, 4-Amino-6-tert.-butyl-3-methylthio-4,5-dihydro-1,2,4-triazin-5-on, 2-[4-(2,4-Dichlorphenoxy)-phenoxy]-propionsäuremethylester, 2-[4-(3,5-Dichlorpyrid-2-yloxy)-phenoxy]-propionsäure-trimethylsilylmethylester, 2,4-Dichlorphenoxyessigsäure, 2,4-Dichlorphenoxypropionsäure, (2-Methyl-4-chlorphenoxy)-essigsäure, (4-Chlor-2-methyl-phenoxy)-propionsäure, 3,5-Diiodo-4-hydroxybenzonitril, Methyl-5-(2,4-dichlorphenoxy)-2-nitrobenzoat, 3,6-Dichlor-picolinsäure, 3-Isopropyl-2,1,3-benzothiadiazin-4-on-2,2-dioxid und 6-Chlor-3-phenyl-pyridazin-4-yl-5-octylthiocarbonat sind gegebenenfalls von Vorteil. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Bei der Verwendung des Wachstumsregulatoren können die erfindungsgemäßen Wirkstoffe in den Formulierungen ebenfalls in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Aufwandmengen können bei der Anwendung als Wachstumsregulator ebenfalls in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

Herstellungsbeispiele

Beispiel 1

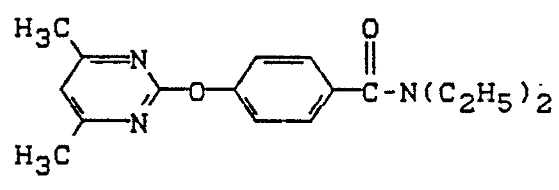

Verfahren A-a,

24,4 g (0,1 Mol) 4-(4,6-Dimethyl-pyrimidyl-2-oxy)-benzoesäure werden in 200 ml Toluol suspendiert. Hierzu gibt man 10,1 g (0,1 Mol) Triethylamin und tropft nach ca. 30 Minuten bei Raumtemperatur 13,1 g - (0,11 Mol) Thionylchlorid zu. Die Mischung wird 4 Stunden unter Rückfluß gekocht. Der ausgefallene Niederschlag wird abgesaugt, die Lösung i. Vak. eingedampft. Der verbleibende Rückstand wird in 150 ml Tetrachlormethan aufgenommen, die Lösung von unlöslichen Bestandteilen dekantiert, mit Kohle verrührt, filtriert und wieder eingedampft. Der Rückstand wird in 150 ml Tetrahydrofuran gelöst und bei 10 bis 15°C tropfenweise mit 14,6 g (0,2 Mol) Diethylamin versetzt. Der Niederschlag von Diethylamin-hydrochlorid wird abgesaugt, die Lösung i. Vak. eingedampft. Der Rückstand wird mit schwach angesäuertem Wasser verrührt, abgesaugt, neutral gewaschen und getrocknet.

Man erhält 25 g (83,6 % der Theorie) 4-(4,6-Dimethylpyrimidyl-2-oxy)-benzoesäure-diethylamid vom Schmelzpunkt Fp. 95 bis 96°C (Umkristallisation aus Waschbenzin).

Beispiel 2

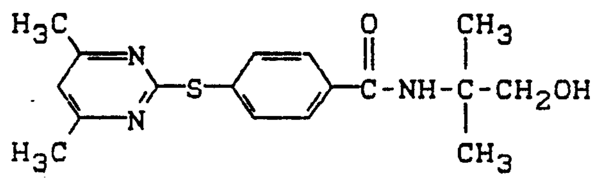

Verfahren (A-a,)

13 g (0,05 Mol) 4-(4,6-Dimethyl-pyrimidyl-2-mercapto)-benzoesäure und 5,1 g (0,05 Mol) Triethylamin werden in 100 ml Tetrahydrofuran gelöst. Hierzu tropft man unter Rühren und Kühlen bei 10 bis 15°C 5,5 g (0,05 Mol) Chlorameisensäure-ethylester. Die Mischung wird 2 Stunden bei Raumtemperatur nachgerührt und anschließend mit 4,9 g (0,05 Mol) 2-Amino-2-methyl-propanol versetzt. Die Mischung wird 1 Stunde bei Raumtemperatur nachgerührt und anschließend 2 Stunden unter Rückfluß gekocht und nach dem Abkühlen mit 500 ml Wasser verdünnt. Die abgeschiedenen Kristalle werden abgesaugt, 30 Minuten in einer 1-%igen Natronlauge verrührt, erneut abgesaugt, mit Wasser neutral gewaschen und getrocknet.

Man erhält 10,5 g (63,5 % der Theorie) 4-(4,6-Dimethylpyrimidyl-2-mercapto)-benzoesäure-(2-methyl-3-hydroxypropyl-2)-amid vom Schmelzpunkt Fp. 90 bis 91°C (Umkristallisation aus Toluol).

Beispiel 3

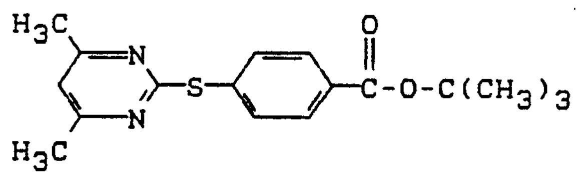

Verfahren (A-a,)

6,5 g (25 mmol) 4-(4,6-Dimethyl-pyrimidyl-2-mercapto)-benzoesäure werden in 50 ml absolutem Pyridin gelöst. Unter Kühlung bei 15 bis 20°C tropft man 6,4 ml (50 mmol) Benzolsulfochlorid zu und verrührt 1 Stunde bei Raumtemperatur. Nach Abkühlen im Eisbad werden 2,4 ml (25 mmol) tert.-Butanol zugetropft, mit 100 mg Steglich-Base versetzt und 1 Stunde bei Raumtemperatur gerührt. Man erwärmt 6 Stunden auf 60°C und gießt die Lösung anschließend in Eiswasser ein. Die Mischung wird mit Ether extrahiert, die Etherlösung getrocknet, im Vakuum eingedampft und als Lösung in Cyclohexan/Essigester im Verhältnis 2:1 über eine Säule mit Kieselgel filtriert.

Man erhält nach Eindampfen des Eluats 3,77 g (47,7 % der Theorie) 4-(4,6-Dimethyl-pyrimidyl-2-mercapto)-benzoesäure-tert.-butylester vom Schmelzpunkt Fp. 75 bis 77°C (Umkristallisation aus Cyclohexan/n-Hexan).

Beispiel 4

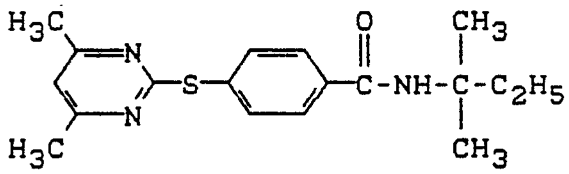

Verfahren (A-a₁)

13 g (0,05 Mol) 4-(4,6-Dimethyl-pyrimidyl-2-mercapto)-benzoesäure, 4,4 g (0,05 Mol) 1,1-Dimethylpropylamin und 15,2 g (0,15 Mol) Triethylamin werden in 200 ml Dichlormethan verrührt. Hierzu tropft man bei Raumtemperatur 7,7 g (0,05 Mol) Phosphoroxychlorid. Die Mischung wird 1 Stunde unter Rückfluß gekocht und nach dem Abkühlen in 500 ml Eiswasser eingerührt. Die organische Phase wird abgetrennt, mit Wasser, ca. 1-%iger Natronlauge und wieder mit Wasser gewaschen, getrocknet und im Vakuum eingedampft.

Man erhält 13,5 g (82 % der Theorie) 4-(4,6-Dimethyl-pyrimidyl-2-mercapto)-benzoesäure-1,1-dimethyl-propylamid vom Schmelzpunkt Fp. 129 bis 130°C (Umkristallisation aus Waschbenzin).

Beispiel 5

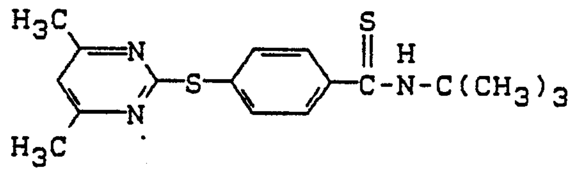

Verfahren (A-a₄)/Variante γ

4,7 g (15 mmol) 4-(4,6-Dimethyl-pyrimidyl-2-mercapto)-benzoesäure-tert.-butylamid und 3,3 g (7,8 mmol) Lawesson-Reagenz werden in 20 ml absolutem Toluol 2 Stunden unter Rückfluß gekocht. Nach Abkühlen auf Raumtemperatur gibt man die Lösung auf Kieselgel und chromatographiert mit Cyclohexan/Essigester im Verhältnis 2:1.

Man erhält nach Abdestillieren des Lösungsmittels 3,5 g (71 % der Theorie) 4-(4,6-Dimethyl-pyrimidyl-2-mercapto)-benzoesäure-tert.-butylthioamid vom Schmelzpunkt Fp. 110 bis 111°C (Umkristallisation aus Toluol/Petrolether).

Beispiel 6

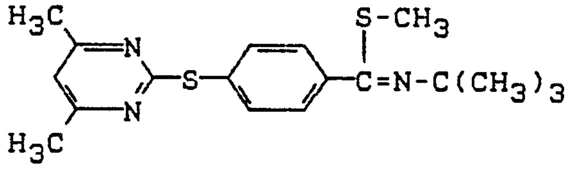

Verfahren ($A-a_5$)/Variante β

0,6 g (20 mmol) Natriumhydrid werden unter Stickstoff in 20 ml absolutem Dimethylformamid vorgelegt. Dazu tropft man bei Raumtemperatur die Lösung von 6,62 g (20 mmol) 4-(4,6-Dimethyl-pyrimidyl-2-mercapto)-benzoesäure-tert.-butyl-thioamid in 20 ml absolutem Dimethylformamid und rührt bis keine Wasserstoff-Entwicklung mehr zu beobachten ist. Dann tropft man bei Raumtemperatur unter Rühren 5,25 g (37,5 mmol) Methyliodid ein und rührt noch 2 Stunden bei Raumtemperatur nach. Die Mischung wird in 250 ml Wasser eingegossen, mit Dichlormethan extrahiert, die organische Lösung getrocknet und eingedampft.

Als Rückstand verbleiben 4,6 g (66,8 % der Theorie) 4-(4,6-Dimethyl-pyrimidyl-2-mercapto)-thiobenzoesäure-methylester-tert.-butylimid vom Schmelzpunkt Fp. 88 bis 90°C (Umkristallisation aus Toluol/n-Hexan).

Beispiel 7

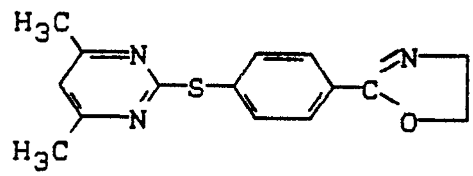

Verfahren ($A-a_7$)/Variante α

7,42 g (30 mmol) 4-(4,6-Dimethyl-pyrimidyl-2-mercapto)-benzonitril, 2,72 ml (45 mmol) 2-Hydroxyethylamin und 250 mg wasserfreies Zinkchlorid werden 4 Stunden auf 150°C erwärmt. Nach erfolgter Umsetzung fügt man 30 ml Tetrahydrofuran hinzu, gießt auf Wasser und extrahiert mit Dichlormethan. Die organische Phase wird getrocknet und im Vakuum eingedampft, der Rückstand mit Cyclohexan/Essigester im Verhältnis 1:2 chromatographiert.

Man erhält 3,5 g (40,8 % der Theorie) 2-[4-(4,6-Dimethyl-pyrimidyl-2-mercapto-phenyl)]-1,3-oxazolin vom Schmelzpunkt Fp. 98 bis 99°C (Umkristallisation aus Toluol/n-Hexan).

Beispiel 8

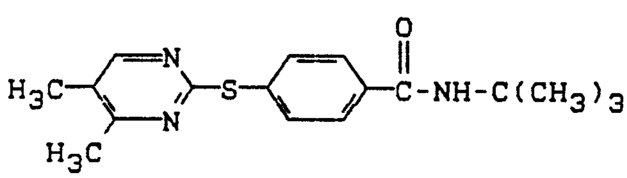

Verfahren ($B-b_1$)

10,45 g (0,05 Mol) 4-Mercapto-benzoesäure-tert.-butylamid werden in 50 ml Sulfolan gelöst und bei Raumtemperatur portionsweise mit 2,8 g (0,05 Mol) pulverigem Kaliumhydroxid versetzt. Man verrührt 30 Minuten bei Raumtemperatur und gibt dann 7,1 g (0,05 Mol) 2-Chlor-4,5-dimethyl-pyrimidin zu. Die Mischung wird allmählich auf 110°C erhizt und noch 5 Stunden bei dieser Temperatur gerührt. Nach dem Abkühlen gießt man in 1 Liter Wasser ein. Die wäßrige Phase wird dekantiert, das halbfeste Reaktionsprodukt in Toluol aufgenommen. Die Lösung wird einmal mit ca. 1-%iger Natronlauge und noch zweimal mit Wasser gewaschen, über Natriumsulfat getrocknet und im Vakuum eingedampft.

Als Rückstand verbleiben 10,7 g (68 % der Theorie) 4-(4,5-Dimethyl-pyrimidyl-2-mercapto)-benzoesäure-tert.-butylamid vom Schmelzpunkt Fp. 172 bis 128°C (Umkristallisation aus Waschbenzin).

Beispiel 9

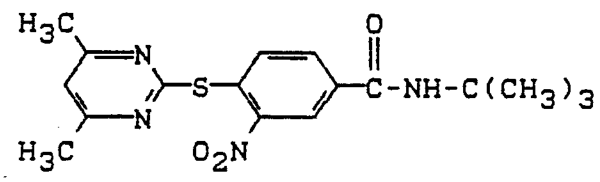

Verfahren (B-$b_2$)

35,3 g (0,2 Mol) 2-Mercapto-4,6-dimethyl-pyrimidin-hydrochlorid werden in 170 ml Sulfolan suspendiert. Hierzu gibt man bei Raumtemperatur unter Rühren in Portionen 24,7 g (0,44 Mol) pulvriges Kaliumhydroxid. Man verrührt noch 30 Minuten bei Raumtemperatur und trägt dann 51,3 g (0,2 Mol) 3-Nitro-4-chlor-benzoesäure-tert.-butylamid ein. Die Mischung wird 2 Stunden bei 50°C und 4 Stunden bei 120°C gerührt, anschließend abgekühlt und in 1 Liter Eiswasser eingerührt. die abgeschiedenen Kristalle werden abgesaugt und aus Ethanol umkristallisiert.

Man erhält 58,6 g (81,3 % der Theorie) 3-Nitro-4-(4,6-dimethyl-pyrimidyl-2-mercapto-)benzoesäure-tert.-butylamid vom Schmelzpunkt Fp. 157 bis 158°C.

Beispiel 10

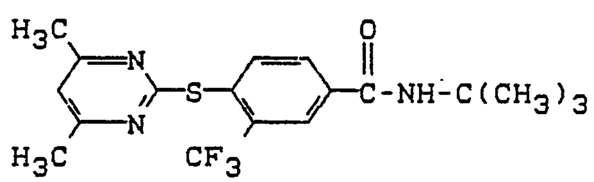

In entsprechender Weise erhält man aus 4-Chlor-3-trifluormethyl-benzoesäure-tert.-butylamid nach Verfahren (B-$b_2$) 3-Trifluormethyl-4-(4,6-dimethyl-pyrimidyl-2-mercapto)-benzoesäure-tert.-butylamid vom Schmelzpunkt Fp. 131°C (Umkristallisation aus Waschbenzin).

Beispiel 11

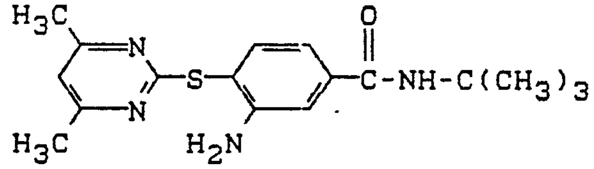

Verfahren (C-$c_1$)

36 g (0,1 Mol) 3-Nitro-4-(4,6-dimethyl-pyrimidyl-2-mercapto)-benzoesäure-tert.-butylamid werden in 350 ml Dioxan im Autoklaven unter Zusatz von 7 g Raney-Nickel bei 20 bis 50°C erschöpfend hydriert. Der Katalysator wird abgesaugt, die Lösung im Vakuum eingedampft. Als Rückstand verbleiben ca. 32 g - (Ausbeute praktisch quantitativ) 3-Amino-4-(4,6-dimethyl-pyrimidyl-2-mercapto)-benzoesäure-tert.-butylamid vom Schmelzpunkt Fp. 182 bis 183°C (Umkristallisation aus Toluol).

Beispiel 12

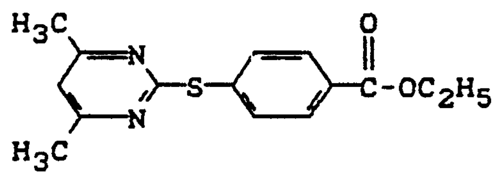

Verfahren (B-$b_1$)

91 g (0,5 Mol) 4-Mercapto-benzoesäureethylester werden in 250 ml Dimethylsulfoxid gelöst. Hierzu trägt man 28 g (0,5 Mol) pulverisiertes Kaliumhydroxid unter Rühren ein. Nach Abklingen der leicht exothermen Reaktion (30 Minuten) gibt man bei Raumtemperatur 71,2 g (0,5 Mol) 2-Chlor-4,6-dimethyl-pyrimidin in Portionen zu und erwärmt die Mischung allmählich auf 110°C bis 120°C. Nach 5-stündigem Rühren bei dieser Temperatur kühlt man auf Raumtemperatur ab und verdünnt dann mit 1,5 l Wasser. Die abgeschiedenen Kristalle werden abgesaugt und getrocknet.

Man erhält 118 g (82 % der Theorie) 4-(4,6-Dimethylpyrimidyl-2-mercapto)-benzoesäure-ethylester vom Schmelzpunkt 93°C bis 94°C (Umkristallisation aus Waschbenzin).

Beispiel 13

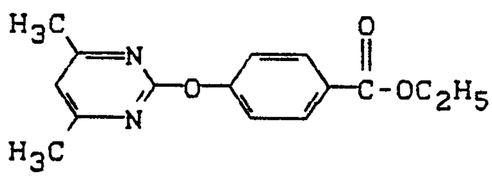

Schmelzpunkt 95°C bis 96°C (Umkristallisation aus Waschbenzin)

Verfahren (B-$b_1$)

Analog zu Beispiel 12 erhält man aus 4-Hydroxy-benzoesäure-ethylester und 2-Chlor-4,6-dimethylpyrimidin den 4-(4,6-Dimethyl-pyrimidyl-2-oxy)-benzoesäureethylester.

In entsprechender Weise zu den Herstellungsbeispielen und gemäß den allgemeinen Angaben zur Herstellung erhält man die in der folgenden Tabelle1 formelmäßig aufgeführten Pyri(mi)dyloxy-und -thiobenzoesäure-Derivate der Formel (I):

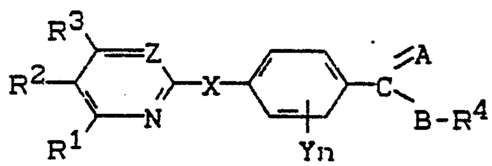

(I)

Tabelle 1

| Beispiel Nr. | Z | $R^1$ | $R^2$ | $R^3$ | X | Y | A | B | $R^4$ | Fp. (°C) |
|---|---|---|---|---|---|---|---|---|---|---|
| 14 | CH | $CH_3$ | H | H | S | - | O | NH | $-C(CH_3)_3$ | 133-134 |
| 15 | CH | $CH_3$ | H | $CH_3$ | S | - | O | NH | $-C(CH_3)_3$ | 142-143 |
| 16 | CH | $CH_3$ | H | Cl | S | - | O | NH | $-C(CH_3)_3$ | 150-152 |
| 16a | CH | H | $CH_3$ | H | S | - | O | NH | $-C(CH_3)_3$ | 106-108 |
| 17 | CH | H | H | $CH_3$ | S | - | O | NH | $-C(CH_3)_3$ | |
| 18 | N | $CH_3$ | H | H | S | - | O | O | $-C(CH_3)_3$ | 45 |
| 19 | N | $CH_3$ | H | H | S | - | O | NH | $-C(CH_3)_3$ | 104-105 |
| 20 | N | $CH_3$ | H | H | S | - | O | NH | 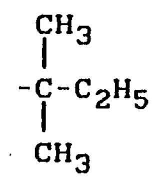 | 88- 90 |
| 21 | N | $CH_3$ | H | H | S | - | O | NH | 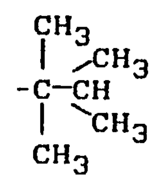 | |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 22 | N | $CH_3$ | H | H | S | - | O | NH | 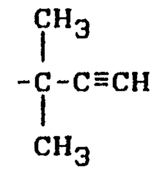 | |
| 23 | N | $CH_3$ | H | H | S | - | O | NH | 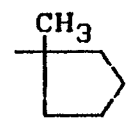 | 90- 91 |
| 24 | N | $CH_3$ | H | H | S | - | O | NH | 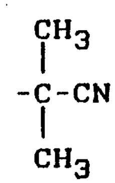 | |
| 25 | N | $CH_3$ | H | H | S | - | O | NH | 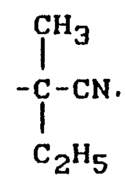 | 118-120 |
| 26 | N | $CH_3$ | H | H | S | - | S | NH | $-C(CH_3)_3$ | 128-129 |
| 26a | N | $CH_3$ | H | H | S | 2-Cl | O | O | $-C(CH_3)_3$ | Öl |
| 27 | N | $CH_3$ | H | H | S | 2-Cl | O | NH | $-C(CH_3)_3$ | |

<u>Tabelle 1</u> (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 28 | N | $CH_3$ | H | H | S | 3-Cl | O | NH | $-C(CH_3)_3$ | |
| 29 | N | $CH_3$ | H | H | S | $2-CH_3$ | O | NH | $-C(CH_3)_3$ | |
| 30 | N | $CH_3$ | H | H | S | $3-CH_3$ | O | NH | $-C(CH_3)_3$ | |
| 31 | N | $CH_3$ | H | H | S | $3-NO_2$ | O | NH | $-C(CH_3)_3$ | 155-156 |
| 32 | N | $CH_3$ | H | H | S | $3-NH_2$ | O | NH | $-C(CH_3)_3$ | 94 |
| 33 | N | $CH_3$ | H | Cl | S | - | O | NH | $-C(CH_3)_3$ | |
| 34 | N | $CH_3$ | H | $OCH_3$ | S | - | O | NH | $-C(CH_3)_3$ | |
| 35 | N | $CH_3$ | H | $OCH(CH_3)_2$ | S | - | O | NH | $-C(CH_3)_3$ | |
| 36 | N | $CH_3$ | H | $O-CH_2-CH=CH_2$ | S | - | O | NH | $-C(CH_3)_3$ | |
| 37 | N | $CH_3$ | H | $O-CH_2-CH_2-OCH_3$ | S | - | O | NH | $-C(CH_3)_3$ | |
| 38 | N | $CH_3$ | H | $NH_2$ | S | - | O | NH | $-C(CH_3)_3$ | 184 |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 39 | N | $CH_3$ | H | $NH-CH_3$ | S | - | O | NH | $-C(CH_3)_3$ | 70- 72 |
| 39a | N | $CH_3$ | H | NH - 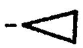 | S | - | O | NH | $-C(CH_3)_3$ | Öl |
| 40 | N | $CH_3$ | H | $NH-C_2H_5$ | S | - | O | NH | $-C(CH_3)_3$ | |
| 41 | N | $CH_3$ | H | $NH-CH_2-CH=CH_2$ | S | - | O | NH | $-C(CH_3)_3$ | |
| 42 | N | $CH_3$ | H | $NH-CH(CH_3)_2$ | S | - | O | NH | $-C(CH_3)_3$ | Öl |
| 42a | N | $CH_3$ | H | $-NH-C(CH_3)_3$ | S | - | O | NH | $-C(CH_3)_3$ | Öl |
| 43 | N | $CH_3$ | H | $NH(CH_3)_2$ | S | - | O | NH | $-C(CH_3)_3$ | 169 |
| 44 | N | $CH_3$ | H | $N(CH_2-CH=CH_2)_2$ | S | - | O | NH | $-C(CH_3)_3$ | |
| 45 | N | $CH_3$ | H | 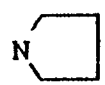 | S | - | O | NH | $-C(CH_3)_3$ | |
| 46 | N | $CH_3$ | H | 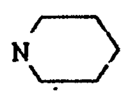 | S | - | O | NH | $-C(CH_3)_3$ | |
| 47 | N | $CH_3$ | H | 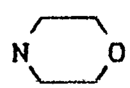 | S | - | O | NH | $-C(CH_3)_3$ | |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 48 | N | $CH_3$ | H | 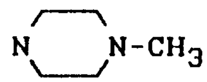 | S | - | O | NH | $-C(CH_3)_3$ | |
| 49 | N | $C_2H_5$ | H | H | S | - | O | NH | $-C(CH_3)_3$ | 114-116 |
| 50 | N | $(CH_3)_2CH$ | H | H | S | - | O | NH | $-C(CH_3)_3$ | |
| 51 | N | H | $CH_3$ | H | S | - | O | O | $-C(CH_3)_3$ | 86- 87 |
| 52 | N | H | $CH_3$ | H | S | - | O | NH | $-C(CH_3)_3$ | 163-164 |
| 53 | N | H | $CH_3$ | H | S | - | S | NH | $-C(CH_3)_3$ | 155-157 |
| 53a | N | H | $CH_3$ | H | S | - | N | $S-CH_3$ | $-C(CH_3)_3$ | 80 |
| 53b | N | H | $CH_3$ | H | S | - | N | $S-C_2H_5$ | $-C(CH_3)_3$ | 48 |
| 53c | N | H | $CH_3$ | H | S | - | N | $S-CH_2CH=CH_2$ | $-C(CH_3)_3$ | 45 |
| 54 | N | H | $C_2H_5$ | H | S | - | O | NH | $-C(CH_3)_3$ | 149-150 |
| 55 | N | H | $C_3H_7$ | H | S | - | O | NH | $-C(CH_3)_3$ | 95- 96 |
| 56 | N | H | $CH(CH_3)_2$ | H | S | - | O | NH | $-C(CH_3)_3$ | 137-138 |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 57 | N | H | $C(CH_3)_3$ | H | S | - | O | NH | $-C(CH_3)_3$ | |
| 58 | N | $CH_3$ | $CH_3$ | H | S | - | O | O | $-C(CH_3)_3$ | Öl |
| 58a | N | $CH_3$ | $CH_3$ | H | S | - | O | O | $-C(CH_3)_2C_2H_5$ | Öl |
| 58b | N | $CH_3$ | $CH_3$ | H | S | 3-$CH_3$ | O | O | $-C(CH_3)_3$ | 55 |
| 58c | N | $CH_3$ | $CH_3$ | H | S | 3-$CH_3$ | O | O | $-C(CH_3)_2C_2H_5$ | 45 |
| 59 | N | $C_2H_5$ | H | H | S | - | O | O | $-C(CH_3)_3$ | 50 |
| 59a | N | $C_2H_5$ | H | H | S | 3-$CH_3$ | O | O | $-C(CH_3)_3$ | 89 |
| 59b | N | $CH_3$ | $CH_3$ | H | S | - | O | NH | 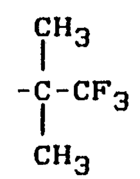 | 106-108 |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 60 | N | $CH_3$ | $CH_3$ | H | S | - | O | NH | 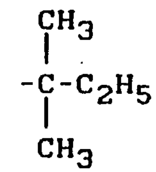 | 109-110 |
| 61 | N | $CH_3$ | $CH_3$ | H | S | - | O | NH | 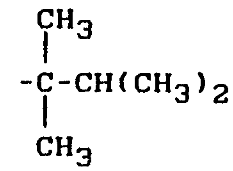 | 137-138 |
| 62 | N | $CH_3$ | $CH_3$ | H | S | - | O | NH | 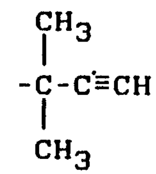 | 108-109 |
| 63 | N | $CH_3$ | $CH_3$ | H | S | - | O | NH | 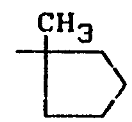 | 125-126 |
| 64 | N | $CH_3$ | $CH_3$ | H | S | - | O | NH | 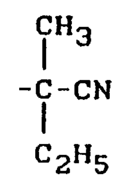 | 125-126 |

<u>Tabelle 1</u> (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 64a | N | $CH_3$ | $CH_3$ | H | S | - | O | NH | $CH_3$<br>$-C-CH_2-C(CH_3)_3$<br>$CH_3$ | 148-149 |
| 65 | N | $CH_3$ | $CH_3$ | H | S | - | O | NH | $CH_3$<br>$-C-CN$<br>$C_3H_7$ | |
| 65a | N | $CH_3$ | $CH_3$ | H | S | - | O | NH | $CH_3$<br>$-C-CH_2-OCH_3$<br>$CH_3$ | 60-61 |
| 65b | N | $CH_3$ | $CH_3$ | H | S | 3-$CH_3$ | O | NH | $-C(CH_3)_3$ | 155 |
| 65c | N | $CH_3$ | $CH_3$ | H | S | 3-$CH_3$ | O | NH | $-C(CH_3)_2C_2H_5$ | 84 |
| 66 | N | $CH_3$ | $CH_3$ | H | S | - | S | NH | $-C(CH_3)_3$ | 144-145 |
| 66a | N | $CH_3$ | $CH_3$ | H | S | - | S | NH | $CH_3$<br>$-C-C_2H_5$<br>$CH_3$ | 120 |

<u>Tabelle 1</u> (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 67 | N | $CH_3$ | $CH_3$ | H | S | - | S | NH | $CH_3$<br>$-C-CH_2-C(CH_3)_3$<br>$CH_3$ | 124-125 |
| 67a | N | $CH_3$ | $CH_3$ | H | S | - | N | $S-CH_3$ | $-C(CH_3)_2C_2H_5$ | 58 |
| 67b | N | $CH_3$ | $CH_3$ | H | S | - | N | $S-CH_2-CH=CH_2$ | $-C(CH_3)_2C_2H_5$ | Öl |
| 68 | N | $CH_3$ | $CH_3$ | Cl | S | - | O | NH | $-C(CH_3)_3$ | |
| 69 | N | $CH_3$ | $CH_3$ | $NH_2$ | S | - | O | NH | $-C(CH_3)_3$ | 198 |
| 70 | N | $CH_3$ | $C_2H_5$ | H | S | - | O | O | $-C(CH_3)_3$ | Öl |
| 71 | N | $CH_3$ | $C_2H_5$ | H | S | - | O | NH | $-C(CH_3)_3$ | 101 |
| 71a | N | $CH_3$ | $C_2H_5$ | H | S | 3-Cl | O | O | $-C(CH_3)_3$ | 52 |
| 71b | N | $CH_3$ | $C_2H_5$ | H | S | - | O | S | $-C(CH_3)_3$ | Öl |
| 71c | N | $CH_3$ | $C_2H_5$ | H | S | 3-Cl | O | S | $-C(CH_3)_3$ | Öl |
| 71d | N | $CH_3$ | $C_2H_5$ | H | S | - | S | NH | $-C(CH_3)_3$ | 98 |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 71e | N | $CH_3$ | $C_2H_5$ | H | S | 3-Cl | O | NH | $-C(CH_3)_3$ | 144 |
| 71f | N | $CH_3$ | $C_2H_5$ | H | S | 3-Cl | S | NH | $-C(CH_3)_3$ | 133-134 |
| 71g | N | $CH_3$ | $C_2H_5$ | H | S | 3-$CH_3$ | O | O | $-C(CH_3)_3$ | 36 |
| 72 | N | $CH_3$ | $CH(CH_3)_2$ | H | S | - | O | NH | $-C(CH_3)_3$ | |
| 73 | N | $C_2H_5$ | $CH_3$ | H | S | - | O | O | $-C(CH_3)_3$ | |
| 73a | N | $C_2H_5$ | $CH_3$ | H | S | 3-$CH_3$ | O | O | $-C(CH_3)_3$ | 45 |
| 73b | N | $C_2H_5$ | $CH_3$ | H | S | 3-$CH_3$ | O | O | $-C(CH_3)_2C_2H_5$ | 30 |
| 74 | N | $C_2H_5$ | $CH_3$ | H | S | - | O | NH | $-C(CH_3)_3$ | 99-100 |
| 74a | N | $C_2H_5$ | $CH_3$ | H | S | 3-$CH_3$ | O | NH | $-C(CH_3)_3$ | 161 |
| 75 | N | $C_3H_7$ | $CH_3$ | H | S | - | O | NH | $-C(CH_3)_3$ | |
| 76 | N | $CH(CH_3)_2$ | $CH_3$ | H | S | - | O | NH | $-C(CH_3)_3$ | |
| 77 | N | $-(CH_2)_3-$ | | H | S | - | O | NH | $-C(CH_3)_3$ | |
| 78 | N | $-(CH_2)_4-$ | | H | S | - | O | NH | $-C(CH_3)_3$ | |
| 79 | N | -CH=CH-CH=CH- | | H | S | - | O | NH | $-C(CH_3)_3$ | |

**Tabelle 1** (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 80 | N | $-CH=CH-C(Cl)=CH-$ | | H | S | - | O | NH | $-C(CH_3)_3$ | |
| 80a | N | $CH_3$ | H | $CH_3$ | O | - | O | O | $C(CH_3)_3$ | Öl |
| 80b | N | $CH_3$ | H | $CH_3$ | O | - | O | S | $C(CH_3)_3$ | Öl |
| 81 | N | $CH_3$ | H | $CH_3$ | O | - | S | S | $-C_2H_5$ | |
| 82 | N | $CH_3$ | H | $CH_3$ | O | - | S | S | $-C(CH_3)_3$ | |
| 83 | N | $CH_3$ | H | $CH_3$ | O | - | O | NH | $-CH_3$ | 166-167 |
| 84 | N | $CH_3$ | H | $CH_3$ | O | - | O | NH | $-C_2H_5$ | |
| 85 | N | $CH_3$ | H | $CH_3$ | O | - | O | NH | $-C_3H_7$ | 127-128 |
| 86 | N | $CH_3$ | H | $CH_3$ | O | - | O | NH | $-CH(CH_3)_2$ | 166-167 |
| 87 | N | $CH_3$ | H | $CH_3$ | O | - | O | NH | $-C_4H_9$ | 117-118 |
| 88 | N | $CH_3$ | H | $CH_3$ | O | - | O | NH | $-CH_2-CH(CH_3)_2$ | 139-140 |
| 89 | N | $CH_3$ | H | $CH_3$ | O | - | O | NH | $-CH(CH_3)(C_2H_5)$ | 139-140 |
| 90 | N | $CH_3$ | H | $CH_3$ | O | - | O | NH | $-C(CH_3)_3$ | 170 |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 91 | N | $CH_3$ | H | $CH_3$ | O | - | O | NH | $-CH_2-C(CH_3)_3$ | 147-148 |
| 92 | N | $CH_3$ | H | $CH_3$ | O | - | O | NH | 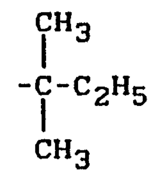 | 144-145 |
| 93 | N | $CH_3$ | H | $CH_3$ | O | - | O | NH | 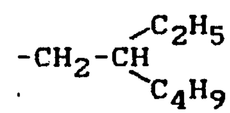 | 95- 96 |
| 94 | N | $CH_3$ | H | $CH_3$ | O | - | O | NH | $-CH_2-CH=CH_2$ | 104-105 |
| 95 | N | $CH_3$ | H | $CH_3$ | O | - | O | NH | 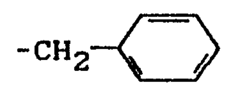 | 139-140 |
| 96 | N | $CH_3$ | H | $CH_3$ | O | - | O | NH | 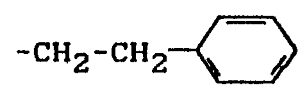 | 131-132 |
| 97 | N | $CH_3$ | H | $CH_3$ | O | - | O | NH | 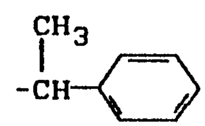 | 178-179 |

Tabelle 1 (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 98 | N | $CH_3$ | H | $CH_3$ | O | - | O | NH | $-CH_2-$ 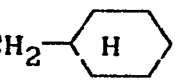 | 168-169 |
| 99 | N | $CH_3$ | H | $CH_3$ | O | - | O | NH | $-(CH_2)_3-OCH_3$ | 72- 73 |
| 100 | N | $CH_3$ | H | $CH_3$ | O | - | O | NH | 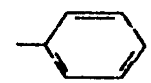 | 153-154 |
| 101 | N | $CH_3$ | H | $CH_3$ | O | - | O | NH | 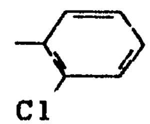 | 119-120 |
| 102 | N | $CH_3$ | H | $CH_3$ | O | - | O | NH | 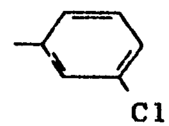 | 123-124 |
| 103 | N | $CH_3$ | H | $CH_3$ | O | - | O | NH | 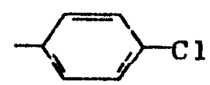 | 171-172 |
| 104 | N | $CH_3$ | H | $CH_3$ | O | - | O | NH | 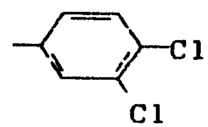 | 114-115 |

Tabelle 1 (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 105 | N | $CH_3$ | H | $CH_3$ | O | - | O | NH | 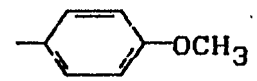 | 160 |
| 106 | N | $CH_3$ | H | $CH_3$ | O | - | O | NH | 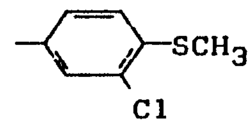 | 178-179 |
| 107 | N | $CH_3$ | H | $CH_3$ | O | - | O | NH | 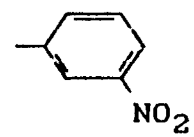 | 202-203 |
| 108 | N | $CH_3$ | H | $CH_3$ | O | - | O | NH | 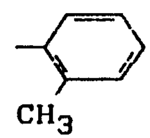 | 159-160 |
| 109 | N | $CH_3$ | H | $CH_3$ | O | - | O | NH | 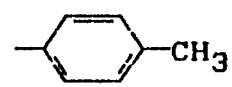 | 153-154 |
| 110 | N | $CH_3$ | H | $CH_3$ | O | - | O | NH | 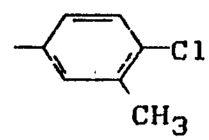 | 160 |

<u>Tabelle 1</u> (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 111 | N | $CH_3$ | H | $CH_3$ | O | - | O | NH | 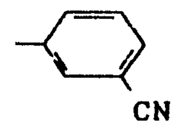 | 151-152 |
| 112 | N | $CH_3$ | H | $CH_3$ | O | - | O | NH | 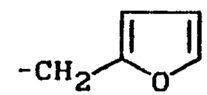 | 134-135 |
| 113 | N | $CH_3$ | H | $CH_3$ | O | - | O | NH | 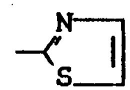 | 165-166 |
| 114 | N | $CH_3$ | H | $CH_3$ | O | - | O | $NCH_3$ | $CH_3$ | 93- 94 |
| 115 | N | $CH_3$ | H | $CH_3$ | O | - | O | $NCH_3$ | 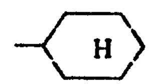 | 120-121 |
| 116 | N | $CH_3$ | H | $CH_3$ | O | - | O | $NCH_3$ | 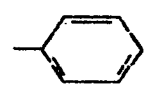 | 171-172 |
| 117 | N | $CH_3$ | H | $CH_3$ | O | - | O | $NC_3H_7$ | $C_3H_7$ | 93- 94 |

<u>Tabelle 1</u> (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 118 | N | $CH_3$ | H | $CH_3$ | O | - | O | $NCH(CH_3)_2$ | $CH(CH_3)_2$ | 91- 92 |
| 119 | N | $CH_3$ | H | $CH_3$ | O | - | O | $NC_4H_9$ | $C_4H_9$ | Öl |
| 120 | N | $CH_3$ | H | $CH_3$ | O | - | O | $NCH_2-CH=CH_2$ | $CH_2-CH=CH_2$ | 71- 72 |
| 121 | N | $CH_3$ | H | $CH_3$ | O | - | O | 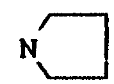 | | 156-157 |
| 122 | N | $CH_3$ | H | $CH_3$ | O | - | O | 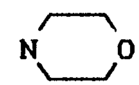 | | 110-111 |
| 123 | N | $CH_3$ | H | $CH_3$ | O | 3-Cl | O | O | $-C_2H_5$ | 117-118 |
| 124 | N | $CH_3$ | H | $CH_3$ | O | 3-Cl | O | NH | $-C(CH_3)_3$ | 199-200 |
| 125 | N | $CH_3$ | H | $CH_3$ | O | 3-Cl | O | NH | 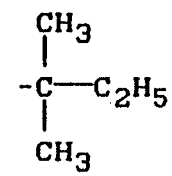 | 184 |
| 126 | N | $CH_3$ | H | $CH_3$ | O | 3-Cl | O | NH | 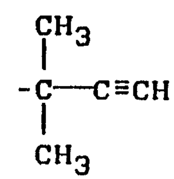 | 216-218 |

<u>Tabelle 1</u> (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 127 | N | $CH_3$ | H | $CH_3$ | O | 3-Cl | O | NH | 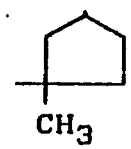 | 196-198 |
| 128 | N | $CH_3$ | H | $CH_3$ | S | - | O | O | $-C_2H_5$ | 93- 94 |
| 128a | N | $CH_3$ | H | $CH_3$ | S | - | O | O | 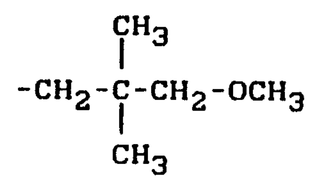 | 67 |
| 128b | N | $CH_3$ | H | $CH_3$ | S | - | O | O | $-CH_2-C(CH_3)_2-CH_2-O-C_2H_5$ | Öl |
| 128c | N | $CH_3$ | H | $CH_3$ | S | - | O | O | $-C_2H_4-O-C_2H_5$ | Öl |
| 129 | N | $CH_3$ | H | $CH_3$ | S | - | O | O | $-CH(CH_3)_2$ | 87- 88 |

Tabelle 1 (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 130 | N | $CH_3$ | H | $CH_3$ | S | - | O | O | 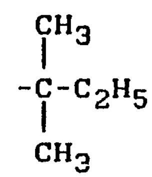 | Öl |
| 131 | N | $CH_3$ | H | $CH_3$ | S | - | O | O | 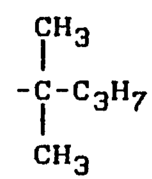 | Öl |
| 132 | N | $CH_3$ | H | $CH_3$ | S | - | O | O | 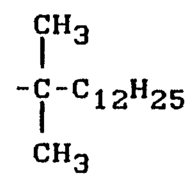 | Öl |

<u>Tabelle 1</u> (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 133 | N | $CH_3$ | H | $CH_3$ | S | - | O | O | 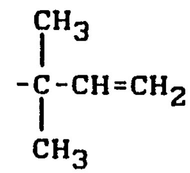 | 69- 70 |
| 134 | N | $CH_3$ | H | $CH_3$ | S | - | O | O | 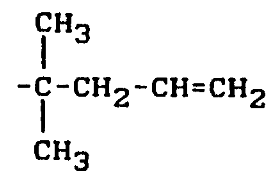 | Öl |
| 135 | N | $CH_3$ | H | $CH_3$ | S | - | O | O | 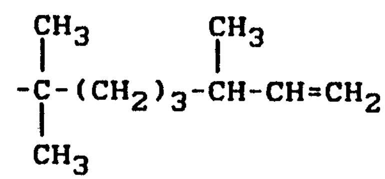 | Öl |
| 136 | N | $CH_3$ | H | $CH_3$ | S | - | O | O | 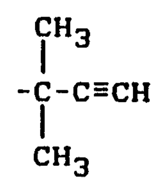 | 71- 72 |
| 137 | N | $CH_3$ | H | $CH_3$ | S | - | O | O | 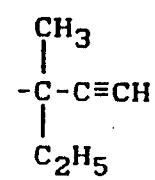 | 73- 74 |

<u>Tabelle 1</u> (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 138 | N | $CH_3$ | H | $CH_3$ | S | - | O | O | 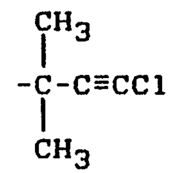 | 90- 91 |
| 139 | N | $CH_3$ | H | $CH_3$ | S | - | O | O | 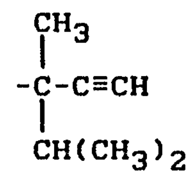 | 85- 86 |
| 140 | N | $CH_3$ | H | $CH_3$ | S | - | O | O | 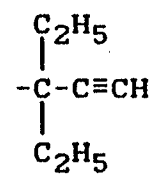 | 90- 91 |
| 141 | N | $CH_3$ | H | $CH_3$ | S | - | O | O | 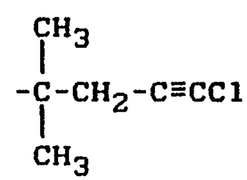 | Öl |
| 142 | N | $CH_3$ | H | $CH_3$ | S | - | O | O | 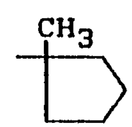 | 70- 72 |

<u>Tabelle 1</u> (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 143 | N | $CH_3$ | H | $CH_3$ | S | - | O | O | 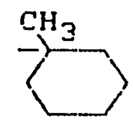 | 64- 65 |
| 144 | N | $CH_3$ | H | $CH_3$ | S | - | O | O | 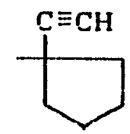 | 96- 98 |
| 145 | N | $CH_3$ | H | $CH_3$ | S | - | O | O | 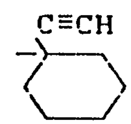 | 137-138 |
| 146 | N | $CH_3$ | H | $CH_3$ | S | - | O | O | 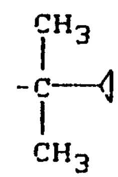 | 48- 50 |
| 147 | N | $CH_3$ | H | $CH_3$ | S | - | O | O | 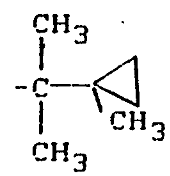 | 77- 78 |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 148 | N | $CH_3$ | H | $CH_3$ | S | - | O | O | 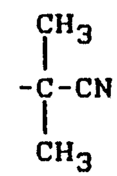 | 83- 84 |
| 149 | N | $CH_3$ | H | $CH_3$ | S | - | O | O | 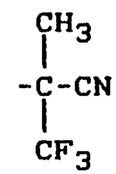 | 67- 68 |
| 150 | N | $CH_3$ | H | $CH_3$ | S | - | O | O | 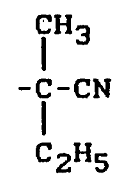 | 71- 72 |
| 150a | N | $CH_3$ | H | $CH_3$ | S | - | O | O | 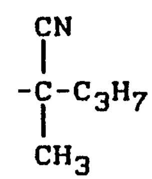 | Öl |
| 150b | N | $CH_3$ | H | $CH_3$ | S | - | O | O | 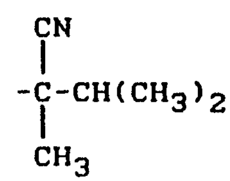 | 72-73 |

Tabelle 1 (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 151 | N | $CH_3$ | H | $CH_3$ | S | - | O | O | 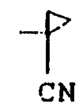 | 76- 77 |
| 151a | N | $CH_3$ | H | $CH_3$ | S | - | O | O | 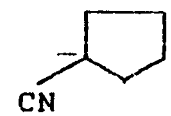 | 104-105 |
| 152 | N | $CH_3$ | H | $CH_3$ | S | - | O | O | 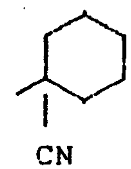 | 115-116 |
| 152a | N | $CH_3$ | H | $CH_3$ | S | - | O | O | 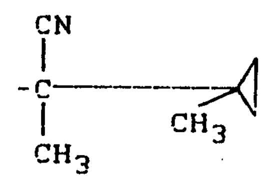 | 72 |
| 153 | N | $CH_3$ | H | $CH_3$ | S | - | O | O | 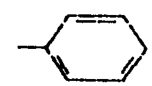 | 121-122 |
| 154 | N | $CH_3$ | H | $CH_3$ | S | - | O | O | 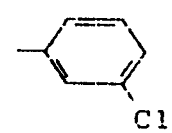 | 88- 89 |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 155 | N | $CH_3$ | H | $CH_3$ | S | - | O | O | 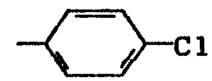 | 119-120 |
| 156 | N | $CH_3$ | H | $CH_3$ | S | - | O | O | 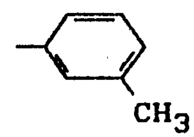 | 103-104 |
| 157 | N | $CH_3$ | H | $CH_3$ | S | - | O | O | 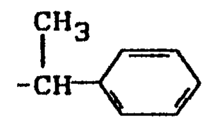 | Öl |
| 158 | N | $CH_3$ | H | $CH_3$ | S | - | O | O | 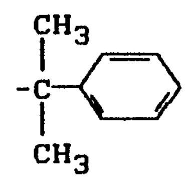 | 103-104 |
| 159 | N | $CH_3$ | H | $CH_3$ | S | - | O | O | 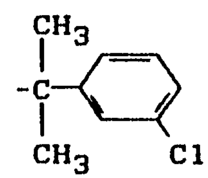 | 87- 88 |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 160 | N | $CH_3$ | H | $CH_3$ | S | - | O | O | 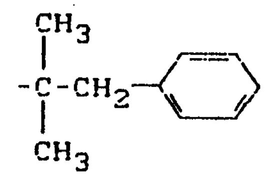 | Öl |
| 161 | N | $CH_3$ | H | $CH_3$ | S | - | O | O | 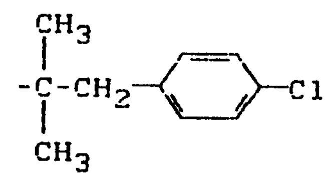 | Öl |
| 162 | N | $CH_3$ | H | $CH_3$ | S | - | O | O | 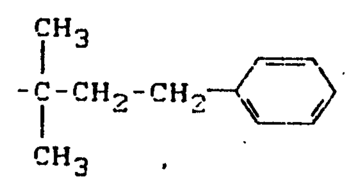 | Öl |
| 162a | N | $CH_3$ | H | $CH_3$ | S | - | O | S | $-CH(CH_3)_2$ | 70-71 |
| 163 | N | $CH_3$ | H | $CH_3$ | S | - | O | S | $-C(CH_3)_3$ | 69-70 |
| 163a | N | $CH_3$ | H | $CH_3$ | S | - | O | S | 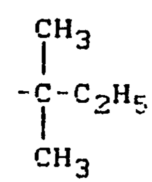 | 70-71 |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 164 | N | $CH_3$ | H | $CH_3$ | S | - | O | S | 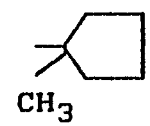 | |
| 165 | N | $CH_3$ | H | $CH_3$ | S | - | O | S | 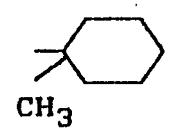 | |
| 165a | N | $CH_3$ | H | $CH_3$ | S | - | O | S | 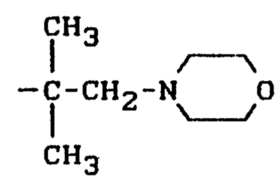 | Öl |
| 166 | N | $CH_3$ | H | $CH_3$ | S | - | O | NH | $-C(CH_3)_3$ | 153-154 |
| 167 | N | $CH_3$ | H | $CH_3$ | S | - | O | NH | 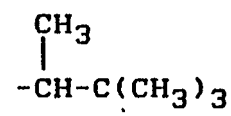 | 135-136 |
| 168 | N | $CH_3$ | H | $CH_3$ | S | - | O | NH | 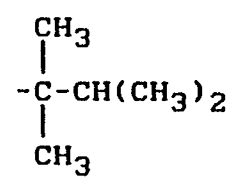 | 112-113 |

Tabelle 1 (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 169 | N | $CH_3$ | H | $CH_3$ | S | - | O | NH | 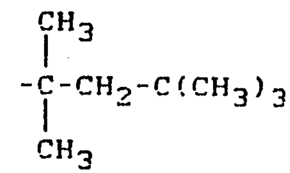 | 75-78 |
| 170 | N | $CH_3$ | H | $CH_3$ | S | - | O | NH | 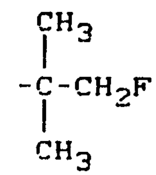 | 145-146 |
| 171 | N | $CH_3$ | H | $CH_3$ | S | - | O | NH | 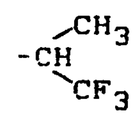 | 154-156 |
| 172 | N | $CH_3$ | H | $CH_3$ | S | - | O | NH | 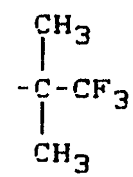 | 119-121 |
| 173 | N | $CH_3$ | H | $CH_3$ | S | - | O | NH | 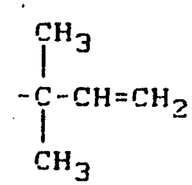 | |

Tabelle 1 (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 174 | N | $CH_3$ | H | $CH_3$ | S | - | O | NH | 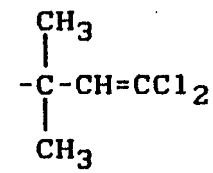 | 157-158 |
| 175 | N | $CH_3$ | H | $CH_3$ | S | - | O | NH | 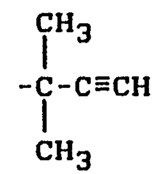 | 147-148 |
| 176 | N | $CH_3$ | H | $CH_3$ | S | - | O | NH | 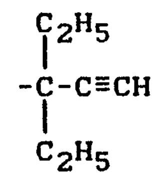 | 138-139 |
| 176a | N | $CH_3$ | H | $CH_3$ | S | - | O | NH | 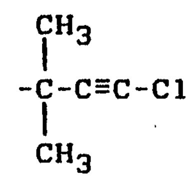 | Öl |
| 177 | N | $CH_3$ | H | $CH_3$ | S | - | O | NH | 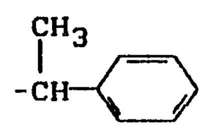 | 156-157 |

<u>Tabelle 1</u> (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 178 | N | $CH_3$ | H | $CH_3$ | S | - | O | NH | 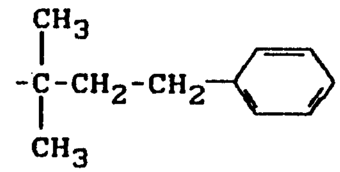 | 148-150 |
| 179 | N | $CH_3$ | H | $CH_3$ | S | - | O | NH | 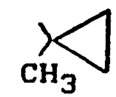 | 178-180 |
| 180 | N | $CH_3$ | H | $CH_3$ | S | - | O | NH | 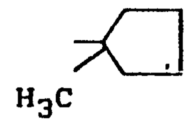 | 137-138 |
| 181 | N | $CH_3$ | H | $CH_3$ | S | - | O | NH | 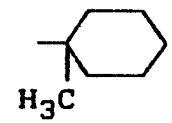 | 114-115 |
| 182 | N | $CH_3$ | H | $CH_3$ | S | - | O | NH | 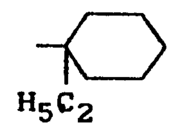 | 149-150 |
| 183 | N | $CH_3$ | H | $CH_3$ | S | - | O | NH | 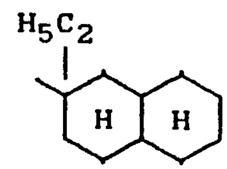 | 171-172 |

Tabelle 1 (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 184 | N | $CH_3$ | H | $CH_3$ | S | - | O | NH | 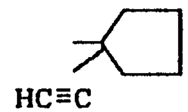 | |
| 184 | N | $CH_3$ | H | $CH_3$ | S | - | O | NH | 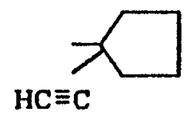 | |
| 185 | N | $CH_3$ | H | $CH_3$ | S | - | O | NH | 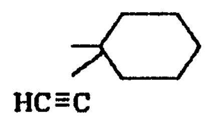 | 171-172 |
| 186 | N | $CH_3$ | H | $CH_3$ | S | - | O | NH | 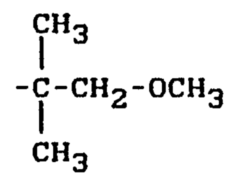 | 104-106 |
| 187 | N | $CH_3$ | H | $CH_3$ | S | - | O | NH | 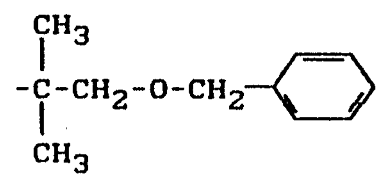 | 95- 96 |

Tabelle 1 (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 188 | N | $CH_3$ | H | $CH_3$ | S | - | O | NH | 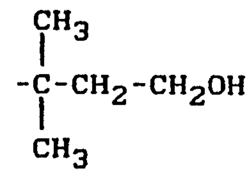 | 147-148 |
| 189 | N | $CH_3$ | H | $CH_3$ | S | - | O | NH | 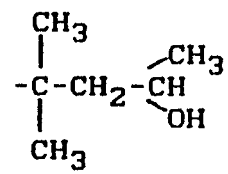 | 119-120 |
| 190 | N | $CH_3$ | H | $CH_3$ | S | - | O | NH | 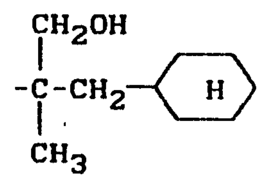 | 107-108 |
| 191 | N | $CH_3$ | H | $CH_3$ | S | - | O | NH | 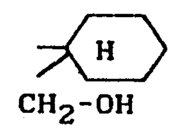 | 179-180 |
| 192 | N | $CH_3$ | H | $CH_3$ | S | - | O | NH | 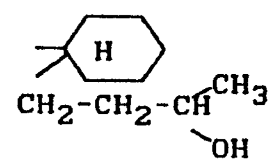 | 124-125 |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 193 | N | $CH_3$ | H | $CH_3$ | S | - | O | NH | 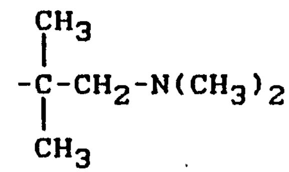 | 96- 97 |
| 194 | N | $CH_3$ | H | $CH_3$ | S | - | O | NH | 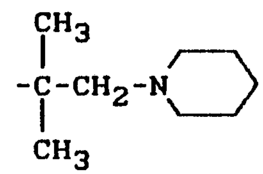 | 108-110 |
| 195 | N | $CH_3$ | H | $CH_3$ | S | - | O | NH | 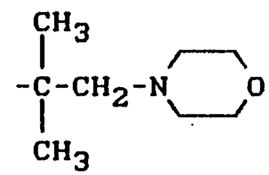 | 150-152 |
| 196 | N | $CH_3$ | H | $CH_3$ | S | - | O | NH | 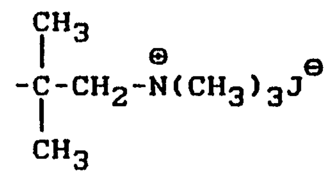 | 66 (Zers.) |
| 197 | N | $CH_3$ | H | $CH_3$ | S | - | O | NH | 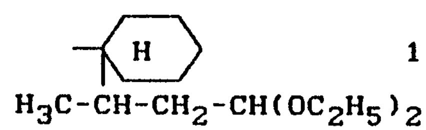 | 115-116 |

<u>Tabelle 1</u> (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 198 | N | $CH_3$ | H | $CH_3$ | S | - | O | NH | 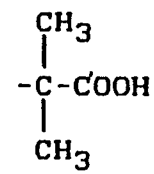 | 246-248 |
| 199 | N | $CH_3$ | H | $CH_3$ | S | - | O | NH | 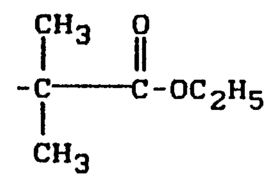 | 158-160 |
| 200 | N | $CH_3$ | H | $CH_3$ | S | - | O | NH | 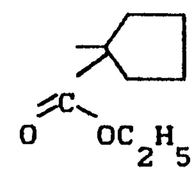 | 147-148 |
| 201 | N | $CH_3$ | H | $CH_3$ | S | - | O | NH | 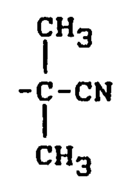 | 197 |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 202 | N | $CH_3$ | H | $CH_3$ | S | - | O | NH | 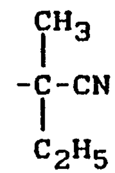 | 169-170 |
| 203 | N | $CH_3$ | H | $CH_3$ | S | - | O | NH | 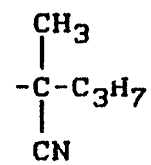 | |
| 204 | N | $CH_3$ | H | $CH_3$ | S | - | O | NH | 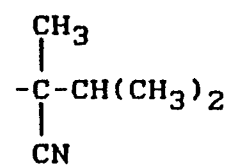 | |
| 205 | N | $CH_3$ | H | $CH_3$ | S | - | O | NH | 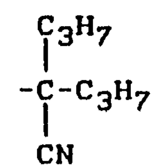 | 176-177 |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 206 | N | $CH_3$ | H | $CH_3$ | S | - | O | NH | 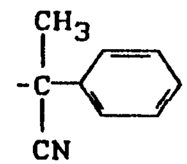 | > 260 |
| 207 | N | $CH_3$ | H | $CH_3$ | S | - | O | NH | 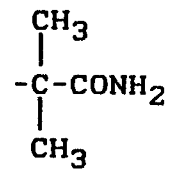 | |
| 208 | N | $CH_3$ | H | $CH_3$ | S | - | O | NH | 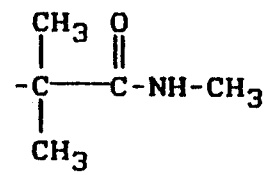 | 184-186 |
| 209 | N | $CH_3$ | H | $CH_3$ | S | - | O | NH | 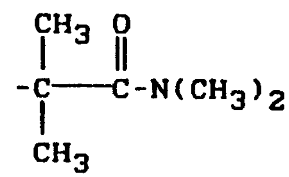 | 220-222 |
| 209a | N | $CH_3$ | H | $CH_3$ | S | - | O | NH | 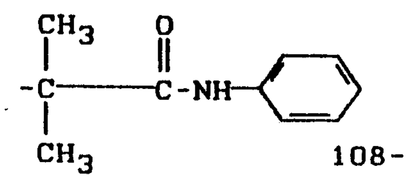 | 108-110 |

Tabelle 1 (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 210 | N | $CH_3$ | H | $CH_3$ | S | - | O | NH | 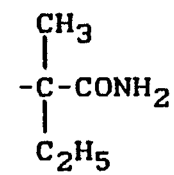 | 155-156 |
| 211 | N | $CH_3$ | H | $CH_3$ | S | - | O | NH | 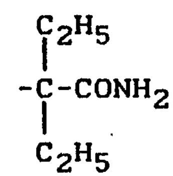 | 189-190 |
| 212 | N | $CH_3$ | H | $CH_3$ | S | - | O | NH | 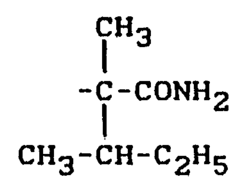 | 183 |
| 213 | N | $CH_3$ | H | $CH_3$ | S | - | O | NH | 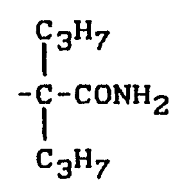 | 76- 77 |
| 214 | N | $CH_3$ | H | $CH_3$ | S | - | O | NH | 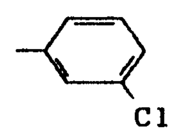 | 141-142 |

Tabelle 1 (Fortsetzung)

| Bei- spiel Nr. | Z | $R^1$ | $R^2$ | $R^3$ | X | Y | A | B | $R^4$ | Fp. (°C) |
|---|---|---|---|---|---|---|---|---|---|---|
| 215 | N | $CH_3$ | H | $CH_3$ | S | - | O | NH | 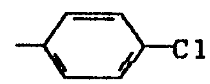 | 165-166 |
| 216 | N | $CH_3$ | H | $CH_3$ | S | - | O | NH | 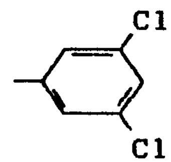 | 151-152 |
| 217 | N | $CH_3$ | H | $CH_3$ | S | - | O | NH | 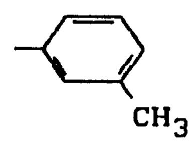 | 153-154 |
| 218 | N | $CH_3$ | H | $CH_3$ | S | - | O | $N-CH_3$ | 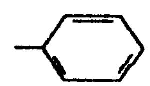 | 124-125 |
| 219 | N | $CH_3$ | H | $CH_3$ | S | - | O | $N-CH_3$ | 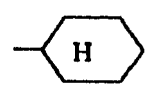 | 155-156 |

<u>Tabelle 1</u> (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 220 | N | $CH_3$ | H | $CH_3$ | S | - | O | 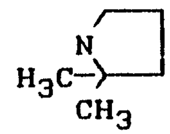 | | |
| 221 | N | $CH_3$ | H | $CH_3$ | S | - | O | 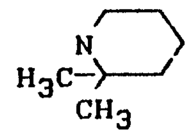 | | |
| 222 | N | $CH_3$ | H | $CH_3$ | S | - | O | NOH | H | 190-192 |
| 222a | N | $CH_3$ | H | $CH_3$ | S | - | O | NOH | $-C(CH_3)_3$ | 156-158 |
| 223 | N | $CH_3$ | H | $CH_3$ | S | - | O | $NOCH_3$ | H | 160-162 |
| 223a | N | $CH_3$ | H | $CH_3$ | S | - | S | O | $C_2H_5$ | 70- 71 |
| 224 | N | $CH_3$ | H | $CH_3$ | S | - | S | O | $-C-(CH_3)_3$ | |
| 225 | N | $CH_3$ | H | $CH_3$ | S | - | S | O | 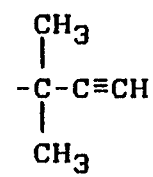 | |
| 226 | N | $CH_3$ | H | $CH_3$ | S | - | S | O | 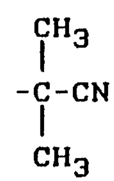 | |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 227 | N | $CH_3$ | H | $CH_3$ | S | - | S | O | 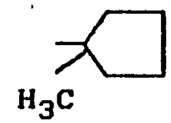 | |
| 228 | N | $CH_3$ | H | $CH_3$ | S | - | S | O | 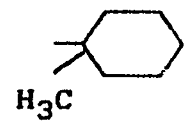 | |
| 229 | N | $CH_3$ | H | $CH_3$ | S | - | S | S | $-C(CH_3)_3$ | |
| 230 | N | $CH_3$ | H | $CH_3$ | S | - | S | S | 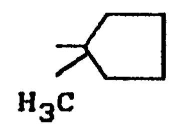 | |
| 231 | N | $CH_3$ | H | $CH_3$ | S | - | S | S | 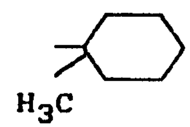 | |

Tabelle 1 (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 232 | N | $CH_3$ | H | $CH_3$ | S | - | S | NH | 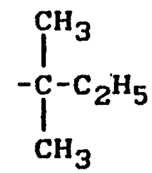 | 102-103 |
| 233 | N | $CH_3$ | H | $CH_3$ | S | - | S | NH | 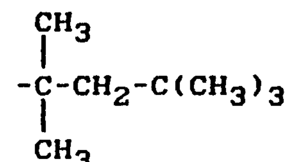 | 81- 82 |
| 234 | N | $CH_3$ | H | $CH_3$ | S | - | S | NH | 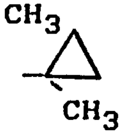 | 121-122 |
| 235 | N | $CH_3$ | H | $CH_3$ | S | - | S | NH | 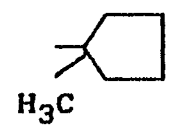 | 101-102 |
| 236 | N | $CH_3$ | H | $CH_3$ | S | - | S | NH | 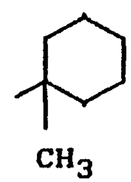 | 99-100 |

<u>Tabelle 1</u> (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 237 | N | $CH_3$ | H | $CH_3$ | S | - | S | NH | 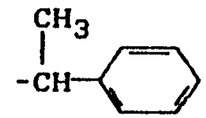 | 45- 46 |
| 238 | N | $CH_3$ | H | $CH_3$ | S | - | S | NH | 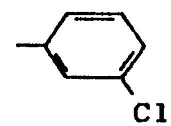 | 173-174 |
| 239 | N | $CH_3$ | H | $CH_3$ | S | - | S | NH | 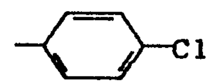 | 193-194 |
| 240 | N | $CH_3$ | H | $CH_3$ | S | - | $NCH_3$ | O | $-C(CH_3)_3$ | |
| 241 | N | $CH_3$ | H | $CH_3$ | S | - | $N-C(CH_3)_3$ | O | $CH_3$ | |
| 242 | N | $CH_3$ | H | $CH_3$ | S | - | 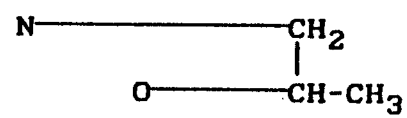 | | | Öl |
| 243 | N | $CH_3$ | H | $CH_3$ | S | - | 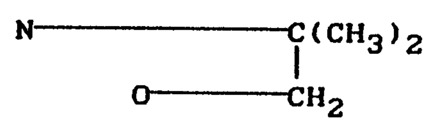 | | | 68- 69 |
| 244 | N | $CH_3$ | H | $CH_3$ | S | - | 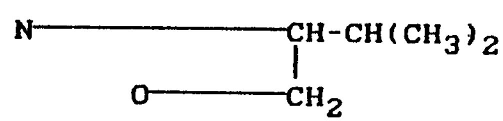 | | | Öl |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 245 | N | $CH_3$ | H | $CH_3$ | S | - | 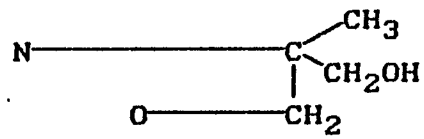 | | | 124-125 |
| 246 | N | $CH_3$ | H | $CH_3$ | S | - | 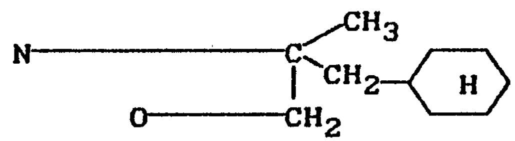 | | | Öl |
| 246a | N | $CH_3$ | H | $CH_3$ | S | - | 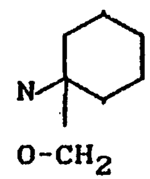 | | | 103 |
| 246b | N | $CH_3$ | H | $CH_3$ | S | - | 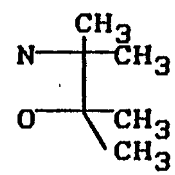 | | | 114 |
| 246c | N | $CH_3$ | H | $CH_3$ | S | - | 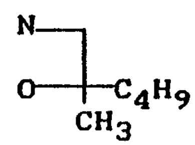 | | | Öl |

<u>Tabelle 1</u> (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 246d | N | $CH_3$ | H | $CH_3$ | S | - | 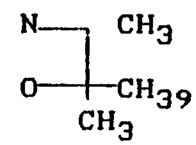 | | | 82 |
| 246e | N | $CH_3$ | H | $CH_3$ | S | - | 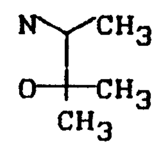 | | | 74 |
| 247 | N | $CH_3$ | H | $CH_3$ | S | - | 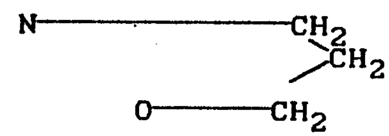 | | | 122-123 |
| 248 | N | $CH_3$ | H | $CH_3$ | S | - | 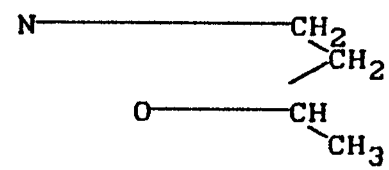 | | | 94- 95 |
| 249 | N | $CH_3$ | H | $CH_3$ | S | - | 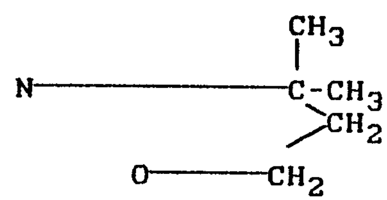 | | | 96- 97 |
| 249a | N | $CH_3$ | H | $CH_3$ | S | - | 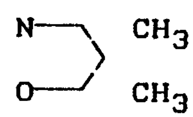 | | | 101 |

<u>Tabelle 1</u> (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 250 | N | $CH_3$ | H | $CH_3$ | S | - | 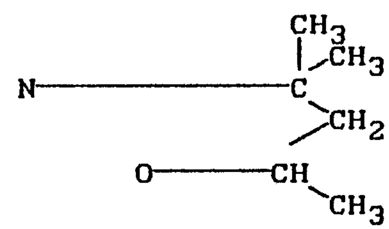 | | | 98- 99 |
| 250a | N | $CH_3$ | H | $CH_3$ | S | - | 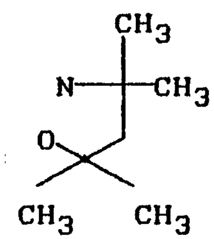 | | | Öl |
| 250b | N | $CH_3$ | H | $CH_3$ | S | 2-$CH_3$ | 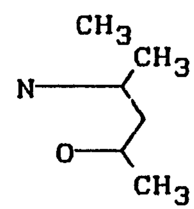 | | | Öl |
| 251 | N | $CH_3$ | H | $CH_3$ | S | - | N | $SCH_3$ | $C(CH_3)_3$ | 88-90 |

Tabelle 1 (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 251a | N | $CH_3$ | H | $CH_3$ | S | | N | $SC_2H_5$ | $C(CH_3)_3$ | 65-67 |
| 251b | N | $CH_3$ | H | $CH_3$ | S | - | N | $SCH_2-CH=CH_2$ | $C(CH_3)_3$ | 69-70 |
| 251c | N | $CH_3$ | H | $CH_3$ | S | - | N | $S-C_3H_7-i$ | $C(CH_3)_3$ | 78 |
| 251d | N | $CH_3$ | H | $CH_3$ | S | - | N | $S-CH_2-CH=CH_2$ | $C(CH_3)_2-C_2H_5$ | Öl |
| 251e | N | $CH_3$ | H | $CH_3$ | S | 3-$CH_3$ | N | $S-CH_2-CH=CH_2$ | $C(CH_3)_2-CH(CH_3)_2$ | 89-90 |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 252 | N | $CH_3$ | H | $CH_3$ | S | - | 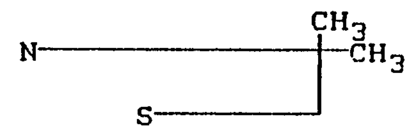 | | | 115-117 |
| 253 | N | $CH_3$ | H | $CH_3$ | S | - | 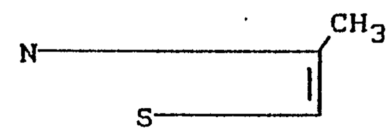 | | | 63- 64 |
| 254 | N | $CH_3$ | H | $CH_3$ | S | - | 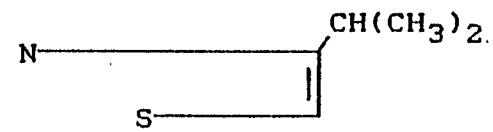 | | | |
| 255 | N | $CH_3$ | H | $CH_3$ | S | - | 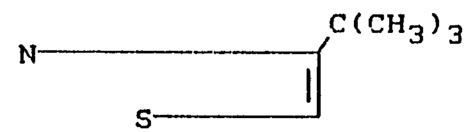 | | | 118-119 |
| 256 | N | $CH_3$ | H | $CH_3$ | S | - | 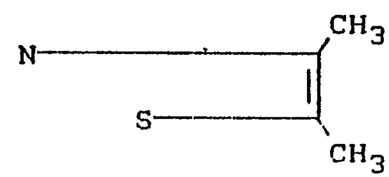 | | | |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 257 | N | $CH_3$ | H | $CH_3$ | S | - | NH | NH | $C(CH_3)_3$ | |
| 258 | N | $CH_3$ | H | $CH_3$ | S | - | $N-CH_3$ | NH | $C(CH_3)_3$ | |
| 259 | N | $CH_3$ | H | $CH_3$ | S | - | $N-C(CH_3)_3$ | $NCH_3$ | $CH_3$ | |
| 260 | N | $CH_3$ | H | $CH_3$ | S | - | 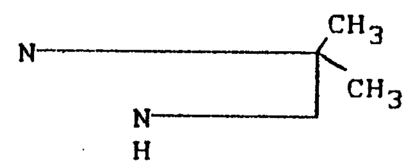 | | | |
| 261 | N | $CH_3$ | H | $CH_3$ | S | - | 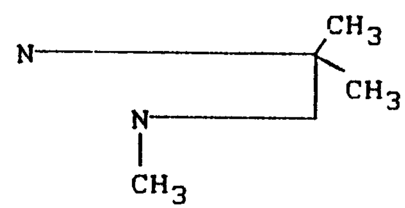 | | | |
| 261a | N | $CH_3$ | H | $CH_3$ | S | - | 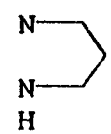 | x HCl | | > 230° C |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 262 | N | $CH_3$ | H | $CH_3$ | S | - | 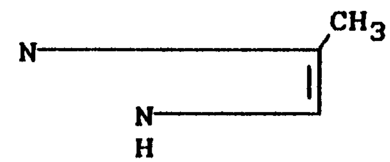 | | | |
| 263 | N | $CH_3$ | H | $CH_3$ | S | - | 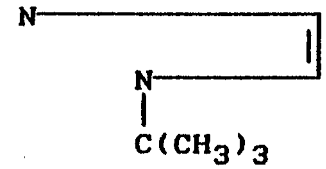 | | | |
| 264 | N | $CH_3$ | H | $CH_3$ | S | - | N-OH | O | $C(CH_3)_3$ | |
| 265 | N | $CH_3$ | H | $CH_3$ | S | - | N-OH | S | $C(CH_3)_3$ | |
| 266 | N | $CH_3$ | H | $CH_3$ | S | - | N-OH | NH | H | 174 |
| 267 | N | $CH_3$ | H | $CH_3$ | S | - | N-OH | NH | $C(CH_3)_3$ | |
| 268 | N | $CH_3$ | H | $CH_3$ | S | - | $NOCH_3$ | O | $C(CH_3)_3$ | |
| 269 | N | $CH_3$ | H | $CH_3$ | S | - | $NOCH_3$ | S | $C(CH_3)_3$ | |
| 270 | N | $CH_3$ | H | $CH_3$ | S | - | $NOCH_3$ | NH | $C(CH_3)_3$ | |
| 271 | N | $CH_3$ | H | $CH_3$ | S | - | 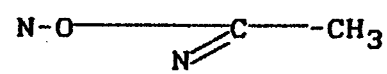 | | | 93- 95 |

Tabelle 1 (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 272 | N | $CH_3$ | H | $CH_3$ | S | - | 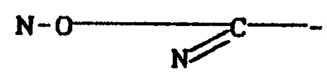 | | $-CH(CH_3)_2$ | |
| 273 | N | $CH_3$ | H | $CH_3$ | S | - | 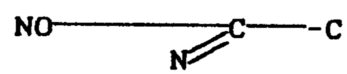 | | $-C(CH_3)_3$ | 100-102 |
| 274 | N | $CH_3$ | H | $CH_3$ | S | 2-F | O | NH | $-C(CH_3)_3$ | |
| 275 | N | $CH_3$ | H | $CH_3$ | S | 2-Cl | O | NH | $-C(CH_3)_3$ | 138-139 |
| 276 | N | $CH_3$ | H | $CH_3$ | S | 3-Cl | O | NH | $-C(CH_3)_3$ | 175-176 |
| 277 | N | $CH_3$ | H | $CH_3$ | S | 3-Cl | S | NH | $-C(CH_3)_3$ | 161-162 |
| 278 | N | $CH_3$ | H | $CH_3$ | S | 3-Br | O | NH | $-C(CH_3)_3$ | |
| 279 | N | $CH_3$ | H | $CH_3$ | S | 3,5-$Cl_2$ | O | NH | $-C(CH_3)_3$ | |
| 280 | N | $CH_3$ | H | $CH_3$ | S | 2-$OCH_3$ | O | NH | $-C(CH_3)_3$ | |
| 281 | N | $CH_3$ | H | $CH_3$ | S | 3-$OCH_3$ | O | NH | $-C(CH_3)_3$ | |
| 282 | N | $CH_3$ | H | $CH_3$ | S | 3-NH-C(=O)-$CH_3$ | O | NH | $-C(CH_3)_3$ | 181 |

<u>Tabelle 1</u> (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 283 | N | $CH_3$ | H | $CH_3$ | S | $3\text{-NH-}\overset{O}{\overset{\|}{C}}\text{-C(CH}_3)_3$ | O | NH | $-C(CH_3)_3$ | 181-182 |
| 284 | N | $CH_3$ | H | $CH_3$ | S | $3\text{-N(COOCH}_3)_2$ | O | NH | $-C(CH_3)_3$ | 221 |
| 285 | N | $CH_3$ | H | $CH_3$ | S | $3\text{-NH-}\overset{O}{\overset{\|}{C}}\text{-OC}_6H_5$ | O | NH | $-C(CH_3)_3$ | 180 |
| 286 | N | $CH_3$ | H | $CH_3$ | S | $2-CH_3$ | O | NH | $-C(CH_3)_3$ | |
| 287 | N | $CH_3$ | H | $CH_3$ | S | $3-CH_3$ | O | O | $-C(CH_3)_3$ | 75- 76 |
| 288 | N | $CH_3$ | H | $CH_3$ | S | $3-CH_3$ | O | NH | $-C(CH_3)_3$ | 177-178 |
| 289 | N | $CH_3$ | Cl | $CH_3$ | S | - | O | NH | $-C(CH_3)_3$ | 177-178 |
| 289a | N | $CH_3$ | Cl | $NH_2$ | S | - | O | NH | $-C(CH_3)_3$ | 98 |
| 290 | N | $CH_3$ | Br | $CH_3$ | S | - | O | NH | $-C(CH_3)_3$ | 164 |
| 291 | N | $CH_3$ | H | $CF_3$ | S | - | O | NH | $-C(CH_3)_3$ | |
| 292 | N | $CH_3$ | H | $C_2H_5$ | S | - | O | NH | $-C(CH_3)_3$ | |
| 293 | N | $CH_3$ | H | $C_3H_7$ | S | - | O | NH | $-C(CH_3)_3$ | |

<u>Tabelle 1</u> (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 294 | N | $CH_3$ | H | $C_4H_9$ | S | - | O | NH | $-C(CH_3)_3$ | |
| 295 | N | $C_2H_5$ | H | $C_2H_5$ | S | - | O | NH | $-C(CH_3)_3$ | |
| 296 | N | $CH_3$ | $CH_3$ | $CH_3$ | S | - | O | O | $-C_2H_5$ | 74- 76 |
| 297 | N | $CH_3$ | $CH_3$ | $CH_3$ | S | - | O | O | $-C(CH_3)_3$ | 66- 67 |
| 297a | N | $CH_3$ | $CH_3$ | $CH_3$ | S | - | O | O | 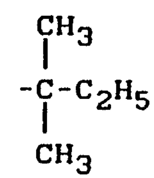 | 65 |
| 297b | N | $CH_3$ | $CH_3$ | $CH_3$ | S | - | O | O | $-CH_2-CH_2-O-C_2H_5$ | Öl |
| 297c | N | $CH_3$ | $CH_3$ | $CH_3$ | S | - | O | O | $-CH_2-C(CH_3)_2-O-CH_3$ | 83 |
| 297d | N | $CH_3$ | $CH_3$ | $CH_3$ | S | - | O | O | $-CH_2-C(CH_3)_2-O-C_2H_5$ | Öl |
| 297e | N | $CH_3$ | $CH_3$ | $CH_3$ | S | 3-$CH_3$ | O | O | $-C(CH_3)_3$ | 82 |

<u>Tabelle 1</u> (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 298 | N | $CH_3$ | $CH_3$ | $CH_3$ | S | - | O | NH | $-C(CH_3)_3$ | 186-188 |
| 299 | N | $CH_3$ | $CH_3$ | $CH_3$ | S | - | O | NH | 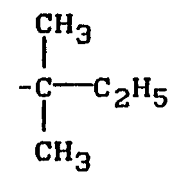 | 184-186 |
| 300 | N | $CH_3$ | $CH_3$ | $CH_3$ | S | - | O | NH | 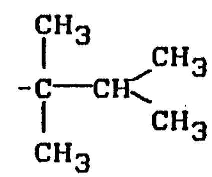 | 144-145 |
| 301 | N | $CH_3$ | $CH_3$ | $CH_3$ | S | - | O | NH | 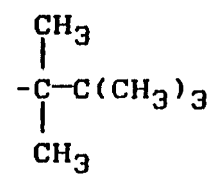 | 148-149 |

Tabelle 1 (Fortsetzung)

| Bei-spiel | Z | $R^1$ | $R^2$ | $R^3$ | X | Y | A | B | $R^4$ | Fp. (°C) |
|---|---|---|---|---|---|---|---|---|---|---|
| 302 | N | $CH_3$ | $CH_3$ | $CH_3$ | S | - | O | NH | 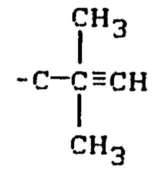 | 172-174 |
| 303 | N | $CH_3$ | $CH_3$ | $CH_3$ | S | - | O | NH | 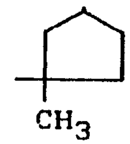 | 147-148 |
| 304 | N | $CH_3$ | $CH_3$ | $CH_3$ | S | - | O | NH | 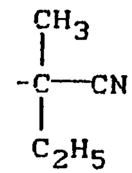 | 198-200 |
| 305 | N | $CH_3$ | $CH_3$ | $CH_3$ | S | - | S | NH | $-C(CH_3)_3$ | 179-180 |
| 306 | N | $CH_3$ | $C_2H_5$ | $CH_3$ | S | - | O | NH | $-C(CH_3)_3$ | 103-104 |
| 307 | N | $CH_3$ | $-(CH_2)_3-$ | | S | - | O | NH | $-C(CH_3)_3$ | 197-198 |
| 308 | N | $CH_3$ | $-(CH_2)_4-$ | | S | - | O | NH | $-C(CH_3)_3$ | |
| 309 | N | $CH_3$ | -CH=CH-CH=CH- | | S | - | O | NH | $-C(CH_3)_3$ | |
| 310 | N | $CH_3$ | H | $CH_3$ | S | - | O | NH | 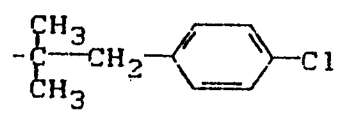 | 189-190 |
| 310a | N | $CH_3$ | H | $CH_3$ | S | 3-Cl | O | S | $-C(CH_3)_3$ | 43 |
| 311 | N | $CH_3$ | H | $CH_3$ | S | 3-Cl | O | NH | 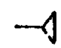 | 193-194 |

Tabelle 1 (Fortsetzung)

| Bei-spiel Nr. | Z | $R^1$ | $R^2$ | $R^3$ | X | Y | A | B | $R^4$ | Fp. (° C) |
|---|---|---|---|---|---|---|---|---|---|---|
| 312 | N | $CH_3$ | H | $CH_3$ | S | 3-Cl | O | NH | 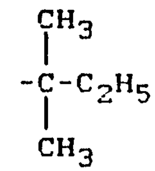 | 120 |
| 313 | N | $CH_3$ | H | $CH_3$ | S | 3-Cl | O | NH | 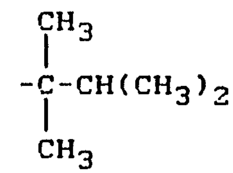 | 91 |
| 314 | N | $CH_3$ | H | $CH_3$ | S | 3-Cl | O | NH | 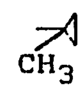 | 201 |
| 315 | N | $CH_3$ | H | $CH_3$ | S | 3-Cl | O | NH | 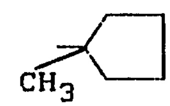 | 162 |
| 316 | N | $CH_3$ | H | $CH_3$ | S | 3-Cl | S | NH | 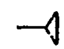 | 113 |
| 317 | N | $CH_3$ | H | $CH_3$ | S | 3-Cl | S | NH | 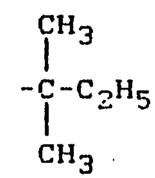 | 104 |

Tabelle 1 (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 318 | N | $CH_3$ | H | $CH_3$ | S | 3-Cl | S | NH | 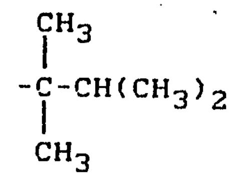 | 108 |
| 319 | N | $CH_3$ | H | $CH_3$ | S | 3-Cl | S | NH | 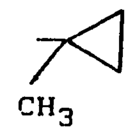 | 113 |
| 320 | N | $CH_3$ | H | $CH_3$ | S | 3-Cl | S | NH | 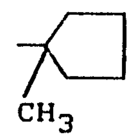 | 104 |
| 321 | N | $CH_3$ | H | $CH_3$ | S | 3-F | O | NH | $-C(CH_3)_3$ | 175 |
| 322 | N | $CH_3$ | H | $CH_3$ | S | 3-F | S | NH | $-C(CH_3)_3$ | 133 |
| 323 | N | $CH_3$ | H | $CH_3$ | S | 2-Cl | O | O | $-C(CH_3)_3$ | 65-66 |
| 324 | N | $CH_3$ | H | $CH_3$ | S | 2-Cl | O | O | 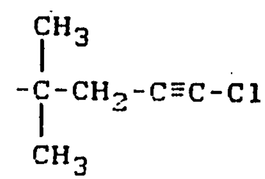 | Öl |

<u>Tabelle 1</u> (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 325 | N | $CH_3$ | H | $CH_3$ | S | 2-Cl | O | O | 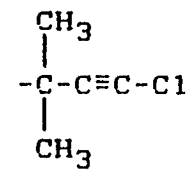 | Öl |
| 326 | N | $CH_3$ | H | $CH_3$ | S | 2-Cl | O | NH | 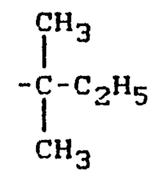 | 106-107 |
| 327 | N | $CH_3$ | H | $CH_3$ | S | 2-Cl | O | NH | 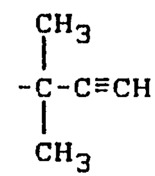 | 142-143 |
| 328 | N | $CH_3$ | H | $CH_3$ | S | 2-Cl | O | NH | 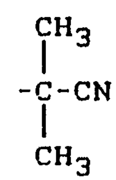 | 144-145 |
| 329 | N | $CH_3$ | H | $CH_3$ | S | 2-Cl | S | NH | $-C(CH_3)_3$ | 123 |
| 330 | N | $CH_3$ | H | $CH_3$ | S | 3-Cl | O | O | $-C(CH_3)_3$ | 99 |

Tabelle 1 (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 331 | N | $CH_3$ | H | $CH_3$ | S | 3-Cl | O | O | 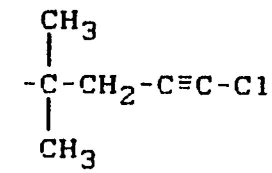 | 53 |
| 332 | N | $CH_3$ | H | $CH_3$ | S | 3-Cl | O | O | 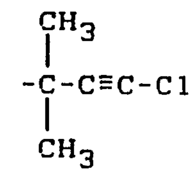 | 77 |
| 333 | N | $CH_3$ | H | $CH_3$ | S | 3-$CH_3$ | O | O | 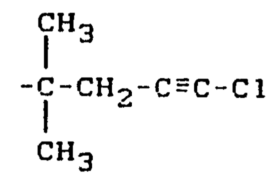 | Öl |
| 334 | N | $CH_3$ | H | $CH_3$ | S | 3-$CH_3$ | O | O | 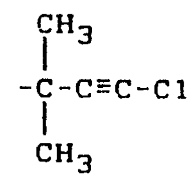 | 70-71 |
| 335 | N | $CH_3$ | H | $CH_3$ | S | 3-$CH_3$ | O | O | 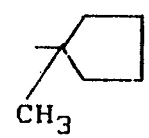 | 59-60 |
| 336 | N | $CH_3$ | H | $CH_3$ | S | 3-$CH_3$ | O | S | $-C(CH_3)_3$ | 64-65 |

Tabelle 1 (Fortsetzung)

| Beispiel Nr. | Z | $R^1$ | $R^2$ | $R^3$ | X | Y | A | B | $R^4$ | Fp. (°C) |
|---|---|---|---|---|---|---|---|---|---|---|
| 337 | N | $CH_3$ | H | $CH_3$ | S | 3-$CH_3$ | O | NH | 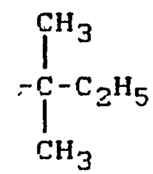 | 94-95 |
| 338 | N | $CH_3$ | H | $CH_3$ | S | 3-$CH_3$ | O | NH | 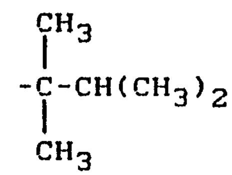 | 106 |
| 339 | N | $CH_3$ | H | $CH_3$ | S | 3-$CH_3$ | O | NH | 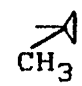 | 188 |
| 340 | N | $CH_3$ | H | $CH_3$ | S | 3-$CH_3$ | O | NH | 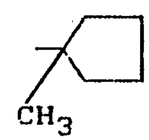 | 133-134 |
| 341 | N | $CH_3$ | H | $CH_3$ | S | 3-$CH_3$ | O | NH | 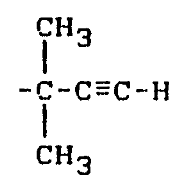 | 123-124 |

<u>Tabelle 1</u> (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 342 | N | $CH_3$ | H | $CH_3$ | S | 3-$CH_3$ | O | NH | 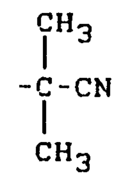 | 207-208 |
| 343 | N | $CH_3$ | H | $CH_3$ | S | 3-$CH_3$ | S | NH | $-C(CH_3)_3$ | 169-170 |
| 344 | N | $CH_3$ | H | $CH_3$ | S | 3-$CH_3$ | S | NH | 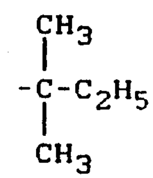 | 126-127 |
| 345 | N | $CH_3$ | H | $CH_3$ | S | 3-$CH_3$ | S | NH | 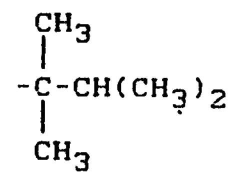 | 112-113 |
| 346 | N | $CH_3$ | H | $CH_3$ | S | 3-$CH_3$ | S | NH | 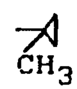 | 109 |
| 347 | N | $CH_3$ | $CH_3$ | $CH_3$ | S | 2-Cl | O | O | $-C(CH_3)_3$ | 95 |
| 348 | N | $CH_3$ | $CH_3$ | $CH_3$ | S | 2-Cl | O | NH | $-C(CH_3)_3$ | 145 |

Tabelle 1 (Fortsetzung)

| Bei-spiel Nr. | Z | $R^1$ | $R^2$ | $R^3$ | X | Y | A | B | $R^4$ | Fp. (°C) |
|---|---|---|---|---|---|---|---|---|---|---|
| 349 | N | $CH_3$ | $CH_3$ | $CH_3$ | S | 2-Cl | S | NH | $-C(CH_3)_3$ | 124-125 |
| 350 | N | $CH_3$ | $CH_3$ | $CH_3$ | S | 3-Cl | O | O | $-C(CH_3)_3$ | 60 |
| 351 | N | $CH_3$ | $CH_3$ | $CH_3$ | S | 3-Cl | O | S | $-C(CH_3)_3$ | 58 |
| 352 | N | $CH_3$ | $CH_3$ | $CH_3$ | S | 3-Cl | O | NH | $-C(CH_3)_3$ | 196 |
| 353 | N | $CH_3$ | $CH_3$ | $CH_3$ | S | 3-Cl | O | NH | $CH_3$<br>$-C-C_2H_5$<br>$CH_3$ | 156 |
| 354 | N | $CH_3$ | $C_2H_5$ | $CH_3$ | S | - | O | O | $-C(CH_3)_3$ | 50 |

Herstellung der Ausgangsverbindungen

Beispiel XXV-1

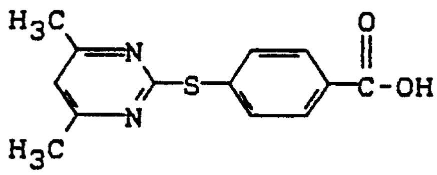

144 g (0,5 Mol) 4-(4,6-Dimethyl-pyrimidyl-2-mercapto)-benzoesäureethylester werden in 380 ml Ethanol suspendiert. Hierzu tropft man bei Raumtemperatur die Lösung von 40 g (1 Mol) Natriumhydroxid in wenig Wasser und läßt die Mischung 2 Stunden bei Raumtemperatur stehen. Dann gibt man soviel Wasser zu, daß eine klare Lösung entsteht und erwärmt noch 30 Minuten auf 50°C. Dann wird mit 1 l Wasser verdünnt und mit Salzsäure angesäuert. Die abgeschiedenen Kristalle werden abgesaugt und getrocknet.

Man erhält 123 g (94,6 % der Theorie) 4-(4,6-Dimethylpyrimidyl-2-mercapto)-benzoesäure vom Schmelzpunkt 179-180°C (Umkristallisation aus Toluol).

In analoger Weise erhält man:

Beispiel XXV-2

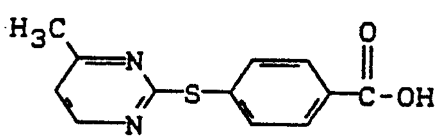

Schmelzpunkt

185-186° C
(Umkristallisation
aus Essigester)

Schmelzpunkt

Beispiel XXV-3

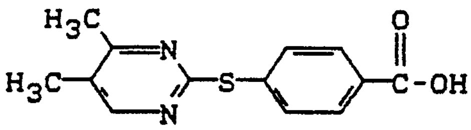

197-198° C
(Umkristallisation aus Ethanol)

Beispiel XXV-4

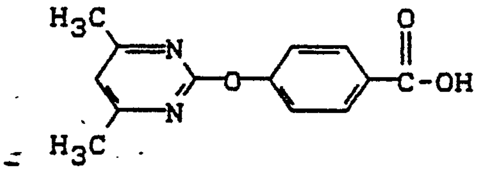

170-171° C
(Umkristallisation aus Essigester

Beispiel XIII-1

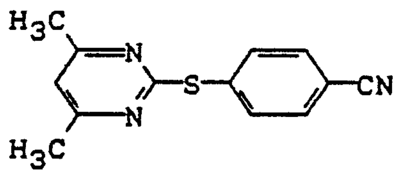

67,5 g (0,5 Mol) 4-Mercapto-benzonitril werden in 250 ml N-Methylpyrrolidon gelöst. Hierzu gibt man bei Raumtemperatur unter Rühren portionsweise 28 g (0,5 Mol) pulverisiertes Kaliumhydroxid und nach 30 Minuten 71,2 g (0,5 Mol) 2-Chlor-4,6-dimethyl-pyrimidin. Die Mischung wird allmählich auf 120°C erhitzt und noch 4 Stunden bei dieser Temperatur gerührt. Nach Abkühlen auf Raumtemperatur verdünnt man mit 1,5 l Wasser. Die abgeschiedenen Kristalle werden abgesaugt und getrocknet.

Man erhält 110 g (91,2 % der Theorie) 4-(4,6-Dimethylpyrimidyl-2-mercapto)-benzonitril vom Schmelzpunkt 90°C (Umkristalisation aus Waschbenzin).

Beispiel XVI-1

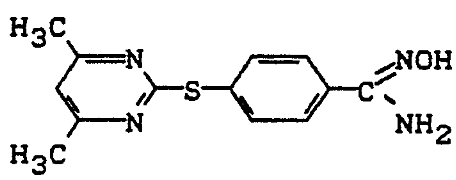

7,65 g (0,11 Mol) Hydroxylammoniumchlorid werden in sehr wenig Wasser gelöst. Unter Eiskühlung gibt man eine Lösung von 6,16 g (0,11 Mol) Kaliumhydroxid in Ethanol zu. Nach 1-stündigem Stehen saugt man das abgeschiedene Kaliumchlorid ab und trägt bei Raumtemperatur portionsweise 24,1 g (0,1 Mol) 4-(4,6-Dimethyl-pyrimidyl-2-mercapto)-benzonitril ein. Diese Mischung wird 15 Stunden bei 60°C bis 80°C gerührt und anschließend im Vakuum eingedampft. Der Rückstand wird in verdünnter Salzsäure gelöst, die Lösung filtriert und mit Ammoniak neutralisiert. Die abgeschiedenen Kristalle werden abgesaugt, mit Wasser gewaschen und an der Luft getrocknet.

Man erhält 21,4 g (78 % der Theorie) 4-(4,6-Dimethylpyrimidyl-2-mercapto)-benzamidoxim vom Schmelzpunkt 174°C (Zers.) (nach Umkristallisation aus wenig Butanol).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 -15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine sehr gute Wirksamkeit bei der selektiven Bekämpfung mono-und dikotyler Unkräuter in monokotylen Kulturen wie beispielsweise Gerste, Weizen und Mais zeigen in diesem Test z.B. die Verbindungen gemäß den Herstellungsbeispielen:
3, 4, 5, 8, 71, 102, 166, 175, 176, 202 und 219.

Beispiel B

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
\+ leichtes Austrocknen der Blätter, geringer Blattfall
\+ + starkes Austrocknen der Blätter, starker Blattfall
\+ + + sehr starkes Austrocknen der Blätter, sehr starker Blattfall

Eine deutliche Wirkung zeigen in diesem Test beispielsweise die Verbindungen gemäß den Herstellungsbeispielen:
4, 167 und 202.

## Ansprüche

1. Pyri(mi)dyl-oxy-bzw. -thio-benzoesäure-Derivate der Formel (I),

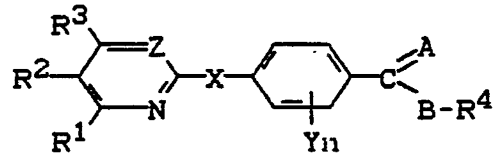

(I)

in welcher

$R^1$, $R^2$ und $R^3$ unabhängig voneinander jeweils für Wasserstoff, Halogen, Alkyl, für gegebenenfalls substituiertes Alkoxy, Halogenalkyl, Alkenyl oder für gegebenenfalls substituiertes Amino steht oder

$R^1$ und $R^2$ oder $R^2$ und $R^3$ gemeinsam für einen ankondensierten 3-bis 6-gliedrigen carbocyclischen Ring stehen,

mit der Maßgabe, daß mindestens einer der Reste $R^1$, $R^2$ oder $R^3$ für Alkyl oder für einen Teil des 3-bis 6-gliedrigen ankondensierten carbocyclischen Ringes steht,

Z für eine (-CH=)-Gruppe oder ein Stickstoffatom steht,

X für Sauerstoff oder Schwefel steht,

Y für Halogen, Nitro, Cyano, Amino, Alkylcarbonylamino, Alkoxycarbonylamino, Phenoxycarbonylalkylamino, Alkyl, Alkoxy oder Halogenalkyl steht, wobei Y gleich oder verschieden sein kann,

n für eine ganze Zahl von 0 bis 4 steht,

A für Sauerstoff, Schwefel, einen Rest $R^5$-N= oder einen Rest $R^6$O-N= steht,

wobei

$R^5$ für Wasserstoff, Alkyl, Hydroxyalkyl, Alkenyl, für jeweils gegebenenfalls substituiertes Cycloalkyl, Aryl oder Aralkyl steht,

$R^6$ für Wasserstoff, jeweils gegebenenfalls substituiertes Alkyl oder einfach oder mehrfach ungesättigtes Alkenyl steht,

B für Sauerstoff, Schwefel, für einen Rest -N(-)-$R^7$ oder -N(-)-O$R^8$ steht,

wobei

$R^7$ für Wasserstoff, für gegebenenfalls durch Halogen, Cyano oder durch einen Rest -D-$R^9$ substituiertes Alkyl oder für Alkenyl steht, wobei

D für Sauerstoff, Schwefel, Sulfinyl oder Sulfonyl steht und

$R^9$ für Wasserstoff, Alkyl, einfach oder mehrfach ungesättigtes Alkenyl oder für jeweils gegebenenfalls substituiertes Aryl oder Aralkyl oder für -CO-Alkyl steht,

$R^8$ für Wasserstoff, jeweils gegebenenfalls substituiertes Alkyl oder einfach oder mehrfach ungesättigtes Alkenyl steht und

$R^4$ für gegebenenfalls substituiertes gesättigtes oder ungesättigtes Alkyl steht, wobei als Substituenten ausgewählt sind:

Halogen, Nitro, Cyano, ein Rest -D-$R^9$; Alkoximo, die Gruppen

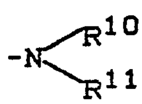 , 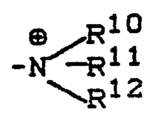 ,

-CO-O$R^{13}$, -CO-N$R^{14}R^{15}$, -CS-N$R^{14}R^{15}$,

-$SO_2$-N$R^{14}R^{15}$;

Cycloalkyl, Aryl oder ein 5-bzw. 6-gliedriger Heterocyclus,

$R^4$ weiterhin für jeweils gegebenenfalls substituiertes Cycloalkyl, Aryl oder einen 5-bis 7-gliedrigen Heterocyclus steht,

wobei

D und $R^9$ die oben angegebene Bedeutung haben,

$R^{10}$ für Wasserstoff oder Alkyl steht,

$R^{11}$ für Alkyl, Acyl, Alkylsulfonyl, Arylsulfonyl oder jeweils gegebenenfalls substituiertes Aryl oder Aralkyl steht oder

$R^{10}$ und $R^{11}$ gemeinsam mit dem Stickstoff, an den sie gebunden sind, auch für einen 5-bis 7-gliedrigen Heterocyclus stehen,

$R^{12}$ für Alkyl steht,

$R^{13}$ für Alkyl steht,

$R^{14}$ und $R^{15}$ unabhängig voneinander jeweils für Wasserstoff, Alkyl, Cycloalkyl oder für jeweils gegebenenfalls substituiertes Aryl oder Aralkyl stehen oder

$R^{14}$ und $R^{15}$ gemeinsam mit dem Stickstoff, an den sie gebunden sind, für einen 5-bis 7-gliedriegen Heterocyclus stehen oder

$R^4$ gemeinsam mit $R^5$ und B oder mit $R^6$ und B oder mit $R^7$ und B oder mit $R^8$ und B oder mit A und B einen 5-oder 6-gliedrigen Ring bilden kann.

2. Pyri(mi)dyl-oxy-und -thiobenzoesäure-Derivate der Formel (I) gemäß Anspruch 1, in welcher

$R^1$, $R^2$ und $R^3$ unabhängig voneinander jeweils für Wasserstoff, Fluor, Chlor, Brom, für Alkyl mit 1 bis 6 Kohlenstoffatomen, für Alkenyl mit 2 bis 6 Kohlenstoffatomen, für gegebenenfalls durch $C_2$-$C_4$-Alkenyl oder $C_1$-$C_2$-Alkoxy substituiertes Alkoxy, Alkylamino, Dialkylamino mit jeweils 1 bis 4 Kohlenstoffatomen in den einzelnen Alkylteilen, wobei im Fall des Dialkylamino die Alkylsubstituenten mit dem Stickstoffatom, an das sie gebunden sind, einen 5-bis 7-gliedrigen heterocyclischen Ring bilden können, für Amino, Alkenylamino oder Dialkenylamino mit jeweils 3 bis 4 Kohlenstoffatomen in den einzelnen Alkenylteilen, oder für Trifluormethyl stehen oder

$R^1$ und $R^2$ oder $R^2$ und $R^3$ gemeinsam für einen 5-oder 6-gliedrigen carbocyclischen Ring stehen, mit der Maßgabe, daß mindestens einer der Reste $R^1$, $R^2$ oder $R^3$ für Alkyl mit 1 bis 6 Kohlenstoffatomen, Trifluormethyl oder für einen Teil des ankondensierten 5-oder 6-gliedrigen carbocyclischen Ring steht,

Z für eine (-CH=)-Gruppe oder ein Stickstoffatom steht,

X für Sauerstoff oder Schwefel steht,

Y für Fluor, Chlor, Brom, Iod, Nitro, Cyano, Amino, für Alkyl, Alkoxy, Halogenalkyl, Alkylcarbonylamino, Alkoxycarbonylamino mit jeweils 1 bis 4 Kohlenstoffatomen im Alkylteil und im Fall des Halogenalkyl mit 1 bis 5 Halogenatomen steht, wobei Halogen für Fluor, Chlor, Brom und Iod steht, für Phenoxycarbonylamino steht, wobei Y gleich oder verschieden sein kann,

n für eine ganze Zahl 0, 1, 2, 3 oder 4 steht,

A für Sauerstoff, Schwefel, einen Rest $R^5$-N= oder einen Rest $R^6$O-N= steht,

wobei

$R^5$ für Wasserstoff, für Alkyl, Hydroxyalkyl oder Alkoxyalkyl mit jeweils 1 bis 4 Kohlenstoffatomen in den einzelnen Alkylteilen, für Alkenyl mit 3 bis 4 Kohlenstoffatomen, für gegebenenfalls durch Fluor, Chlor, Brom, $C_1$-$C_4$-Alkyl oder $C_1$-$C_4$-Alkoxy substituiertes Cycloalkyl mit 3 bis 7 Kohlenstoffatomen, für jeweils gegebenenfalls durch Fluor, Chlor, Brom, Iod, $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl oder Halogen-$C_1$-$C_4$-alkylsulfonyl substituiertes Phenyl, Benzyl oder Phenethyl steht,

$R^6$ für Wasserstoff oder für Alkyl mit 1 bis 4 Kohlenstoffatomen oder Alkenyl mit 3 bis 4 Kohlenstoffatomen steht,

B für Sauerstoff, Schwefel oder für einen Rest $-\underset{|}{N}-R^7$ steht,

wobei

$R^7$ für Wasserstoff, für gegebenenfalls durch Fluor, Chlor, Brom, Iod, Cyano oder durch einen Rest -D-$R^9$ substituiertes Alkyl mit 1 bis 4 Kohlenstoffatomen oder für Alkenyl mit 3 bis 4 Kohlenstoffatomen steht, wobei

D für Sauerstoff, Schwefel, Sulfinyl oder Sulfonyl steht und

$R^9$ für Wasserstoff, für Alkyl mit 1 bis 4 Kohlenstoffatomen, für Alkenyl mit 3 bis 4 Kohlenstoffatomen, für jeweils gegebenenfalls substituiertes Phenyl, Benzyl oder Phenethyl steht, wobei als Phenylsubstituenten Fluor, Chlor, Brom, $C_1$-$C_4$-Alkyl oder $C_1$-$C_4$-Alkoxy ausgewählt sind,

$R^4$ für jeweils gegebenenfalls substituiertes Alkyl mit 1 bis 12 Kohlenstoffatomen, jeweils einfach oder mehrfach ungesättigtes Alkenyl oder Alkinyl mit jeweils bis zu 12 Kohlenstoffatomen, wobei als Substituenten jeweils ausgewählt sind:

Fluor, Chlor, Cyano, Alkoximino mit 1 bis 4 Kohlenstoffatomen im Alkylteil, gegebenenfalls durch $C_1$-$C_4$-Alkyl und/oder Fluor und/oder Chlor substituiertes Cycloalkyl mit 3 bis 6 Kohlenstoffatomen, gegebenenfalls durch Fluor, Chlor, Brom, Nitro, Trifluormethyl, $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Phenyl, einen 5-oder 6-gliedrigen Heterocyclus der 1 bis 3 Heteroatome aus der Reihe Sauerstoff und/oder Schwefel und/oder Stickstoff enthalten kann, ein Rest -D-$R^9$, ein Rest

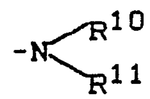 , 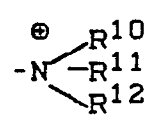 ,

-CO-$OR^{13}$, -CO-$NR^{14}R^{15}$, -CS-$NR^{14}R^{15}$, -$SO_2$-$NR^{14}R^{15}$;

wobei

D und $R^9$ die oben angegebene Bedeutung haben,

$R^{10}$ für Wasserstoff oder Alkyl mit 1 bis 4 Kohlenstoffatomen steht,

$R^{11}$ für Alkyl mit 1 bis 4 Kohlenstoffatomen, Acyl, Alkylsulfonyl mit 1 bis 4 Kohlenstoffatomen oder Phenylsulfonyl steht oder

$R^{10}$ und $R^{11}$ gemeinsam mit dem Stickstoff, an den sie gebunden sind, für einen 5-bis 7-gliedriegen Heterocyclus stehen,

$R^{12}$ für Alkyl mit 1 bis 4 Kohlenstoffatomen steht,

$R^{13}$ für Alkyl mit 1 bis 4 Kohlenstoffatomen steht,

$R^{14}$ und $R^{15}$ jeweils unabhängig voneinander für Wasserstoff oder Alkyl mit 1 bis 6 Kohlenstoffatomen stehen oder

$R^{14}$ und $R^{15}$ gemeinsam mit dem Stickstoff, an den sie gebunden sind, für einen 5-oder 6-gliedrigen Heterocyclus stehen, oder

$R^4$ weiterhin für gegebenenfalls durch Fluor, Chlor, $C_1$-$C_4$-Alkyl, $C_1$-$C_4$-Alkoxy, $C_2$-$C_4$-Alkenyl und/oder Halogen-$C_1$-$C_4$-alkyl, wobei Halogen für 1 bis 5 Fluor-und/oder Chloratome steht, substituiertes Cycloalkyl mit 3 bis 8 Kohlenstoffatomen steht, für gegebenenfalls substituiertes Phenyl steht, wobei als Phenylsubstituenten ausgewählt sind: Fluor, Chlor, Brom, $C_1$-$C_4$-Alkyl, Halogen-$C_1$-$C_4$-alkyl, wobei Halogen für 1 bis 5 Fluor-und/oder Chlor-und/oder Bromatome steht, oder ein Rest -$D^1$-$R^{17}$, wobei

$D^1$ fur Sauerstoff, Schwefel, Sulfinyl oder Sulfonyl steht,

$R^{17}$ für Wasserstoff, $C_1$-$C_4$-Alkyl, Halogen-$C_1$-$C_4$-alkyl, wobei Halogen für 1 bis 5 Fluor-und/oder Chloratome steht, $C_3$-$C_4$-Alkenyl, ein Rest

$$-CO-O-C_1-C_4\text{-Alkyl},\quad CO-N\begin{matrix}C_1-C_4\text{-Alkyl}\\ C_1-C_4\text{-Alkyl}\end{matrix},$$

gegebenenfalls am Stickstoff einfach oder zweifach durch $C_1$-$C_4$-Alkyl substituiertes Sulfonamid,

$R^4$ weiterhin für einen 5-bis 7-gliedrigen Heterocyclus steht, der ein bis drei gleiche oder verschiedene Heteroatome aus der Reihe Sauerstoff, Stickstoff oder Schwefel enthalten kann und welcher gegebenenfalls substituiert ist durch Fluor, Chlor, Brom, Iod, $C_1$-$C_4$-Alkyl, Halogen-$C_1$-$C_4$-alkyl, wobei Halogen für 1 bis 5 gleiche oder verschiedene Fluor-, Chlor-, Brom-oder Iodatome steht, durch Amino, Alkylamino oder Dialkylamino mit jeweils 1 bis 4 Kohlenstoffatomen in den einzelnen Alkylteilen, durch Nitro, Cyano, durch den Rest -D-$R^9$ (mit gleicher Bedeutung für D und $R^9$ wie oben), den Rest -CO-O-$C_1$-$C_4$-Alkyl, CO-NH-$C_1$-$C_4$-Alkyl oder -CO-N($C_1$-$C_4$-Alkyl)$_2$ steht und der carbocyclische oder weitere heterocyclische Ringe ankondensiert enthalten kann, oder

$R^4$ gemeinsam mit $R^5$, $R^6$, $R^7$ oder $R^8$ und B oder gemeinsam mit A und B einen 5-oder 6-gliedrigen Ring bildet.

3. Pyri(mi)dyl-oxy-und -thio-benzoesäure-Derivate der Formel (I) gemäß Anspruch 1, in welcher

$R^1$, $R^2$ und $R^3$ unabhängig voneinander jeweils für Wasserstoff, Fluor, Chlor, Brom, für Alkyl mit 1 bis 4 Kohlenstoffatomen, für gegebenenfalls durch $C_2$-$C_4$-Alkenyl oder $C_1$-$C_2$-Alkoxy substituiertes Alkoxy, Alkylamino, Dialkylamino mit jeweils 1 bis 4 Kohlenstoffatomen in den einzelnen Alkylteilen, wobei im Fall des Dialkylamino die Alkylsubstituenten mit dem Stickstoffatom, an das sie gebunden sind, einen 5-bis 7-gliedrigen heterocyclischen Ring bilden können, für Amino, Alkenylamino oder Dialkenylamino mit jeweils 3 bis 4 Kohlenstoffatomen in den einzelnen Alkenylteilen, für Alkenyl mit 2 bis 6 Kohlenstoffatomen, oder für Trifluormethyl stehen oder

$R^1$ und $R^2$ oder $R^2$ und $R^3$ gemeinsam für einen 5-oder 6-gliedrigen carbocyclischen Ring stehen, mit der Maßgabe, daß mindestens einer der Reste $R^1$, $R^2$ oder $R^3$ für Alkyl mit 1 bis 4 Kohlenstoffatomen, Trifluormethyl oder für einen Teil des ankondensierten 5-oder 6-gliedrigen carbocyclischen Ring steht,

Z für eine (-CH=)-Gruppe oder ein Stickstoffatom steht,

X für Sauerstoff oder Schwefel steht,

Y für Fluor, Chlor, Brom, Iod, Nitro, Amino, für Alkyl, Alkoxy, Halogenalkyl, Alkylcarbonylamino Alkoxycarbonylamino mit jeweils 1 bis 3 Kohlenstoffatomen im Alkylteil und im Fall des Halogenalkyl mit 1 bis 3 gleichen oder verschiedenen Halogenatomen steht, wobei Halogen für Fluor, Chlor, Brom oder Iod steht, für Phenoxycarbonylamino steht, wobei Y gleich oder verschieden sein kann,

n für eine ganze Zahl 0, 1, 2, 3 oder 4 steht,

A für Sauerstoff, Schwefel, einen Rest $R^5$-N= oder einen Rest $R^6$O-N= steht,

wobei

$R^5$ für Wasserstoff, für Alkyl oder Hydroxyalkyl mit jeweils 1 bis 4 Kohlenstoffatomen, für Alkenyl mit 3 bis 4

Kohlenstoffatomen, für gegebenenfalls einfach bis dreifach, gleich oder verschieden durch Fluor, Chlor, Brom, $C_1$-$C_2$-Alkyl oder $C_1$-$C_2$-Alkoxy substituiertes Cycloalkyl mit 3 bis 6 Kohlenstoffatomen, für jeweils gegebenenfalls einfach bis dreifach, gleich oder verschieden durch Fluor, Chlor, Brom, Iod, $C_1$-$C_2$-Alkyl, $C_1$-$C_2$-Alkoxy, $C_1$-$C_2$-Alkylthio, $C_1$-$C_2$-Alkylsulfinyl, $C_1$-$C_2$-Alkylsulfonyl oder Halogen-$C_1$-$C_2$-alkylsulfonyl substituiertes Phenyl, Benzyl oder Phenethyl steht,

$R^6$ für Wasserstoff oder für Alkyl mit 1 bis 4 Kohlenstoffatomen steht,

B für Sauerstoff, Schwefel oder für einen Rest 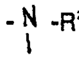

steht,

wobei

$R^7$ für Wasserstoff, für gegebenenfalls einfach bis dreifach, gleich oder verschieden durch Fluor, Chlor, Brom, Iod, Cyano, Alkenyl mit 3 bis 4 Kohlenstoffatomen oder durch einen Rest -D-$R^8$ substituiertes Alkyl mit 1 bis 4 Kohlenstoffatomen steht, wobei

D für Sauerstoff, Schwefel oder Sulfonyl steht und

$R^8$ für Wasserstoff, für Alkyl mit 1 bis 4 Kohlenstoffatomen, für Alkenyl mit 3 bis 4 Kohlenstoffatomen, für jeweils gegebenenfalls einfach bis dreifach, gleich oder verschieden substituiertes Phenyl, Benzyl oder Phenethyl, wobei als Phenylsubstituenten Fluor, Chlor, Brom, $C_1$-$C_2$-Alkyl oder $C_1$-$C_2$-Alkoxy ausgewählt sind,

$R^4$ für gebenenfalls einfach bis fünffach, gleich oder verschieden substituiertes Alkyl mit 1 bis 10 Kohlenstoffatomen, jeweils einfach bis dreifach, gleich oder verschieden substituiertes ungesättigtes Alkenyl oder Alkinyl mit jeweils bis zu 10 Kohlenstoffatomen steht, wobei als Substituenten jeweils ausgewählt sind: Fluor, Chlor, Cyano, Alkoximino mit 1 bis 4 Kohlenstoffatomen im Alkylteil, gegebenenfalls einfach bis dreifach, gleich oder verschieden durch $C_1$-$C_4$-Alkyl substituiertes Cycloalkyl mit 3 bis 6 Kohlenstoffatomen, gegebenenfalls einfach bis dreifach, gleich oder verschieden durch Fluor, Chlor, Brom, Iod, Nitro, Trifluormethyl, $C_1$-$C_2$-Alkyl, $C_1$-$C_2$-Alkoxy, $C_1$-$C_2$-Alkylthio, $C_1$-$C_2$-Alkylsulfinyl oder durch $C_1$-$C_4$-Alkylsulfonyl substituiertes Phenyl, einen 5-oder 6-gliedrigen Heterocyclus der 1 bis 3 Heteroatome aus der Reihe Sauerstoff und/oder Schwefel und/oder Stickstoff enthalten kann, ein Rest -D-$R^8$, ein Rest

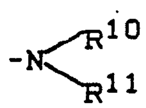 , 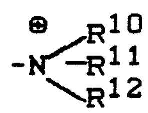 ,

-CO-$OR^{13}$, -CO-$NR^{14}R^{15}$, -CS-$NR^{14}R^{15}$, wobei

D und $R^8$ die oben angegebene Bedeutung haben,

$R^{10}$ für Wasserstoff oder Alkyl mit 1 bis 4 Kohlenstoffatomen steht,

$R^{11}$ für Alkyl mit 1 bis 4 Kohlenstoffatomen oder Acyl steht,

$R^{10}$ und $R^{11}$ gemeinsam mit dem Stickstoff, an den sie gebunden sind, für einen 5-bis 6-gliedriegen Heterocyclus stehen,

$R^{12}$ für Alkyl mit 1 bis 4 Kohlenstoffatomen steht,

$R^{13}$ für Alkyl mit 1 bis 4 Kohlenstoffatomen steht,

$R^{14}$ und $R^{15}$ jeweils unabhängig voneinander für Wasserstoff oder Alkyl mit 1 bis 6 Kohlenstoffatomen stehen oder

$R^{14}$ und $R^{15}$ gemeinsam mit dem Stickstoff, an den sie gebunden sind, für einen 5-oder 6-gliedrigen Heterocyclus stehen, oder

$R^4$ weiterhin für gegebenenfalls durch Fluor, Chlor oder $C_1$-$C_4$-Alkyl, $C_1$-$C_4$-Alkoxy, $C_2$-$C_4$-Alkenyl oder Halogen-$C_1$-$C_2$-alkyl, wobei Halogen für 1 bis 3 Fluor-und/oder Chloratome steht, substituiertes Cycloalkyl mit 3 bis 7 Ring-Kohlenstoffatomen steht, für gegebenenfalls einfach bis dreifach, gleich oder verschieden substituiertes Phenyl steht, wobei als Phenylsubstituenten ausgewählt sind: Fluor, Chlor, Brom, $C_1$-$C_4$-Alkyl, Trifluormethyl oder ein Rest -$D^1$-$R^{17}$ steht, wobei

$D^1$ für Sauerstoff, Schwefel, Sulfinyl oder Sulfonyl steht,

$R^{17}$ für Wasserstoff oder $C_1$-$C_4$-Alkyl steht,

$R^4$ weiterhin für einen 5-bis 7-gliedrigen Heterocyclus steht, der ein bis drei gleiche oder verschiedene Heteroatome aus der Reihe Sauerstoff, Stickstoff oder Schwefel enthalten kann und welcher gegebenenfalls einfach bis dreifach, gleich oder verschieden substituiert ist durch Fluor, Chlor, Brom, Iod, $C_1$-$C_2$-Alkyl, Halogen-$C_1$-$C_2$-alkyl, wobei Halogen für 1 bis 3 gleiche oder verschiedene Fluor-oder Chloratome steht, durch Amino, Alkylamino oder Dialkylamino mit jeweils 1 bis 2 Kohlenstoffatomen in den einzelnen Alkylteilen, durch Nitro, Cyano, oder durch einen $C_1$-$C_4$-Alkoxy-, $C_1$-$C_4$-Alkylthio-oder $C_1$-$C_4$-Alkylsulfonylrest,

oder

$R^4$ gemeinsam mit $R^5$, $R^6$, $R^7$ oder $R^8$ und B oder gemeinsam mit A und B einen 5-oder 6-gliedrigen Ring bildet.

4. Pyri(mi)dyl-oxy-und thio-benzoesäure-Derivate der Formel (I) gemäß Anspruch 1, in welcher

$R^1$, $R^2$ und $R^3$ unabhängig voneinander jeweils für Wasserstoff, Fluor, Chlor, Brom, für Alkyl mit 1 bis 4 Kohlenstoffatomen, für gegebenenfalls durch $C_2$-$C_4$-Alkenyl oder $C_1$-$C_2$-Alkoxy substituiertes Alkoxy, Alkylamino, Dialkylamino mit jeweils 1 bis 4 Kohlenstoffatomen in den einzelnen Alkylteilen, wobei im Fall des Dialkylamino die Alkylsubstituenten mit dem Stickstoffatom, an das sie gebunden sind, einen 5-bis 7-gliedrigen heterocylischen Ring bilden können, für Amino, Alkenylamino oder Dialkenylamino mit jeweils 3 bis 4 Kohlenstoffatomen in den einzelnen Alkenylteilen, für Alkenyl mit 2 bis 6 Kohlenstoffatomen, für Trifluormethyl oder

$R^1$ und $R^2$ oder $R^2$ und $R^3$ gemeinsam für einen 5-oder 6-gliedrigen carbocyclischen Ring stehen,

mit der Maßgabe, daß mindestens einer der Reste $R^1$, $R^2$ oder $R^3$ für Alkyl mit 1 bis 4 Kohlenstoffatomen, Trifluormethyl oder für einen Teil des ankondensierten 5-oder 6-gliedrigen carbocyclischen Ring steht,

Z für ein Stickstoffatom steht,

X für Sauerstoff oder Schwefel steht,

Y für Fluor, Chlor, Brom, Iod, Nitro, Amino, für Alkyl, Alkoxy, Halogenalkyl, Alkylcarbonylamino, Alkoxycarbonylamino mit jeweils 1 bis 3 Kohlenstoffatomen im Alkylteil und im Fall des Halogenalkyl mit 1 bis 3 Halogenatomen steht, wobei Halogen für Fluor, Chlor, Brom und/oder Iod steht, oder für Phenoxycarbonylamino steht, wobei Y gleich oder verschieden sein kann,

n für eine ganze Zahl 0, 1, 2 oder 3 steht,

A für Sauerstoff oder Schwefel steht,

B für Sauerstoff, Schwefel oder für einen Rest $-\underset{|}{N}-R^7$ steht,

wobei

$R^7$ für Wasserstoff, für gegebenenfalls einfach bis dreifach, gleich oder verschieden durch Fluor, Chlor, Brom, Iod, Cyano oder Alkenyl mit 3 bis 4 Kohlenstoffatomen substituiertes Alkyl mit 1 bis 4 Kohlenstoffatomen steht,

$R^4$ für jeweils gegebenenfalls einfach bis fünffach, gleich oder verschieden substituiertes Alkyl mit 1 bis 10 Kohlenstoffatomen, jeweils einfach oder mehrfach ungesättigtes Alkenyl oder Alkinyl mit jeweils bis zu 10 Kohlenstoffatomen steht, wobei als Substituenten jeweils ausgewählt sind:

Fluor, Chlor, Cyano, Alkoximino mit 1 bis 4 Kohlenstoffatomen im Alkylteil, gegebenenfalls durch $C_1$-$C_4$-Alkyl substituiertes Cycloalkyl mit 3 bis 6 Kohlenstoffatomen, gegebenenfalls durch Fluor, Chlor, Brom, Iod, Nitro, Trifluormethyl, $C_1$-$C_2$-Alkyl, $C_1$-$C_2$-Alkoxy, $C_1$-$C_2$-Alkylthio, $C_1$-$C_2$-Alkylsulfinyl oder durch $C_1$-$C_4$-Alkylsulfonyl substituiertes Phenyl, einen 5-oder 6-gliedrigen Heterocyclus der 1 bis 3 Heteroatome aus der Reihe Sauerstoff und/oder Schwefel und/oder Stickstoff enthalten kann, ein Rest -D-$R^9$, ein Rest

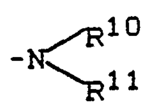 , 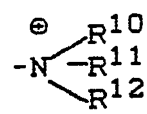 ,

-CO-$OR^{13}$, -CO-$NR^{14}R^{15}$, -CS-$NR^{14}R^{15}$, wobei

D für Sauerstoff, Schwefel oder Sulfonyl steht und

$R^9$ für Wasserstoff, für Alkyl mit 1 bis 4 Kohlenstoffatomen, für Alkenyl mit 3 bis 4 Kohlenstoffatomen, für jeweils gegebenenfalls einfach bis dreifach, gleich oder verschieden substituiertes Phenyl, Benzyl oder Phenethyl steht, wobei als Phenylsubstituenten Fluor, Chlor, Brom, $C_1$-$C_2$-Alkyl oder $C_1$-$C_2$-Alkoxy ausgewählt sind,

$R^{10}$ für Wasserstoff oder Alkyl mit 1 bis 4 Kohlenstoffatomen steht,

$R^{11}$ für Alkyl mit 1 bis 4 Kohlenstoffatomen oder Acyl steht,

$R^{10}$ und $R^{11}$ gemeinsam mit dem Stickstoff, an den sie gebunden sind, für einen 5-bis 6-gliedrigen Heterocyclus stehen,

$R^{12}$ für Alkyl mit 1 bis 4 Kohlenstoffatomen steht,

$R^{13}$ für Alkyl mit 1 bis 4 Kohlenstoffatomen steht,

$R^{14}$ und $R^{15}$ jeweils unabhängig voneinander für Wasserstoff oder Alkyl mit 1 bis 6 Kohlenstoffatomen stehen oder

$R^{14}$ und $R^{15}$ gemeinsam mit dem Stickstoff, an den sie gebunden sind, für einen 5-oder 6-gliedrigen Heterocyclus stehen, oder

$R^4$ weiterhin für gegebenenfalls einfach bis dreifach, gleich oder verschieden durch Fluor, Chlor oder $C_1$-$C_4$-Alkyl, $C_1$-$C_4$-Alkoxy, $C_2$-$C_4$-Alkenyl oder Halogen-$C_1$-$C_2$-alkyl, wobei Halogen für 1 bis 3 Fluor-und/oder Chloratome steht, substituiertes Cycloalkyl mit 3 bis 6 Kohlenstoffatomen, steht, für Benzyl, Phenethyl, für gegebenenfalls einfach bis dreifach, gleich oder verschieden substituiertes Phenyl, wobei als Phenylsubstituenten ausgewählt sind: Fluor, Chlor, Brom, $C_1$-$C_4$-Alkyl oder Trifluormethyl, oder ein Rest -$D^1$-$R^{17}$ steht, wobei

$D^1$ für Sauerstoff, steht,

$R^{17}$ für Wasserstoff oder $C_1$-$C_4$-Alkyl steht,

$R^4$ weiterhin für einen 5-bis 7-gliedrigen Heterocyclus steht, der ein bis drei gleiche oder verschiedene Heteroatome aus der Reihe Sauerstoff, Stickstoff oder Schwefel enthalten kann und welcher gegebenenfalls einfach bis dreifach, gleich oder verschieden substituiert ist durch Fluor, Chlor, Brom, Iod, $C_1$-$C_2$-Aklyl, Halogen-$C_1$-$C_2$-alkyl, wobei Halogen für 1 bis 3 gleiche oder verschiedene Fluor-oder Chloratome steht, durch Amino, Alkylamino oder Dialkylamino mit jeweils 1 bis 2 Kohlenstoffatomen in den einzelnen Alkylteilen, durch Nitro, Cyano, $C_1$-$C_4$-Alkoxy-, $C_1$-$C_4$-Alkylthio oder $C_1$-$C_4$-Alkylsulfonyl, oder

$R^4$ gemeinsam mit $R^5$, $R^6$, $R^7$ oder $R^8$ und B oder gemeinsam mit A und B einen 5-oder 6-gliedrigen Ring bildet.

5. Pyri(mi)dyl-oxy-und -thio-benzoesäure-Derivate der Formel (I), gemäß Anspruch 1, in welcher

$R^1$, $R^2$ und $R^3$ unabhängig voneinander jeweils für Wasserstoff, Fluor, Chlor, Brom, für Alkyl mit 1 bis 4 Kohlenstoffatomen, für gegebenenfalls durch $C_2$-$C_4$-Alkenyl oder $C_1$-$C_2$-Alkoxy substituiertes Alkoxy, Alkylamino, Dialkylamino mit jeweils 1 bis 4 Kohlenstoffatomen in den einzelnen Alkylteilen, wobei im Fall des Dialkylamino die Alkylsubstituenten mit dem Stickstoffatom, an das sie gebunden sind, einen 5-bis 7-gliedrigen heterocyclischen Ring bilden können, für Amino, Alkenylamino oder Dialkenylamino mit jeweils 3 bis 4 Kohlenstoffatomen in den einzelnen Alkenylteilen, für Alkenyl mit 2 bis 6 Kohlenstoffatomen oder für Trifluormethyl stehen oder

$R^1$ und $R^2$ oder $R^2$ und $R^3$ gemeinsam für einen 5-oder 6-gliedrigen carbocyclischen Ring stehen,

mit der Maßgabe, daß mindestens einer der Reste $R^1$, $R^2$ oder $R^3$ für Alkyl mit 1 bis 4 Kohlenstoffatomen, Trifluormethyl oder für einen Teil des ankondensierten 5-oder 6-gliedrigen carbocyclischen Ring steht,

Z für ein Stickstoffatom steht,

X für Sauerstoff oder Schwefel steht,

Y für Fluor, Chlor, Brom, Iod, Nitro, Amino, für Alkyl, Alkoxy, Halogenalkyl, Alkylcarbonylamino, Alkoxycarbonylamino mit jeweils 1 bis 3 Kohlenstoffatomen in den Alkylteilen und im Fall des Halogenalkyl mit 1 bis 3 Halogenatomen steht, wobei Halogen für Fluor, Chlor, Brom und/oder Iod steht, für Phenoxycarbonylamino steht, wobei Y gleich oder verschieden sein kann,

n für eine ganze Zahl 0, 1, 2 oder 3 steht,

A für einen Rest $R^5$-N= oder einen Rest $R^6$O-N= steht,

wobei

$R^5$ für Wasserstoff, für Alkyl oder Hydroxyalkyl mit jeweils 1 bis 4 Kohlenstoffatomen oder für Alkenyl mit 3 bis 4 Kohlenstoffatmen steht,

$R^6$ für Wasserstoff oder für Alkyl mit 1 bis 4 Kohlenstoffatomen steht,

B für Sauerstoff, Schwefel oder für einen Rest -N(-)-$R^7$ steht,

wobei

$R^7$ für Wasserstoff, für gegebenenfalls einfach bis dreifach, gleich oder verschieden durch Fluor, Chlor, Brom, Iod, Cyano oder Alkenyl mit 3 bis 4 Kohlenstoffatomen substituiertes Alkyl mit 1 bis 4 Kohlenstoffatomen steht,

$R^4$ für jeweils gegebenenfalls einfach bis fünffach, gleich oder verschieden substituiertes Alkyl mit 1 bis 10 Kohlenstoffatomen steht, jeweils einfach oder mehrfach ungesättigtes Alkenyl oder Alkinyl mit jeweils bis zu 10 Kohlenstoffatomen, wobei als Substituenten jeweils ausgewählt sind:

Fluor, Chlor, Cyano, Alkoximino mit 1 bis 4 Kohlenstoffatomen im Alkylteil, gegebenenfalls einfach bis dreifach durch $C_1$-$C_4$-Alkyl substituiertes Cycloalkyl mit 3 bis 6 Kohlenstoffatomen, gegebenenfalls einfach bis dreifach, gleich oder verschieden durch Fluor, Chlor, Brom, Iod, Nitro, Trifluormethyl, $C_1$-$C_2$-Alkyl, $C_1$-$C_2$-Alkoxy, $C_1$-$C_2$-Alkylthio, $C_1$-$C_2$-Alkylsulfinyl oder durch $C_1$-$C_4$-Alkylsulfonyl substituiertes Phenyl, einen 5-oder 6-gliedrigen Heterocyclus der 1 bis 3 Heteroatome aus der Reihe Sauerstoff und/oder Schwefel und/oder Stickstoff enthalten kann, ein Rest -D-$R^9$,

wobei

D für Sauerstoff, Schwefel oder Sulfonyl steht und

$R^9$ für Wasserstoff, für Alkyl mit 1 bis 4 Kohlenstoffatomen, für Alkenyl mit 3 bis 4 Kohlenstoffatomen, für jeweils gegebenenfalls einfach bis dreifach, gleich oder verschieden substituiertes Phenyl, Benzyl oder

Phenethyl steht, wobei als Phenylsubstituenten Fluor, Chlor, Brom, $C_1$-$C_2$-Alkyl oder $C_1$-$C_2$-Alkoxy ausgewählt sind,

$R^4$ weiterhin für gegebenenfalls einfach bis dreifach, gleich oder verschieden durch Fluor, Chlor oder $C_1$-$C_4$-Alkyl substituiertes Cycloalkyl mit 3 bis 6 Kohlenstoffatomen steht, für gegebenenfalls einfach bis dreifach, gleich oder verschieden substituiertes Phenyl steht, wobei als Phenylsubstituenten ausgewählt sind: Fluor, Chlor, Brom, $C_1$-$C_4$-Alkyl, Trifluormethyl, oder ein Rest -$D^1$-$R^{17}$ steht, wobei

$D^1$ für Sauerstoff steht,

$R^{17}$ für Wasserstoff oder $C_1$-$C_4$-Alkyl steht,

$R^4$ weiterhin für einen 5-bis 7-gliedrigen Heterocyclus steht, der ein bis drei gleiche oder verschiedene Heteroatome aus der Reihe Sauerstoff, Stickstoff oder Schwefel enthalten kann und welcher gegebenenfalls einfach bis dreifach, gleich oder verschieden substituiert ist durch Fluor, Chlor, Brom, Iod, $C_1$-$C_2$-Alkyl, Halogen-$C_1$-$C_2$-alkyl, wobei Halogen für 1 bis 3 gleiche oder verschiedene Fluor-oder Chloratome steht, durch Amino, Alkylamino oder Dialkylamino mit jeweils 1 bis 2 Kohlenstoffatomen in den einzelnen Alkylteilen, durch Nitro, Cyano, $C_1$-$C_4$-Alkoxy, $C_1$-$C_4$-Alkylthio oder $C_1$-$C_4$-Alkylsulfonyl, oder

$R^4$ gemeinsam mit $R^5$, $R^6$, $R^7$ oder $R^8$ und B oder gemeinsam mit A und B einen 5-oder 6-gliedrigen Ring bildet.

6. Verfahren zur Herstellung von Pyri(mi)dyl-oxy-und -thio-benzoesäure-Derivate der Formel (I),

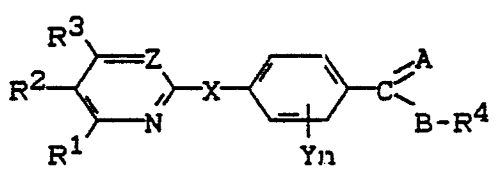

(I)

in welcher

$R^1$, $R^2$ und $R^3$ unabhängig voneinander jeweils für Wasserstoff, Halogen, Alkyl, für gegebenenfalls substituiertes Alkoxy, Halogenalkyl, Alkenyl oder fur gegebenenfalls substituiertes Amino steht oder

$R^1$ und $R^2$ oder $R^2$ und $R^3$ gemeinsam für einen ankondensierten 3-bis 6-gliedrigen carbocyclischen Ring stehen,

mit der Maßgabe, daß mindestens einer der Reste $R^1$, $R^2$ oder $R^3$ für Alkyl oder für einen Teil des 3-bis 6-gliedrigen ankondensierten carbocyclischen Ringes steht,

Z für eine (-CH=)-Gruppe oder ein Stickstoffatom steht,

X für Sauerstoff oder Schwefel steht,

Y für Halogen, Nitro, Cyano, Amino, Alkylcarbonylamino, Alkoxycarbonylamino, Phenoxycarbonylamino, Alkyl, Alkoxy oder Halogenalkyl steht, wobei Y gleich oder verschieden sein kann,

n für eine ganze Zahl von 0 bis 4 steht,

A für Sauerstoff, Schwefel, einen Rest $R^5$-N= oder einen Rest $R^6$O-N= steht,

wobei

$R^5$ für Wasserstoff, Alkyl, Hydroxyalkyl, Alkenyl, für jeweils gegebenenfalls substituiertes Cycloalkyl, Aryl oder Aralkyl steht,

$R^6$ für Wasserstoff, jeweils gegebenenfalls substituiertes Alkyl oder einfach oder mehrfach ungesättigtes Alkenyl steht,

B für Sauerstoff, Schwefel, für einen Rest -N(-)-$R^7$ oder -N(-)-$OR^8$ steht,

wobei

$R^7$ für Wasserstoff, für gegebenenfalls durch Halogen, Cyano oder durch einen Rest -D-$R^9$ substituiertes Alkyl oder für Alkenyl steht, wobei

D für Sauerstoff, Schwefel, Sulfinyl oder Sulfonyl steht und

$R^9$ für Wasserstoff, Alkyl, einfach oder mehrfach ungesättigtes Alkenyl oder für jeweils gegebenenfalls substituiertes Aryl oder Aralkyl steht,

oder für -CO-Alkyl steht,

$R^8$ für Wasserstoff, jeweils gegebenenfalls substituiertes Alkyl oder einfach oder mehrfach ungesättigtes Alkenyl steht und

$R^4$ für gegebenenfalls substituiertes gesättigtes oder ungesättigtes Alkyl steht, wobei als Substituenten ausgewählt sind:

Halogen, Nitro, Cyano, ein Rest -D-$R^9$, Alkoximo, die Gruppen

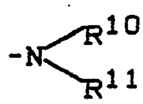 , 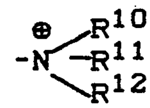 ,

$-CO-OR^{13}$, $-CO-NR^{14}R^{15}$, $-CS-NR^{14}R^{15}$, $-SO_2-NR^{14}R^{15}$, Cycloalkyl, Aryl oder ein 5-bzw. 6-gliedriger Heterocyclus;

$R^4$ weiterhin für jeweils gegebenenfalls substituiertes Cycloalkyl, Aryl oder einen 5-bis 7-gliedrigen Heterocyclus steht,

wobei

D und $R^9$ die oben angegebene Bedeutung haben,

$R^{10}$ für Wasserstoff oder Alkyl steht,

$R^{11}$ für Alkyl, Acyl, Alkylsulfonyl, Arylsulfonyl oder jeweils gegebenenfalls substituiertes Aryl oder Aralkyl steht oder

$R^{10}$ und $R^{11}$ gemeinsam mit dem Stickstoff, an den sie gebunden sind, auch für einen 5-bis 7-gliedrigen Heterocyclus stehen,

$R^{12}$ für Alkyl steht,

$R^{13}$ für Alkyl steht,

$R^{14}$ und $R^{15}$ unabhängig voneinander jeweils für Wasserstoff, Alkyl, Cycloalkyl oder für jeweils gegebenenfalls substituiertes Aryl oder Aralkyl stehen oder

$R^{14}$ und $R^{15}$ gemeinsam mit dem Stickstoff, an den sie gebunden sind, für einen 5-bis 7-gliedrigen Heterocyclus stehen oder

$R^4$ gemeinsam mit $R^5$ und B oder mit $R^6$ und B oder mit $R^7$ und B oder mit $R^8$ und B oder mit A und B einen 5-oder 6-gliedrigen Ring bilden kann,

dadurch gekennzeichnet, daß man

($A-a_1$) Verbindungen der Formel (I), in denen A für Sauerstoff steht, erhält wenn man Benzoesäure-Derivate der Formel (II),

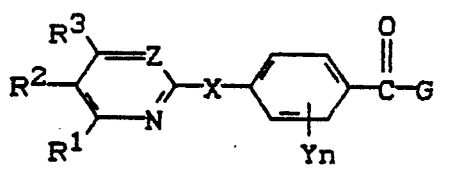 (II)

in welcher

$R^1$, $R^2$, $R^3$, Z, X, Y und n die oben angegebene Bedeutung haben und

G für Halogen, für den Rest $-O-\overset{O}{\overset{\|}{C}}-O-$Alkyl oder $-O-SO_2-$Aryl oder für Imidazolyl steht,

mit Verbindungen der Formel (III);

$H-B-R^4$ (III)

in welcher

B und $R^4$ die oben angegebene Bedeutung haben,

gegebenenfalls in Gegenwart eines Verdünnungsmittels und gegebenenfalls in Gegenwart eines Säurebindemittels umsetzt,

oder daß man

($A-a_2$) Verbindungen der Formel (I), in denen A für Schwefel steht, erhält, wenn man Thiobenzoylchloride der Formel (IV),

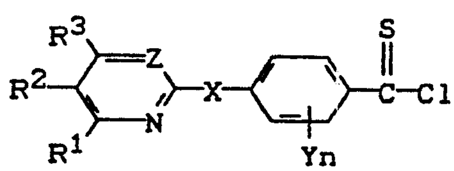 (IV)

in welcher

$R^1$, $R^2$, $R^3$, Z, X, Y und n die oben angegebene Bedeutung haben, mit Verbindungen der Formel (III),

H-B-$R^4$ (III)

in welcher

B und $R^4$ die oben angegebene Bedeutung haben, gegebenenfalls in Gegenwart eines Verdünnungsmittels sowie in Gegenwart eines Säurebindemittels umsetzt,

oder daß man

(A-$a_3$) Verbindungen der Formel (I), in denen A und B für Schwefel stehen, erhält wenn man Dithiobenzoesäuren der Formel (V),

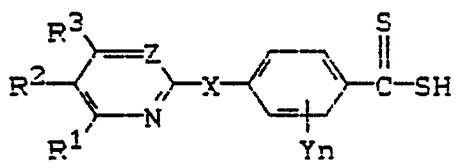

(V)

in welcher

$R^1$, $R^2$, $R^3$, Z, X, Y und n die oben angegebene Bedeutung haben,

($a_3$-$\alpha$) mit Alkylierungsmitteln der Formel (VI),

L-$R^{4-1}$ (VI)

in welcher

$R^{4-1}$ fur gegebenenfalls substituiertes Alkyl oder Aralkyl steht und

L für Halogen oder für den Rest $R^{4-1}$-O-$SO_2$-O-steht,

in Gegenwart eines Verdünnungsmittels und gegebenenfalls in Gegenwart eines Säurebindemittels umsetzt, oder

($a_3$-$\beta$) mit Olefinen der Formel (VII),

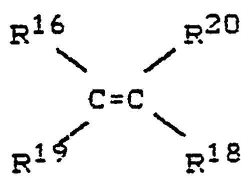

(VII)

in welcher

$R^{16}$, $R^{18}$, $R^{19}$ und $R^{20}$ unabhängig voneinander jeweils für Wasserstoff, Nitro, Cyano, für jeweils gegebenenfalls substituiertes Alkyl, Cycloalkyl oder Aryl stehen oder für einen Rest -COOR$^{13}$, -CO-NR$^{14}$R$^{15}$, -CS-NR$^{14}$R$^{15}$, -$SO_2$-NR$^{14}$R$^{15}$ oder den Rest -D-$R^9$ stehen oder

$R^{16}$ und $R^{20}$ gemeinsam eine dritte Kohlenstoff-Kohlenstoffbindung bilden,

wobei

D, $R^9$, $R^{13}$, $R^{14}$ und $R^{15}$ die oben angegebene Bedeutung haben,

in Gegenwart eines Verdünnungsmittels und gegebenenfalls in Gegenwart eines Katalysators umsetzt,

oder daß man

(A-$a_4$) Verbindungen der Formel (I), in denen A für Schwefel und B für den Rest $-\underset{|}{N}-R^7$ oder $-\underset{|}{N}-OR^8$ stehen, erhält, wenn man

($a_4$-$\alpha$) Dithiobenzoesäure-Derivate der Formel (Va),

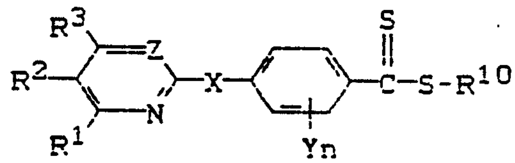

(Va)

in welcher
$R^1$, $R^2$, $R^3$, Z, X, Y und n die oben angegebene Bedeutung haben und
$R^{10}$ für Waserstoff oder Alkyl, insbesondere für Wasserstoff, Methyl oder Ethyl, steht,
mit Aminen der Formel (VIIIa) oder Hydroxylaminen der Formel (VIIIb),

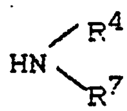

(VIIIa)

oder

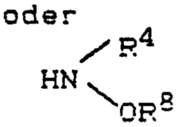

(VIIIb)

in welchen
$R^4$, $R^7$ und $R^8$ die oben angegebene Bedeutung haben,
gegebenenfalls in Gegenwart eines Verdünnungsmittels und gegebenenfalls in Gegenwart eines Katalysators umsetzt, oder daß man
($a_4$-$\beta$) Benzimidchloride der Formel (IXa),

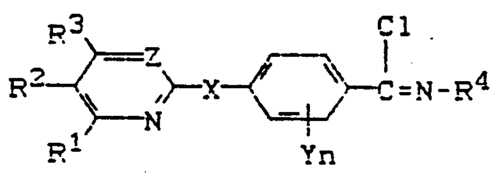

(IXa)

in welcher
$R^1$, $R^2$, $R^3$, $R^4$, Z, X, Y und n die oben angegebene Bedeutung haben,
mit Schwefelwasserstoff in Gegenwart eines Verdünnungsmittels umsetzt, oder daß man
($a_4$-$\gamma$) Benzoesäuremide der Formel (Ia)

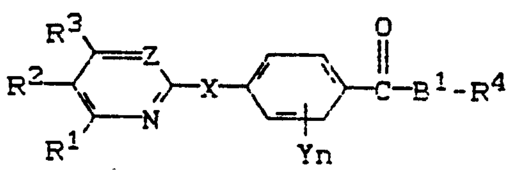

(Ia)

in welcher
$R^1$, $R^2$, $R^3$, $R^4$, Z, X, Y und n die oben angegebene Bedeutung haben und
$B^1$ für den Rest $-\underset{|}{N}-R^7$ oder $-\underset{|}{N}-OR^8$ steht,

mit Schwefelungsagentien gegebenenfalls in Gegenwart eines Verdünnungsmittels umsetzt,
oder daß man
(A-$a_5$) Verbindungen der Formel (I), in denen A für einen Rest $R^5$-N= steht, erhält, wenn man ($a_5$-$\alpha$) Benzimidchloride der Formel (IXb),

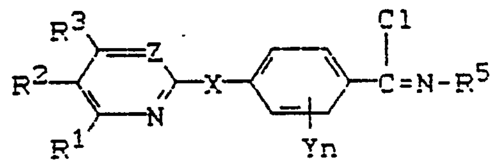

(IXb)

in welcher
$R^1$, $R^2$, $R^3$, $R^5$, Z, X, Y und n die oben angegebene Bedeutung haben,
mit Verbindungen der Formel (III),
H-B-$R^4$ (III)
in welcher
B und $R^4$ die oben angegebene Bedeutung haben,
gegebenenfalls in Gegenwart eines Verdünnungsmittels sowie in Gegenwart eines Säurebindemittels umsetzt,
oder daß man
($a_5$-$\beta$) Thiobenzamide der Formel (X)

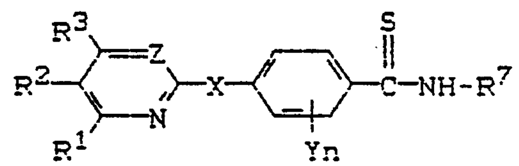

(X)

in welcher
$R^1$, $R^2$, $R^3$, $R^7$, Z, X, Y und n die oben angegebene Bedeutung haben,
mit Alkylierungsmitteln der Formel (VI),
L-$R^{4-1}$ (VI)
in welcher
$R^{4-1}$ und L die oben angegebene Bedeutung haben,
gegebenenfalls in Gegenwart eines Verdünnungsmittels und gegebenenfalls in Gegenwart eines Säurebindemittels umsetzt,
oder daß man
(A-$a_6$) Verbindungen der Formel (I), in denen A für den Rest $R^6$-O-N= steht, erhält, wenn man
($a_6$-$\alpha$) Hydroximsäurehalogenide der Formel (XIa),

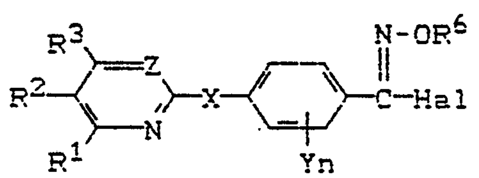

(XIa)

in welcher
$R^1$, $R^2$, $R^3$, $R^6$, Z, X, Y und n die oben angegebene Bedeutung haben und
Hal für Chlor oder Brom steht,
mit Verbindungen der Formel (III),
H-B-$R^4$ (III)
in welcher
B und $R^4$ die oben angegebene Bedeutung haben,
gegebenenfalls in Gegenwart eines Verdünnungsmittels sowie in Gegenwart eines Säurebindemittels umsetzt oder daß man
($a_6$-$\beta$) Hydroxamsäuren der Formel (XIb),

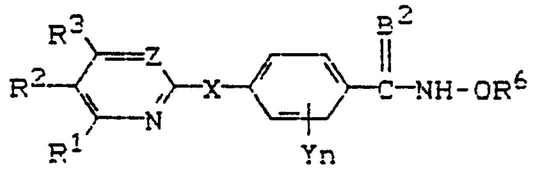 (XIb)

in welcher
$R^1$, $R^2$, $R^3$, $R^6$, Z, X, Y und n die oben angegebene Bedeutung haben und
$B^2$ für Sauerstoff oder Schwefel steht,
mit Alkylierungsmitteln der Formel (VI),
$L-R^{4-1}$ (VI)
in welcher
L und $R^{4-1}$ die oben angegebene Bedeutung haben
gegebenenfalls in Gegenwart eines Verdünnungsmittels und gegebenenfalls in Gegenwart eines Säurebindemittels umsetzt oder daß man
($a_6$-γ) Thiobenzoesäureester der Formel (Ib),

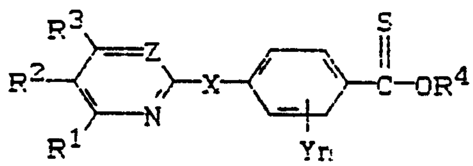 (Ib)

in welcher
$R^1$, $R^2$, $R^3$, $R^4$, Z, X, Y und n die oben angegebene Bedeutung haben,
mit Hydroxylaminen der Formel (XII),
$R^6-O-NH_2$ (XII)
in welcher
$R^6$ die oben angegebene Bedeutung hat,
gegebenenfalls in Gegenwart eines Verdünnungsmittels umsetzt,
oder daß man
(A-$a_7$) Verbindungen der Formel (I), in denen $R^4$, A und B gemeinsam einen heterocyclischen Ring bilden, erhält, wenn man
($a_7$-γ) Benzonitrile der Formel (XIII),

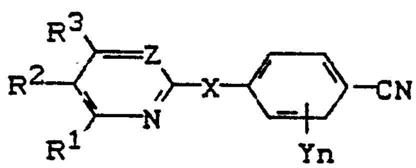 (XIII)

in welcher
$R^1$, $R^2$, $R^3$, Z, X, Y und n die oben angegebene Bedeutung haben,
mit 2-oder 3-Hydroxylaminen der Formel (XIV),

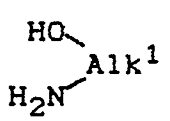 (XIV)

in welcher
$Alk^1$ für eine gegebenenfalls substituierte $C_2$-$C_3$-Alkylenkette steht, wobei als Substituenten ausgewählt sind: Fluor, Chlor, $C_1$-$C_3$-Alkyl, $C_1$-$C_3$-Alkoxy, Hydroxy-$C_1$-$C_3$-alkyl, $C_3$-$C_6$-Cycloalkyl oder gegebenenfalls durch Fluor, Chlor, Methyl, Ethyl, Methoxy und/oder Trifluormethyl substituiertes Phenyl, gegebenenfalls in

Gegenwart eines Verdünnungsmittels und in Gegenwart eines Katalysators umsetzt zu den Verbindungen der Formel (Ic),

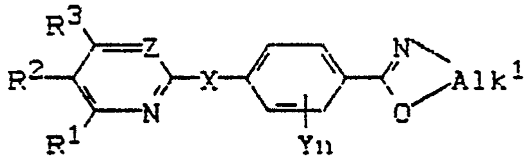

(Ic)

in welcher
$R^1$, $R^2$, $R^3$, $Alk^1$, Z, X, Y und n die oben angegebene Bedeutung haben,
oder daß man
($a_7$-$\beta$) Thiobenzamide der Formel (Xa),

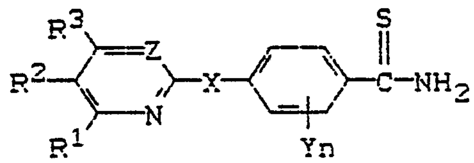

(Xa)

in welcher
$R^1$, $R^2$, $R^3$, Z, X, Y und n die oben angegebene Bedeutung haben,
mit $\alpha$-Halogencarbonyl-Derivaten der Formel (XV),

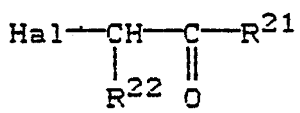

(XV)

in welcher
Hal für Chlor oder Brom steht,
$R^{21}$ und $R^{22}$ unabhängig voneinander für Wasserstoff, Alkyl, Cycloalkyl oder gegebenenfalls substituiertes Aryl steht,
gegebenenfalls in Gegenwart eines Verdünnungsmittels sowie in Gegenwart eines Säurebindemittels umsetzt zu Verbindungen der Formel (Id),

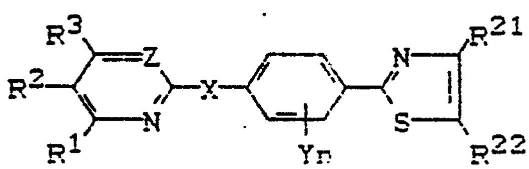

(Id)

in welcher
$R^1$, $R^2$, $R^3$, $R^{21}$, $R^{22}$, Z, X, Y und n die oben angegebene Bedeutung haben,
oder daß man
($a_7$-$\gamma$) Hydroxamsäuren der Formel (Ib-1),

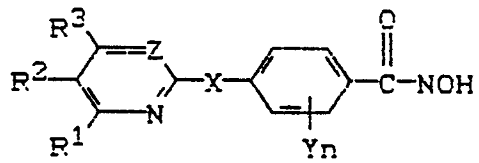

(Ib-1)

in welcher

$R^1$, $R^2$, $R^3$, Z, X, Y und n die oben angegebene Bedeutung haben,

mit bifunktionellen Alkylierungsmitteln der Formel (VIa),

$L^1$-$Alk^2$-$L^2$ (VIa)

in welcher

$L^1$ und $L^2$ für Chlor oder Brom stehen und

$Alk^2$ für eine gegebenenfalls substituierte $C_2$-oder $C_3$-Alkylenkette steht, wobei als Substituenten ausgewählt sind: $C_1$-$C_3$-Alkyl, $C_3$-$C_6$-Cycloalkyl oder gegebenenfalls durch Fluor, Chlor, Methyl, Ethyl, Methoxy und/oder Trifluormethyl substituiertes Phenyl,

gegebenenfalls in Gegenwart eines Verdünnungsmittels sowie in Gegenwart eines Säurebindemittels umsetzt,

oder daß man

($a_7$-$\delta$) Benzonitrile der Formel (XVI),

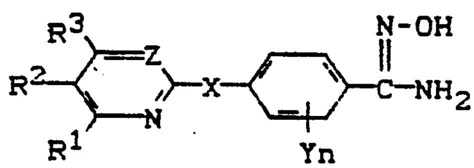

(XIV)

in welcher

$R^1$, $R^2$, $R^3$, Z, X, Y und n die oben angegebene Bedeutung haben,

mit Acylierungsmitteln der Formel (XVII),

$G-\overset{O}{\overset{\|}{C}}-R^{4-2}$ (XVII)

in welcher

G die oben angegebene Bedeutung hat und

$R^{4-2}$ für einen um ein Kohlenstoffatom verkürzten Rest $R^4$ steht, wobei $R^4$ die oben angegebene Bedeutung hat, insbesondere für gegebenenfalls durch Halogen, Aryl oder den Rest -D-$R^8$ substituiertes Alkyl, für Cycloalkyl oder für gegebenenfalls substituiertes Aryl steht,

gegebenenfalls in Gegenwart eines Verdünnungsmittels und gegebenenfalls in Gegenwart eines Säurebindemittels umsetzt und die so erhaltenen Verbindungen der Formel (XVIa),

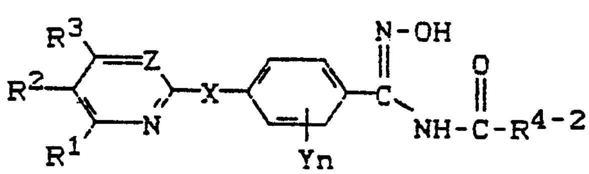

(XVIa)

inwelcher

$R^1$, $R^2$, $R^3$, $R^{4-2}$, Z, X, Y und n die oben angegebene Bedeutung haben,

mit wasserentziehenden Mitteln umsetzt zu den Pyri(mi)dyl-oxy-oder -thiobenzoesäure-Derivate der Formel (Ie),

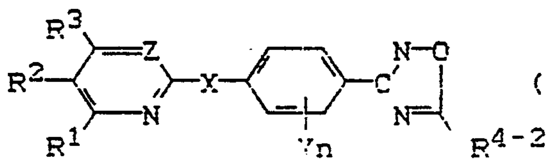

(Ie)

in welcher

$R^1$, $R^2$, $R^3$, $R^{4-2}$, Z, X, Y und n die oben angegebene Bedeutung haben,

oder daß man

(B) Pyri(mi)dyl-oxy-und -thiobenzoesäure-Derivate der Formel (I) erhält, wenn man

($B$-$b_1$) Pyri(mi)din-Derivate der Formel (XVIII)

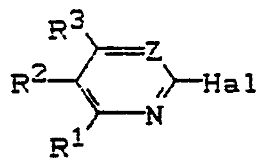

(XVIII)

in welcher

$R^1$, $R^2$, $R^3$ und Z die oben angegebene Bedeutung haben und

Hal für Chlor oder Brom steht,

mit 4-Hydroxy oder 4-Mercapto-benzoesäure-Derivaten der Formel (XIX),

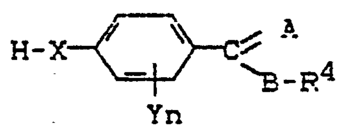

(XIX)

in welcher

X, Y, n, A, B und $R^4$ die oben angegebene Bedeutung haben,

gegebenenfalls in Gegenwart eines Verdünnungsmittels sowie in Gegenwart eines Säurebindemittels umsetzt, oder daß man

($B$-$b_2$) 2-Mercapto-pyri(mi)dine der Formel (XX)

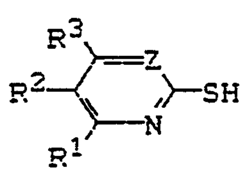

(XX)

in welcher

$R^1$, $R^2$, $R^3$, und Z die oben angegebene Bedeutung haben,

mit 4-Halogenbenzoesäure-Derivaten der Formel (XXI)

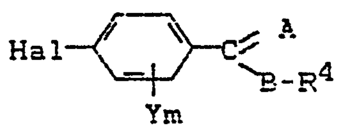

(XXI)

in welcher

$R^4$, A, B und Y die oben angegebene Bedeutung haben,
m für 1, 2 oder 3 steht und
Hal für Chlor oder Brom steht,
gegebenenfalls in Gegenwart eines Verdünnungsmittels sowie in Gegenwart eines Säurebindemittels umsetzt,
oder daß man
(C) Verbindungen der Formel (I), aus anderen Verbindungen der Formel (I) durch Austausch eines Substituenten Y erhält, wenn man
($C$-$C_1$) Verbindungen der Formel (I), in denen Y in 3-und/oder 5-Stellung für Wasserstoff steht, durch Umsetzung mit einem Halogenierungsmittel in Gegenwart eines Verdünnungsmittels und gegebenenfalls in Gegenwart eines Katalysators umwandelt in solche Verbindungen der Formel (I), in denen Y für Chlor oder Brom steht,
oder daß man
($C$-$C_2$) Verbindungen der Formel (I), in denen Y für Nitro steht, durch Umsetzung mit Reduktionsmitteln nach bekannten Verfahren umwandelt in solche Verbindungen der Formel (I), in denen Y für Amino steht, und
($C$-$C_3$) gegebenenfalls diese Amino-substituierten Verbindungen der Formel (I) (Y steht für Amino) mit Säurechloriden der Formel (XXII),

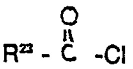 (XXII)

in welcher
$R^{23}$ für $C_1$-$C_4$-Alkyl, $C_1$-$C_4$-Alkoxy oder Di-$C_1$-$C_4$-alkylamino steht,
oder mit Methyl-oder Ethylisocyanat in allgemein üblicher Art und Weise acyliert,
oder daß man
($C$-$C_4$) Verbindungen der Formel (I), in denen Y für Amino steht, durch Umsetzung mit salpetriger Säure oder einem Alkylnitrit gegebenenfalls in Gegenwart eines Katalysators nach bekannten Verfahren umwandelt in solche Verbindungen der Formel (I), in denen Y für Halogen oder Cyano steht,
oder daß man
(D) Verbindungen der Formel (I), in denen $R^3$ für den Rest -O-$R^{24}$ oder -N$R^{25}R^{26}$ steht, wobei $R^{24}$, $R^{25}$ und $R^{26}$ die unten angegebene Bedeutung haben erhält,
wenn man Pyri(mi)dyl-oxy-und -thio-benzoesäure-Derivate der Formel (If),

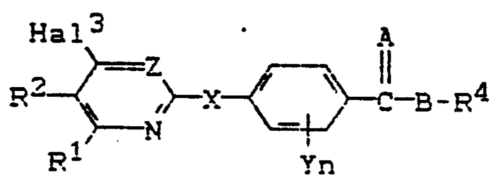 (If)

in welcher
$R^1$, $R^2$, $R^4$, Z, X, Y, n, A und B die oben angegebene Bedeutung haben und
$Hal^3$ für Fluor, Chlor oder Brom steht,
mit Alkoholen der Formel (XXIII),
H-O-$R^{24}$ (XXIII)
in welcher
$R^{24}$ für gegebenenfalls durch Fluor, Chlor oder Methoxy substituiertes $C_1$-$C_4$-Alkyl, oder $C_2$-$C_4$-Alkenyl steht,
gegebenenfalls in Gegenwart eines Verdünnungsmittels und in Gegenwart eines Säurebindemittels umsetzt, oder
mit Ammoniak, primären oder sekundären Aminen der Formel (XXIV),

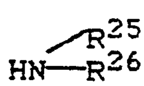 (XXIV)

in welcher
$R^{25}$ und $R^{26}$ unabhängig voneinander jeweils für Wasserstoff oder $C_1$-$C_4$-Alkyl, stehen, oder
$R^{25}$ und $R^{26}$ gemeinsam mit dem N-Atom auch einen 5-bis 7-gliedrigen heterocyclischen Ring bilden

können,
in Gegenwart eines Verdünnungsmittels und in Gegenwart eines Säurebindemittels umsetzt.

7. Herbizide und pflanzenwuchsregulierende Mittel, gekennzeichnet durch einen Gehalt an mindestens einem Pyri(mi)dyl-oxy-bzw. -thio-benzoesäure-Derivat der Formel (I) gemäß den Ansprüchen 1 bis 6.

8. Verfahren zur Bekämpfung von Unkräutern, dadurch gekennzeichnet, daß man Pyri(mi)dyl-oxy-und -thiobenzoesäure-Derivate der Formel (I) gemäß den Ansprüchen 1 bis 6 auf die Unkräuter und/oder ihren Lebensraum einwirken läßt.

9. Verwendung von Pyri(mi)dyl-oxy-und -thio-benzoesäure-Derivate der Formel (I) gemäß den Ansprüchen 1 bis 6 zur Bekämpfung von Unkräutern und/oder als Pflanzenwachstumsregulatoren.

10. Verfahren zur Herstellung von herbiziden und/oder pflanzenwuchsregulierenden Mitteln, dadurch gekennzeichnet, daß man Pyri(mi)dyl-oxy-und -thiobenzoesäure-Derivate der Formel (I) gemäß den Ansprüchen 1 bis 6 mit Streckmitteln und/oder oberflächenaktiven Substanzen vermischt.